(19) Europäisches Patentamt
European Patent Office
Office européen des brevets

(11) EP 0 983 234 B1

(12) # EUROPÄISCHE PATENTSCHRIFT

(45) Veröffentlichungstag und Bekanntmachung des Hinweises auf die Patenterteilung:
**03.07.2002 Patentblatt 2002/27**

(21) Anmeldenummer: **98928323.9**

(22) Anmeldetag: **22.05.1998**

(51) Int Cl.$^7$: **C07C 405/00**

(86) Internationale Anmeldenummer:
**PCT/EP98/03138**

(87) Internationale Veröffentlichungsnummer:
**WO 98/52914 (26.11.1998 Gazette 1998/47)**

(54) **LEUKOTRIEN-B 4-DERIVATE, INSBESONDERE 7-METHYLCYCLOHEXYL-LTB 4-ANTAGONISTEN**

LEUKOTRIENE B 4 DERIVATIVES, IN PARTICULAR 7-METHYLCYCLOHEXYL-LTB 4 ANTAGONISTS

DERIVES DE LEUCOTRIENE B 4 , EN PARTICULIER ANTAGONISTES DE 7-METHYLCYCLOHEXYL-LTB 4

(84) Benannte Vertragsstaaten:
**AT BE CH CY DE DK ES FI FR GB GR IE IT LI LU MC NL PT SE**

(30) Priorität: **23.05.1997 DE 19722846**

(43) Veröffentlichungstag der Anmeldung:
**08.03.2000 Patentblatt 2000/10**

(73) Patentinhaber: **Schering Aktiengesellschaft
13353 Berlin (DE)**

(72) Erfinder:
- **BUCHMANN, Bernd
D-16540 Hohen Neuendorf (DE)**
- **FRÖHLICH, Wolfgang
D-10709 Berlin (DE)**
- **GIESEN, Claudia
D-13587 Berlin (DE)**
- **HENNEKES, Hartwig
D-13507 Berlin (DE)**
- **JAROCH, Stefan
D-10625 Berlin (DE)**
- **SKUBALLA, Werner
D-13465 Berlin (DE)**

(74) Vertreter: **Dörries, Hans Ulrich, Dr. et al
Dörries, Frank-Molnia & Pohlman,
Triftstrasse 13
80538 München (DE)**

(56) Entgegenhaltungen:
**WO-A-95/20563**
**DE-A- 4 236 540**
**DE-A- 4 242 390**

EP 0 983 234 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Leukotrien $B_4$-Derivate, Verfahren zu ihrer Herstellung sowie ihre Verwendung als Arzneimittel. Die neuen Verbindungen sind optisch aktive Strukturanaloge von vorbekannten Leukotrien-$B_4$-Antagonisten, die einen Sechsring als Grundstrukturelement enthalten (DE 39 17 597, DE 42 27 790, DE 42 42 390).

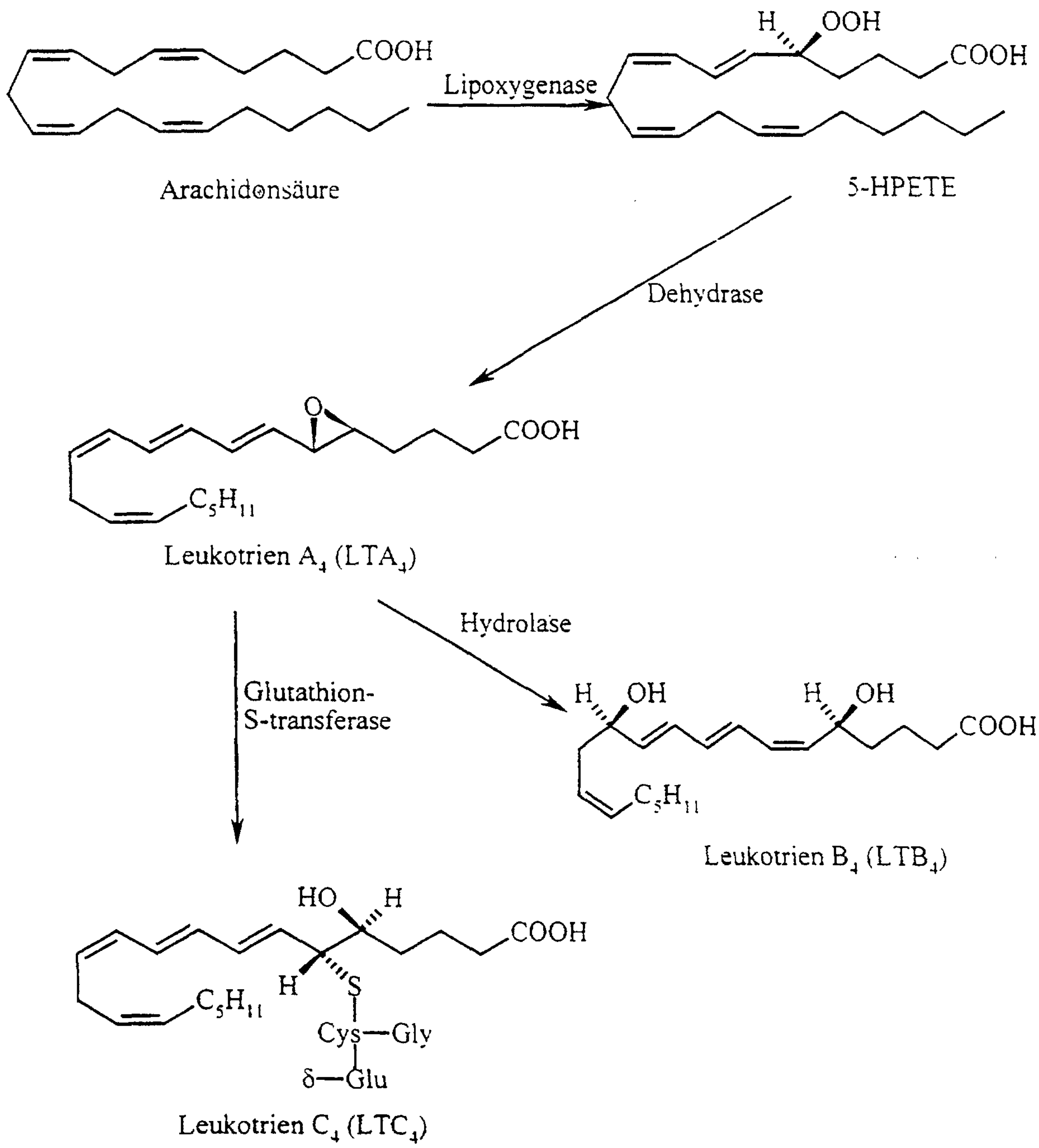


Arachidonsäure

5-HPETE

Leukotrien $A_4$ ($LTA_4$)

Leukotrien $B_4$ ($LTB_4$)

Leukotrien $C_4$ ($LTC_4$)

Leukotrien $B_4$ ($LTB_4$) wurde von B. Samuelsson und Mitarbeitern als Metabolit der Arachidonsäure entdeckt. Bei der Biosynthese wird durch das Enzym 5-Lipoxygenase zunachst als zentralens Zwischenprodukt das Leukotnen $A_4$ gebildet. das dann durch eine spezifische Hydrolase in das $LTB_4$ umgewandelt wird.

Die Nomenklatur der Leukotriene kann folgenden Arbeiten entnommen werden:

a) B. Samuelsson et al., Prostaglandins 19. 654 (1980); 17. 785 (1979);
b) C. N. Serhan et al., Prostaglandins 34, 201 (1987).

Die physiologische und insbesondere die pathophysiologische Bedeutung des Leukotrien $B_4$ wird in einigen

neueren Arbeiten zusammengefaßt: a) The Leukotriens, Chemistry and Biology eds. L W. Chakrin, D. M. Bailey, Academic Press 1984. b) J. W. Gillard et al.; Drugs of the Future 12. 453 (1987). c) B. Samuelsson., Sciences 237. 1171 (1987). d) C. W. Parker. Drug Development Research 10. 277 (1987). e) W. R Henderson. Annals of Internal Medicine 121, 684 (1994). Hieraus ergibt sich. daß $LTB_4$ ein wichtiger Entzündungsmediator für entzündliche Erkrankungen ist. bei denen Leukozyten in das erkrankte Gewebe einwandern.

Die Effekte von $LTB_4$ werden auf zellulärer Ebene durch die Bindung von $LTB_4$ an einen spezifischen Rezeptor ausgelöst.

Von $LTB_4$ weiß man, daß es die Adhäsion von Leukozyten an die Blutgefäßwand verursacht. $LTB_4$ ist chemotaktisch wirksam, d. h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin $E_2$ beobachtet wird. $LTB_4$ spielt offensichtlich bei entzündlichen. allergischen und immunologischen Prozessen eine entscheidende Rolle.

Leukotriene und insbesondere $LTB_4$ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen. wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis, atopischer Dermatitis, allergischer Kontaktdermatitis. bullöse Pemphigoide, verzögerte Duchurtikaria und allergischer Vasculitis gemessen.

Leukotriene und insbesondere $LTB_4$ sind auch an Erkrankungen innerer Organe beteiligt, für die eine akute oder chronische entzündliche Komponente beschrieben wurde, z. B.: Gelenkerkrankungen (Rheumatische Atthritis); Erkrankungen des Respirationstraktes (Asthma und chronisch obstruktive Lungenerkrankungen (OPD)); entzündliche Darmerkrankungen (ulceröse Colitis und Morbus Crohn); sowie Reperfusionsschäden (an Herz-, Darm- oder Nierengewebe), die durch zeitweisen krankhaften Verschluß von Blutgefäßen entstehen, wie Glomerulonephritis. NSAID Gastropathien, Multiple Sklerose. Rhinitis und entzündliche Augenerkrankungen.

Weiterhin sind Leukotriene und insbesondere $LTB_4$ bei der Erkrankung an multipler Sklerose beteiligt und bei dem klinischen Erscheinungsbild des Schocks (ausgelöst durch Infektionen, Verbrennungen oder bei Komplikationen bei der Nierendialyse oder anderen extra besprochenen Perfusionstechniken).

Leukotriene und insbesondere $LTB_4$ haben weiterhin einen Einfluß auf die Bildung von weißen Blutkörperchen im Knochenmark, auf das Wachstum von glatten Muskelzellen, vom Keratinozyten und von B-Lymphozyten. $LTB_4$ ist daher bei Erkrankungen mit entzündlichen Prozessen und bei Erkrankungen mit pathologisch gesteigerter Bildung und Wachstum von Zellen beteiligt.

Erkrankungen mit diesem Erscheinungsbild stellen z. B. Leukemie oder Artherosklerose dar.

Leukotriene und insbesondere $LTB_4$ und seine Derivate sind zur Senkung erhöhter Triglyceridspiegel geeignet und wirken somit antiatherosklerotisch und gegen Fettleibigkeit.

Durch die Antagonisierung der Effekte insbesondere von $LTB_4$ sind die Wirkstoffe und deren Darreichungsformen dieser Erfindung spezifische Heilmittel bei der Erkrankung von Mensch und Tier, bei denen insbesondere Leukotriene eine pathologische Rolle spielen.

Neben therapeutischen Möglichkeiten, die sich aus einer Antagonisierung der $LTB_4$-Wirkung mit $LTB_4$-Analoga ableiten lassen, konnte auch die Nützlichkeit und potentielle Anwendung von Leukotrien $B_4$-Agonisten zur Behandlung von Pilzerkrankungen der Haut gezeigt werden (H. Katayama, Prostaglandins 34, 797 (1988)).

Die Erfindung betrifft Leukotrien-$B_4$-Derivate der allgemeinen Formel I,

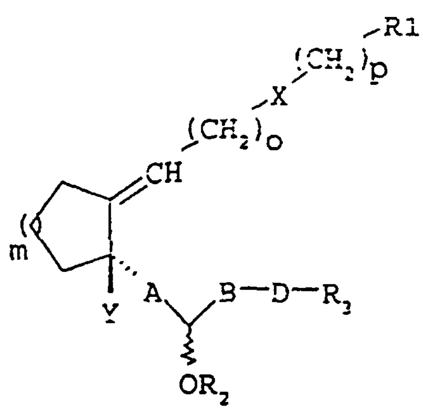

(I)

worin

| | |
|---|---|
| $R_1$ | $CH_2OH$, $CH_3$, $CF_3$, $COOR_4$, $CONR_5R_6$, und |
| $R_2$ | H oder einen organischen Säurerest mit 1-15 C-Atomen darstellt, |
| $R_3$ | H, gegebenenfalls einfach oder mehrfach substituiertes $C_1$-$C_{14}$-Alkyl, $C_3$-$C_{10}$-Cycloalkyl, einen gegebenenfalls unabhängig voneinander einfach oder mehrfach durch Halogen. Phenyl. $C_1$-$C_4$-Alkyl, $C_1$-$C_4$-Alkoxy, Fluormethyl, Chlormethyl. Trifluormethyl. Carbonyl. Carboxyl oder Hydroxy substituierten $C_6$-$C_{10}$-Arylrest oder einen 5-6 gliedrigen aromatischen heterocyclischen Ring mit wenigstens 1 Heteroatom symbolisiert. |
| $R_4$ | Wasserstoff. $C_1$-$C_{10}$-Alkyl, $C_3$-$C_{10}$-Cycloalkyl, einen gegebenenfalls durch 1-3 Halogen, Phenyl, $C_1$-$C_4$-Alkyl, $C_1$-$C_4$-Alkoxy, Fluormethyl, Chlormethyl, Trifluormethyl, Carboxyl oder Hydroxy substituierten $C_6$-$C_{10}$-Arylrest, $CH_2$-CO-($C_6$-$C_{10}$) Aryl oder einen 5-6 gliedrigen Ring mit wenigstens 1 Heteroatom bedeutet. |
| A | eine trans, trans-CH=CH-CH=CH, eine -$CH_2CH_2$-CH=CH- oder eine Tetramethylengruppe. |
| B | eine $C_1$-$C_{10}$- gerad- oder verzweigtkettige Alkylengruppe, die gegebenenfalls durch Fluor substituiert sein kann oder die Gruppe |

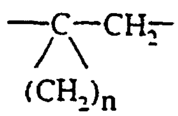

oder

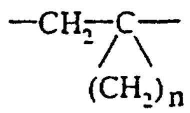

symbolisiert,

| | |
|---|---|
| D | eine Direktbindung, Sauerstoff, Schwefel, -C≡C-, -CH=$CR_7$, oder gemeinsam mit |
| B | auch eine Direktbindung bedeuten kann, |
| $R_5$ und $R_6$ | gleich oder verschieden sind und H oder gegebenenfalls durch Hydroxygruppen substituiertes $C_1$-$C_4$-Alkyl oder $R_6$ H und $R_5$ $C_1$-$C_{15}$-Alkanoyl oder $R_8SO_2$- darstellen, |
| $R_7$ | H, $C_1$-$C_5$-Alkyl, Chlor, Brom bedeutet, |
| $R_8$ | die gleiche Bedeutung wie $R_3$ besitzt, |
| m | 1-3 bedeutet |
| o | 0-5 bedeutet, |
| p | 0-5 bedeutet, |
| x | eine Direktbindung, Sauerstoff, Schwefel, |
| y | ein gegebenenfalls einfach oder mehrfach substituiertes $C_1$-$C_8$-Alkyl, $C_3$-$C_{10}$-Cycloalkyl, und |
| n | 2-5 ist sowie, wenn $R_4$ Wasserstoff bedeutet, deren Salze mit physiologisch verträglichen Basen und deren Cyclodextrinclathrate. |

Die Gruppe $OR_2$ kann $\alpha$- oder $\beta$-ständig sein. Die Formel I umfaßt sowohl Racemate als auch die möglichen reinen Diastereomeren und Enantiomeren.

Als Alkylgruppen $R_4$ kommen gerad- oder verzweigtkettige Alkylgruppen mit 1-10 C-Atomen in Betracht, wie beispielsweise Methyl. Ethyl, Propyl, Butyl, Isobutyl. tert.-Butyl, Pentyl, Neopentyl, Hexyl, Heptyl. Decyl.
Die Alkylgruppen $R_4$ können gegebenenfalls ein- bis mehrfach substituiert sein durch Halogenatome. Alkoxygruppen, gegebenenfalls substituierte Aryl- bzw. Aroylgruppen mit 6-10 C-Atomen (bezüglich möglicher Substituenten siehe unter Aryl $R_4$), Dialkyiamino und Trialkylammonium mit 1-4 C-Atomen im Alkyl-Teil. wobei die einfache Substitution bevorzugt sein soll. Als Substituenten seien beispielsweise genannt Fluor, Chlor oder Brom, Phenyl. Dimethylamino, Diethylamino. Methoxy, Ethoxy. Als bevorzugte Alkylgruppen $R_4$ sind solche mit 1-4 C-Atomen zu nennen.

Die Cycloalkylgruppe $R_4$ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl, Cyclohexyl, Methylcyclohexyl.

Als Arylgruppen $R_4$ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können duch 1-3 Halogenatome (F, Cl. Br). eine Phenylgruppe, 1-3 Alkylgruppen mit jeweils 1-4 C-Atomen, eine Chlormethyl-, eine Fluormethyl-. Trifluormethyl-, Carboxyl-. Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Als heterocyclische Gruppen $R_4$ kommen 5- und 6-gliedrige aromatische Heterocyclen in Frage, die wenigstens 1 Heteroatom, vorzugsweise Stickstoff. Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl u. a.

Als Säurerest $R_5$ kommen solche physiologisch verträglichen Säuren in Frage. Bevorzugte Säuren sind organische Carbonsäuren und Sulfonsäuren mit 1-15 Kohienstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien $C_{1-4}$-Alkyl-, Hydroxy-, $C_{1-4}$-Alkoxy, Oxooder Aminogruppen oder Halogenatome (F, Cl, Br) erwähnt. Beispielweise seien folgende Carbonsäuren genannt: Ameisensäure, Essigsäure, Propionsaure, Buttersäure, Isobuttersäure, Valeriansäure, Isovalenansäure, Capronsäure, Önanthsäure, Caprylsäure, Pelargonsaure, Caprinsäure, Undecylsäure, Laurinsaure, Tridecylsaure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, $C_{1-4}$-Alkoxy- oder Carboxy-Gruppen substituierte Benzoesäuren, Nikotinsäure, Isonikotinsäure, Furan-2-carbonsäure, Cyclopentylpropionsäure. Als bevorzugte Arylsulfonylreste und Alkansulfonylreste $R_8SO_2$- werden solche betrachtet, die sich von einer Sulfonsaure, mit bis zu 10 Kohlenstoffatomen ableiten. Als Sulfonsäuren kommen beispielsweise Methansulfonsäure, Ethansulfonsäure, Isopropansulfonsäure, Cyclohexansulfonsäure, Benzolsulfonsäure, p-Toluolsulfonsäure, p-Chlorbenzolsufonsäure, N,N-Dimethylaminosulfonsäure, N,N-Diisobutylaminosulfonsäure, N,N-Dibutylaminosulfonsäure, Pyrrolidino-, Piperidino-, Piperazino-, M-Methylpiperazino- und Morpholinosulfonsäure in Frage.

Als Alkylgruppen $R_3$ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl $R_5$) substituiert sein können. Beispielsweise seien genannt Methyl-, Ethyl-, Propyl-, Butyl-, Isobutyl-, tert,-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen $R_3$ Halogen-substituiert, kommen als Halogene Fluor, Chlor und Brom in Frage.

Als Beispiele für Halogen-substituierte Alkylgruppen $R_3$ kommen Alkyle mit terminalen Trifluormethylgruppen in Betracht.

Die Cycloalkylgruppe $R_3$ kann im Ring 3-10, vorzugsweise 3-6 Kohlenstoffatome enthalten. Die Ringe können durch Alkylgruppen mit 1-4 Kohlenstoffatomen gegebenenfalls durch Halogene substituiert sein. Beispielsweise genannt seien Cyclopropyl, Cyclobutyl, Cyclopentyl, Cyclohexyl, Methyl-cyclohexyl, Fluor-cyclohexyl.

Als substituierte bzw. unsubstitiuerte Arylgruppen $R_3$ kommen beispielweise in Betracht: Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, Fluormethyl-, Trifluormethyl-, Carboxyl, $C_1$-$C_4$ Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische aromatische Gruppen $R_3$ kommen 5- und 6-gliedrige Heterocyclen in Frage, die wenigstens 1 Heteroatom, vorzugsweise Stickstoff. Sauerstoff oder Schwefel enthalten. Beispielsweise seien genannt 2-Furyl, 1-Thienyl, 2-Pyridyl, 3-Pyridyl, 4-Pyridyl, Oxazolyl, Thiazolyl, Pynmidinyl, Pyndazinyl, Pyrazinyl, 3-Furyl, 3-Thienyl, u. a.

Als Alkylengruppen B kommen geragkettige oder verzweigte, gesättigte oder ungesättigte Alkylenreste, vorzugsweise gesättigte mit 1-10, insbesondere mit 1-5 C-Atomen, in Frage, die gegebenenfalls durch Fluoratome substituiert sein konnen. Beispielsweise seien genannt: Methylen, Fluormethylen, Difluormethylen, Ethylen, 1.2-Propylen, Ethylethylen, Trimethylen, Tetramethylen, Pentamethylen. 1,2-Difluorethylen, 1-Fluorethylen, 1-Methyltetramethylen, 1-Methyl-tri-methylen, 1-Methylen-ethylen, 1-Methylen-tetramethylen.

Die Alkylengruppe B kann weiterhin die Gruppe

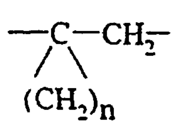 oder 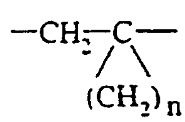

darstellen, wobei n=2-5, bevorzugt 3-5 bedeutet.

Als Säurereste $R_2$ kommen solche von physiologisch verträglichen Säureresten in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch, und/oder in üblicher Weise sustituiert sein. Als Beispiele für die Substituenten seien $C_{1-4}$-Alkyl, Hydroxy, $C_{1-4}$-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyessigsäure, Methoxyessigsäure, Ethoxyessigsäure, Mono-. Di- und Trichloressigsaure, Aminoessigsäure, Diethylaminoessigsäure, Piperidinoessigsäure, Morpholinoessigsäure, Milchsäure, Bemsteinsäure, Adipinsäure, Benzoesäure, mit Halogen (F, Cl, Br) oder Trifluormethyl-, Hydroxy, $C_{1-4}$-Alkoxy- oder Carboxy-Gruppen substituierte Benzoesäuren, Nikotinsäure, Isonikotinsäure, Furan-2-carbonsäure, Cyclopentylpropionsäure. Als bevorzugte Säurereste $R_2$ und $R_3$ werden solche Acylreste mit bis zu 10 Kohlenstoffatomen betrachtet.

Die Alkylreste $R_5$ und $R_6$, die gegebenenfalls Hydroxygruppen enthalten, sind geradkettige oder verzweigte Alkylreste, insbesondere geradkettige wie zum Beispiel Methyl, Ethyl, Propyl, Butyl, Pentyl, Hexyl, besonders bevorzugt Methyl.

$R_7$ als $C_{1-5}$-Alkyl bedeutet geradkettige oder verzweigtkettige Alkylreste wie sie für $R_3$ bzw. $R_4$ bereits genannt wurden. Bevorzugte Alkylreste $R_7$ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxide wie Natrium- und Kaliumhydroxid, Erdalkalihydroxide wie Calciumhydroxid, Ammoniak, Amine, wie Ethanolamin, Diethanolamin, Triethanolamin. N-Methylglucamin, Morpholin, Tris-(hydroxymethyl)-methylamin usw.

Um zu den Cyclodextrinclathraten zu gelangen werden die Verbindungen der Formel I mit α-, β- oder γ-Cyclodextrin umgesetzt. Bevorzugt sind β-Cyclodextrinderivate.

Bevorzugte Verbindungen der vorliegenden Erfindung sind Verbindungen der allgemeinen Formel I, wobei die Reste die folgende Bedeutung haben:

| | |
|---|---|
| $R_1$ | ist $CH_2OH$, $CONR_5R_6$, $COOR_4$ mit $R_4$ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, $C_{1-4}$-Alkyl, $C_{1-4}$-Alkoxy, Chlormethyl, Fluormethyl, Trifluormethyl, Carboxy oder Hydroxy substituierten Phenylrestes. |
| x | ist ein Sauerstoffatom, |
| y | ist eine Methylgruppe, |
| p | ist 1-3, |
| o | ist 1-3, |
| m | ist 1-3, |
| A | ist eine trans-CH=CH-CH=CH- oder Tetramethylengruppe; |
| B | ist eine geradkettige oder verzweigtkettige gesättigte oder ungesättigte Alkylengruppe mit bis zu 10 C-Atomen, die gegebenenfalls durch Fluor substituiert sein kann, oder die Gruppe |

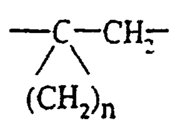 oder 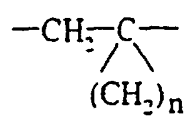

mit n=2-5;

| | |
|---|---|
| D | ist eine Direktbindung, Sauerstoff, Schwefel, eine -C≡C-Gruppe oder eine $-CH=CR_7$-Gruppe mit $R_7$ als Wasserstoff. $C_{1-5}$-Alkyl, Chlor oder Brom; |
| B und D | sind gemeinsam eine Direktbindung; |
| $R_2$ | bedeutet Wasserstoff oder einen organischen Säurerest mit 1-15 C-Atomen; |
| $R_5$ und $R_6$ | die oben angegebenen Bedeutungen aufweisen; |
| $R_3$ | ist ein Wasserstoffatom, $C_{1-10}$-Alkyl, Cycloalkyl mit 5-6 C-Atomen, ein gegebenenfalls durch 1-2 Chlor, Brom, Phenyl, $C_{1-4}$-Alkyl, $C_{1-4}$-Alkoxy, Chlormethyl, Fluormethyl, Trifluormethyl, Carboxy oder Hydroxy substituierter Phenylrest und, falls |
| $R_4$ | ein Wasserstoff bedeutet, deren Salze mit physiologisch verträglichen Basen und Cyclodextrinclathrate. |

Besonders bevorzugte Verbindungen der vorliegenden Erfindung sind Verbindungen der allgemeinen Formel I, wobei die Reste folgende Bedeutung haben:

| | |
|---|---|
| $R_1$ | ist $CH_2OH$, $CONR_5R_6$, $COOR_4$ mit $R_4$ in der Bedeutung eines Wasserstoffatoms, eines Alkylrestes mit 1-4 C-Atomen |
| $R_2$ | bedeutet Wasserstoff oder einen organischen Säurerest mit 1-6 C-Atomen; |
| $R_3$ | ist ein Wasserstoffatom oder $C_{1-10}$-Alkyl; |
| $R_5$ und $R_6$ | die oben angegebenen Bedeutungen aufweisen; |
| A | ist eine trans, trans-CH=CH-CH=CH- oder Tetramethylengruppe, |
| B | ist eine geradkettige oder verzweigtkettige Alkylengruppe mit bis zu 5 C-Atomen oder die Gruppe |

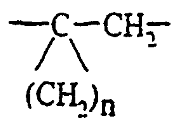 oder 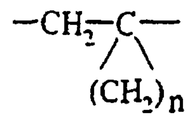

mit n=3,4

| | |
|---|---|
| D | ist eine Direktverbindung oder eine -C≡C-Gruppe oder eine $-CH=CR_7-$ Gruppe mit $R_7$ als Wasserstoff oder $C_{1-5}$-Alkyl; |
| x | ist ein Sauerstoffatom, |
| y | ist eine Methylgruppe, |
| p | ist 1, |
| o | ist 1, |
| m | ist 1,2; |
| B und D | sind gemeinsam eine Direktbindung; |

und, falls $R_4$ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der allgemeinen Formel I, das dadurch gekennzeichnet ist, daß man ein Keton der Formel II,

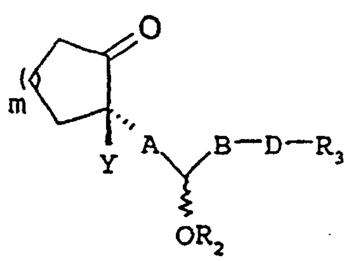

(II)

worin A, B, D, $R_2$, $R_3$ und Y die oben angegebene Bedeutung haben, gegebenenfalls nach Schutz freier Hydroxygruppen in $R_2$ mit einem Olefinierungsreagenz der allgemeinen Formel III

$$E-CH_2-(CH_2)_o-X-(CH_2)_p-R_1 \quad (III)$$

wobei E einen

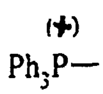 oder 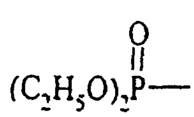 oder 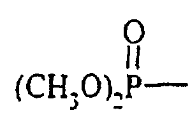 Rest

darstellt und o. X, p, $R_1$ die oben angegebene Bedeutung aufweisen, in Gegenwart einer Base umsetzt und gegebenenfalls anschließend in beliebiger Reihenfolge Isomere trennt, geschützte Hydroxygruppen freisetzt und/oder eine freie Hydroxygruppe verethert und/oder die 1-Hydroxygruppe zur Carbonsäure oxidiert und/oder reduziert und/oder

eine Carboxylgruppe verestert und/oder eine freie Carboxylgruppe in ein Amid überführt oder eine Carboxylgruppe mit einer physiologisch verträglichen Base in ein Salz überführt.

Die Umsetzung der Verbindung der allgemeinen Formel II mit einem Olefinierungsreagenz der allgemeinen Formel III wird bei Temperaturen von -80°C bis 100°C, vorzugsweise -20°C bis 80°C in einem aprotischen Lösungsmittel oder Lösungsmittelgemisch, beispielsweise Tetrahydrofuran. Diethylether. Dimethylsulfoxid in Gegenwart einer Base vorgenommen. Als Basen kommen je nach Bedeutung des Restes E Natriumhydrid, Methylsulfinylmethylnatrium, Kalium-tert.-butylat. Lithiumdiisopropylamid, 1,5-Diazabicyclo[4.3.0]non-5-en in Frage. Die Trennung der dabei erhaltenen Z- und E-konfigurierten Olefine erfolgt auf übliche Art, beispielsweise durch Säulenchromatographie.

Die Reduktion zu den Verbindungen der Formel I mit $R_1$ in der Bedeutung einer $CH_2OH$-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether. Tetrahydrofuran, Dimethoxyethan, Toluol usw. in Frage. Die Reduktion wird bei Temperaturen von -30°C bis zur Siedetemperatur des verwendeten Lösungsmittels. vorzugsweise 0°C bis 30°C vorgenommen.

Die Veresterung der Alkohole der Formel I ($R_2$=H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise Natriumhydrid, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid. Dimethylsulfoxid bei Temperaturen über oder unter Raumtemperatur, zum Beispiel zwischen -80°C bis 100°C, vorzugsweise bei Raumtemperatur, vorgenommen werden. Die Veretherung der Alkohole der Formel I ($R_1$=$CH_2OH$) erfolgt in an sich bekannter Weise. Zum Beispiel erfolgt die Veretherung dadurch, daß man den Alkohol der allgemeinen Formel I ($R_1$=$CH_2OH$), gegebenenfalls nach Schutz anwesender freier Hydroxygruppen mit einem Halogencarbonsäurederivat oder Halogenalkylderivat der allgemeinen Formel IV,

$$\text{Hal-}(CH_2)_p\text{-}R_1 \qquad \text{(IV)}$$

wobei Hal ein Chlor-, Brom- oder Jodatom und $R_1$ die oben angegebene Bedeutung aufweist in Gegenwart einer Base umsetzt und anschließend gegebenenfalls $R_1$ wie oben beschrieben weiter funktionalisiert. Die Umsetzung der Verbindung der allgemeinen Formel I mit einer Halogenverbindung der allgemeinen Formel IV wird bei Temperaturen von 0°C bis 100°C, vorzugsweise 10°C bis 80°C, in einem aprotischen Lösungsmittel oder Lösungsmittelgemisch, beispielsweise Dimethylsulfoxid, Dimethylformamid, Tetrahydrofuran, Toluol usw., vorgenommen. Als Basen kommen die dem Fachmann für Veretherungen bekannten Basen in Frage, beispielsweise Natriumhydrid, Kalium-tert.-butylat, Butyllithium. Die oben genannte Veretherung kann auch vorzugsweise unter Phasentransfer-Bedingungen mit 20-50%iger wäßnger Natriumhydroxid- oder Kaliumhydroxid Lösung ohne ein zusätzliches Lösungsmittel oder in einem aprotischen Lösungsmittel wie beispielsweise Toluol in Gegenwart eines Phasentransfer-Katalysators wie Tetrabutylammoniumhydrogensulfat bei Temperaturen zwischen 0°C und 90°C, vorzugsweise zwischen 20°C und 60°C durchgeführt werden.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters. 1979, 399), Jones Reagenz (J. Chem. Soc. 1953, 2555) oder Platin/Sauerstoff (Adv. in Carbohydrate Chem. 17, 169 (1962) oder Collins-Oxidation (Tetrahedron Letters 1968, 3363) und anschließende Jones-Oxidation. Die Oxidation mit Pyridiniumdichromat wird bei Temperaturen von 0°C bis 100°C vorzugsweise bei 20°C bis 40°C in einem gegen das Oxidationsmittel inerten Lösungsmittel, beispielsweise Dimethylformamid, durchgeführt.

Die Oxidaton mit Jones-Reagenz wird bei Temperaturen von -40°C bis +40°C, vorzugsweise 0°C bis 30°C in Aceton als Lösungsmittel durchgeführt.

Die Oxidation mit Platin/Sauerstoff wird bei Temperaturen von 0°C bis 60°C, vorzugsweise 20°C bis 40°C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden (Asymmetric Synthesis, Vol. 1-5, Ed. J. D. Morrison, Academic Press. Inc. Orlando etc., 1985: Chiral Separations by HPLC, Ed. A. M. Krstulovic; John Wiley & Sons: New York etc. 1989).

Die Freisetzung der funktionell abgewandelten Hydroxygruppen erfolgt nach bekannten Methoden. Beispielsweise wird die Abspaltung - von Hydroxyschutzgruppen. wie beispielsweise des Tetrahydropyranylrestes. in einer wässrigen Lösung einer organischen Säure, wie z. B. Oxalsäure, Essigsäure, Propionsäure u. a., oder in einer wässrigen Lösung einer anorganischen Säure, wie z. B. Salzsäure durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol, Ethanol und Ether, wie Dimethoxyethan. Dioxan und Tetrahydrofuran.

Tetrahydrofuran wird bevorzugt angewendet. Die Abspaltung wird vorzugsweise bei Temperaturen zwischen 20°C und 80°C durchgeführt. Die Abspaltung der Silyletherschutzgruppen erfolgt beispielsweise mit Tetrabutylammoniumfluorid oder mit Kaliumfluond in Gegenwart eines Kronenethers (wie zum Beispiel Dibenzo[18]-Krone-6). Als Lösungsmittel sind beispielsweise geeignet Tetrahydrofuran. Diethylether. Dioxan, Dichlormethan, usw. Die Abspaltung wird vorzugsweise bei Temperaturen zwischen 0°C und 80°C durchgeführt.

Die Verseifung der Acylgruppen erfolgt beispielsweise mit Alkali- oder Erdalkalicarbonaten oder -hydroxiden in einem Alkohol oder in der wäßrigen Lösung eines Alkohols. Als Alkohol kommen niedere aliphatische Alkohole in Betracht, wie z. B. Methanol, Ethanol, Butanol usw., vorzugsweise Methanol. Als Alkalicarbonate und -hydroxide seien Kalium- und Natriumsalze genannt. Bevorzugt sind Kaliumsalze.

Als Erdalkalicarbonate und -hydroxide sind beispielsweise geeignet Calciumcarbonat, Calciumhydroxid und Bariumcarbonat. Die Umsetzung erfolgt bei -10°C bis +70°C, vorzugsweise bei +25°C.

Die Einführung der Estergruppe $-COOR_4$ für $R_1$, bei welcher $R_4$ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffs in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden (Org. Reactions Bd. 8, Seiten 389-394 (1954)).

Die Einführung der Estergruppe $-COOR_4$ für $R_1$, bei welcher $R_4$ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin. Dimethylaminopyridin, Triethylamin, in einem inerten Lösungsmittel umgesetzt. Als Lösungsmittel kommen Methylenchlorid, Ethylenchlorid. Chloroform, Essigester, Tetrahydrofuran, vorzugsweise Chloroform in Frage. Die Reaktion wird bei Temperaturen zwischen -30°C und +50°C, vorzugsweise, bei 10°C durchgeführt.

Sollen im Primärprodukt enthaltene C=C-Doppelbindungen reduziert werden, erfolgt die Hydrierung nach an sich bekannten Methoden.

Die Hydrierung des $\Delta^{8,10}$-Diensystems wird in an sich bekannter Weise bei tiefen Temperaturen, vorzugsweise bei etwa -20°C bis +30°C in einer Wasserstoffatomosphäre in Gegenwart eines Edelmetallkatalysators durchgeführt. Als Katalysator ist zum Beispiel 10% Palladium auf Kohle geeignet.

Die Leukotrien-$B_4$-Derivate der Formel I mit $R_4$ in der Bedeutung eines Wasserstoffs können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält. nach Abdampfen des Wassers oder nach Zugaben eines mit Wasser mischbaren Lösungsmittels, z. B. Alkohol oder Aceton, das feste anorganische Salz.

Zur Herstellung eines Aminosalzes wird $LTB_4$-Säure z. B. in einem geeigneten Lösungsmittel, beispielsweise. Ethanol, Aceton, Diethylether, Acetonitril oder Benzol gelöst und mindestens die stochiometrische Menge des Amins der Lösung zugesetzt. Dabei fällt das Salz gewöhnlich in fester Form an oder wird nach Verdampfen des Lösungsmittels in üblicher Weise isoliert.

Die Einführung der Amidgruppe-$CONHR_5$ mit $R_5$ in der Bedeutung von Alkanoyl erfolgt nach den dem Fachmann bekannten Methoden. Die Carbonsäuren der Formel I ($R_4$=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_5$=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30°C und +60°C, vorzugsweise bei 0°C bis 30°C. Eine weitere Herstellungsart für die Amide besteht in der Amidolyse des 1-Esters ($R_1=COOR_4$) mit dem entsprechenden Amin.

Eine weitere Möglichkeit für die Einführung der Amidgruppe $-CONHR_5$ besteht in der Umsetzung einer 1-Carbonsäure der Formel I ($R_4$=H), in der freie Hydroxygruppen gegebenenfalls intermediär geschützt sind, mit Verbindungen der Formel IV.

$$O = C = N - R_5 \qquad \text{(IV)}$$

worin $R_5$ die oben angegebene Bedeutung hat.

Die Umsetzung der Verbindung der Formel I ($R_4$=H) mit einem Isocyanat der Formel IV erfolgt gegebenenfalls unter Zusatz eines tertiären Amins, wie z. B. Triethylamin oder Pyridin. Die Umsetzung kann ohne Lösungsmittel oder

in einem inerten Lösungsmittel, vorzugsweise Acetonitril, Tetrahydrofuran, Aceton, Dimethylacetamid, Methylenchlorid, Diethylether, Toluol, bei Temperaturen zwischen -80°C bis 100°C, vorzugsweise bei 0°C bis 30°C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen im Leukotrien-$B_4$-Rest, so werden diese OH-Gruppen auch zur Reaktion gebracht. Werden letztlich Endprodukte gewünscht. die freie Hydroxylgruppen enthalten, geht man zweckmäßigerweise von Ausgangsprodukten aus, in denen diese durch vorzugsweise leicht abspaltbare Etheroder Acylreste intermediär geschützt sind.

Die Trennung von Diastereomeren erfolgt nach den dem Fachmann bekannten Methoden, beispielsweise durch Säulenchromatographie.

Die als Ausgangsmaterial dienenden Verbindungen der allgemeinen Formel II können beispielsweise hergestellt werden, in dem man in an sich bekannter Weise einen Ester der allgemeinen Formel V((a) K. Sakai et al., Tetrahedron 50, 3315 (1995); b) K. Koga et al., Tetrahedron 49,1579 (1993)),

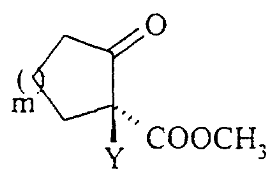

(V)

worin m und Y die oben angegebenen Bedeutungen aufweisen, mit Ethylenglycol ketalisiert, mit Diisobutylaluminiumhydrid reduziert und anschließend mit dem Collins-Reagenz oder durch das Swern-Verfahren (Tetrahedron Letters 34, 1651 (1978)) zum Aldehyd der allgemeinen Formel VI oxidiert.

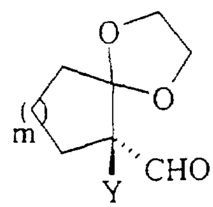

(VI)

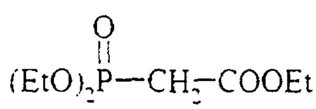

(VII) oder

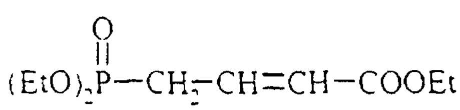

(VIII)

Die Wittig-Horner Olefinierung des Aldehyds VI mit dem Phosphonat der Formel VII und einer Base und gegebenenfalls anschließender Hydrierung sowie anschließender Reduktion der Estergruppe, Oxidation des primären Alkohols, nochmalige Wittig-Horner-Olefinierung mit dem Phosphonat der Formel VII und gegebenenfalls anschließender Hydrierung oder Wittig-Horner Reaktion des Aldehyds VI mit einem Phosphonat der Formel VIII liefert den Ester der allgemeinen Formel IX, wobei

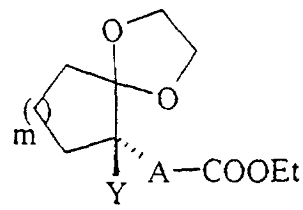

(IX)

m, y und A die oben angegebenen Bedeutungen besitzen. Als Basen kommen beispielsweise Kaliumtert.-butylat. Diazabicyclononan, Diazabicycloundecan oder Natriumhydrid in Frage. Reduktion der Estergruppe, beispielsweise mit Diisobutylaluminiumhydrid und anschließende Oxidation des erhaltenen primären Alkohols z. B. mit Braunstein oder Collinsreagenz führt zum Aldehyd der Formel X.

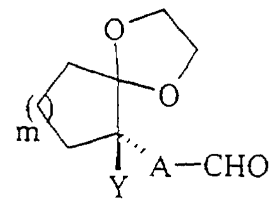

(X)

Die metallorganische Umsetzung des Aldehyds der Formel X mit einem Grignardreagenz der Formel XI, worin B. D

$$X\text{-}Mg\text{-}B\text{-}D\text{-}R_3 \qquad (XI)$$

und $R_3$ die oben angegebenen Bedeutungen aufweisen und X Chlor, Brom oder Jod bedeutet, führt nach Schutz der Hydroxygruppen (beispielsweise durch Acylierung) und gegebenenfalls Diastereomerentrennung zu den Verbindungen der Formel XII.

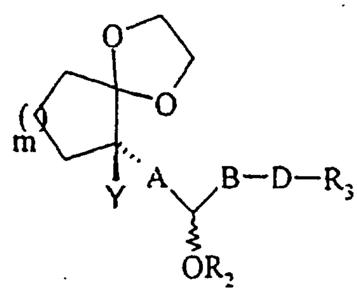

**(XII)**

Die Herstellung der für die metallorganische Umsetzung benötigten Verbindung der Formel XI erfolgt durch Reaktion des entsprechenden terminalen Halogenids mit Magnesium. Durch Umsetzung des Ketals XII mit verdünnter Essigsäure und gegebenenfalls Verseifung des Esters und anschließende Silyletherbildung wird das Keton der Formel XIII erhalten:

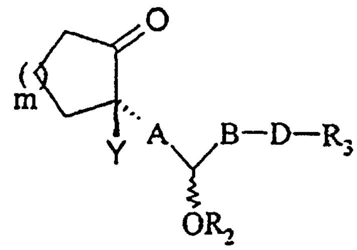 (XIII)

Zu den Verbindungen der Formel XII, worin B eine $CH_2$-Gruppe und D eine -C≡C-Gruppe oder eine $CH=CR_7$-Gruppe bedeutet, kann man gelangen, beispielsweise durch eine metallorganische Umsetzung eines Propargylhalogenids und anschließende Alkylierung mit einem entsprechenden Alkylhalogenid und gegebenenfalls anschließende Lindlar-Hydrierung.

Ein alternativer Aufbau der unteren Kette geht von dem Aldehyd der Formel XIV aus, der aus der Wittig-Horner-Umsetzung des Aldehyds VI und anschließender Reduktion und Oxidation resultierte.

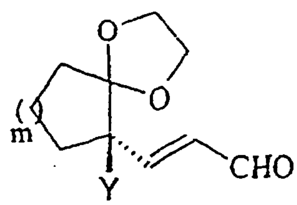 (XIV)

Wittig-Horner-Olefinierung des Aldehyds XIV mit einem Phosphonat der Formel XV

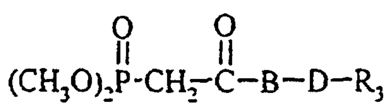 (XV)

und Reduktion des entstandenen Ketons führte dann zum Alkohol der Formel XII der gegebenenfalls in die Diastereomeren getrennt werden kann. Der sich nun anschließende Schutz der Hydroxygruppe. beispielsweise duch Acylierung, Ketalspaltung mit Essigsäure, gegebenenfalls Verseifung des Ester und Silyletherbildung führt zum Keton der Formel XIII.

Die Herstellung der für diese Umsetzung benötigten Phosphonate der allgemeinen Formel XV ist beispielsweise in der DE 42 42 390 beschrieben oder erfolgt in an sich bekannter Weise durch Reaktion eines Alkylhalogenids (herstellbar aus dem entsprechenden Alkohol durch Halogenierung) der allgemeinen Formel XVI

$$\text{Hal-D-}R_3 \qquad \text{(XVI)}$$

mit dem aus Phosphonat der allgemeinen Formel XVII erzeugten Dianion.

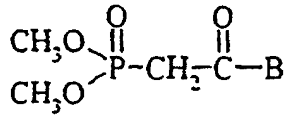 (XVII)

worin B, D und $R_3$ die oben angegebenen Bedeutungen aufweisen.

Ein alternativer Zugang zu den Phosphonaten der allgemeinen Formel XV besteht in der Umsetzung des

Anions von Methytphosphonsäuredimethylester mit einem Ester der allgemeinen Formel XVIII,

$$R_9OOC\text{-}B\text{-}D\text{-}R_3 \qquad (XVIII)$$

'worin $R_3$, B, D die oben angegebenen Bedeutungen aufweisen und $R_9$ eine Alkylgruppe mit 1-5 C-Atomen bedeutet. Diesen Ester kann man beispielsweise durch Alkylierung mit dem entsprechenden Halogenid erhalten.

Der Einbau der chemisch und metabolisch labilen cis-$\Delta^{6,7}$-Doppelbindung des $LTB_4$ in einen cis-1,2-substituierten Cycloalkylring führt zu einer Stabilisierung, wobei insbesondere durch weitere Derivatisierung der funktionellen Gruppen und/oder strukturelle Veränderungen der unteren Seitenkette, $LTB_4$-Derivate erhalten wurden, die als $LTB_4$-Antagonisten wirken können (DE-A 3917597 und DE-A 42 27 790.6 und DE-A 41 08 351 und DE-A 41 39 886.8 und DE-A 42 42 390).

Es wurde nun gefunden, daß durch Einführung einer Alkylgruppe in die 7-Position und durch Einführung einer Doppelbindung in der 5,6-Position (Zählweise beginnend beim Caboxyl-C-Atom mit 1 bei Anwendung der $LTB_4$-Nomenklatur) in derartigen Leukotnen $B_4$-Derivaten eine längere Wirkungsdauer, größere Selektivität und bessere Wirksamkeit erzielt werden kann.

Die Verbindungen der Formel I wirken antientzündlich. antiallergetison und antiproliferativ. Die Verbindungen sind außerdem zur Senkung erhöhter Triglyceridspiegel geeignet. Daneben besitzen sie antimykotische Eigenschaften. Folglich stellen die neuen Leukotrien-$B_4$-Derivate der Formel I wertvolle pharmazeutische Wirkstoffe dar. Die Verbindungen der Formel I sind zur topischen und oralen Applikation geeignet.

Die neuen Leukotrien-$B_4$-Derivate der Formel I eignen sich in Kombination mit den in der galenischen Pharmazie üblichen Hilfs- und Trägermitteln zur lokalen Behandlung von Erkrankungen der Haut, bei denen Leukotriene eine wichtige Rolle spielen. z. B.: Kontaktdermatitis, Ekzemen der verschiedensten Art, Neurodermatosen. Erythrodermie, Pruritis vulvae et ani, Rosacea. Erythermatodes cutaneus. Psonasis. Lichen ruber planus et verrucosus und ähnlichen Hauterkrankungen.

Außerdem sind die neuen Leukotrien-$B_4$-Antagonisten zur Behandlung der multiplen Sklerose und der Symptome des Schocks geeignet.

Die Herstellung der Arzneimittelspezialitäten erfolgt in üblicher Weise. indem man die Wirkstoffe mit geeigneten Zusätzen in die gewünschte Applikationsform. wie zum Beispiel: Lösungen. Salben. Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0,0001% bis 3% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-$B_4$-Derivate auch in Form von Kapseln, Tabletten oder Dragees, die vorzugsweise 0,1 bis 100 mg Wirkstoff enthalten und oral appliziert werden oder in Form von Suspensionen, die vorzugsweise 1-200 mg Wirkstoff pro Dosiseinheit enthalten und rektal appliziert werden auch zur Behandlung von Erkrankungen innerer Organe, bei denen Leukotriene eine wichtige Rolle spielen, wie z. B.: allergische Erkrankungen des Darmtraktes, wie der Colitis ulcerosa und der Colitis granulomatosa.

In diesen neuen Applikationsformen eignen sich die neuen $LTB_4$-Derivate neben der Behandlung von Erkrankungen innerer Organe mit entzündlichen Prozessen auch zur Behandlung von Erkrankungen, bei denen leukotrienabhängig das gesteigerte Wachstum und die Neubildung von Zellen im Vordergrund stehen. Beispiele sind Leukämie (gesteigertes Wachstum weißer Blutkörperchen) oder Artherosklerose (gesteigertes Wachstum glatter Muskelzellen von Blutgefäßen).

Die neuen Leukotrien-$B_4$-Derivate können auch in Kombination, wie z. B. mit Lipoxygenasehemmem, Cyclooxygenasehemmem, Glukokortikoiden, Prostacyclinagonisten. Thromboxanantagonisten, Leukotrien $D_4$-Antagonisten, Leukotrien $E_4$-Antagonisten, Leukotrien-$F_4$-Antagonisten, Phosphodiesterasehemmern, Calciumantagonisten, PAF-Antagonisten oder anderen bekannten Therapieformen der jeweiligen Erkrankungen, verwendet werden.

Die nachfolgenden Ausführungsbeispiele dienen zur näheren Erläuterung des erfindungsgemäßen Verfahrens. In den Beispielen wurden nicht näher charakterisierte Diastereoisomere in der 12-Position als polar beziehungsweise unpolar charakterisiert (z. B. Diastereomer unpol (12)).

**Beispiel 1**

5-{(E)-(2S)-2-((1E,3E)-(5S)-5-Hydroxy-6,6-trimethylen-9-phenyl-1,3-nonadien-8-inyl)-2-methyl-cyclohexyliden}-3-oxa-pentansäure-tert.-butylester

Zu einer Suspension von 501 mg Natriumhydrid (60%ig in Mineralöl) in 7 ml Ethylenglycoldimethylether tropft man bei 0°C unter Stickstoff eine Lösung von 2,5 ml Phosphonoessigsäuretriethylester in 7 ml Ethylenglycoldimethylether und rührt 2 Stunden nach. Nun wird eine Lösung von 3 g des nach Beispiel 1d) hergestellten Ketons in 9 ml Ethylenglycoldimethylether zum Reaktionsgemisch getropft und 18 Stunden bei 50°C gerührt. Anschließend gibt man gesättigte Ammoniumchlorid-Lösung zu und extrahiert mit Ether. Die vereinigten organischen Phasen werden mit Wasser und gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 2.9 g des $\alpha,\beta$-ungesättigten Esters als farbloses Öl.
IR (Film): 2931, 2840, 1716, 1472, 1252, 1174, 994, 836 $cm^{-1}$

Zu einer Lösung von 2,9 g des vorstehend beschriebenen Esters in 35 ml Toluol tropft man bei -70°C unter Stickstoff 9,7 ml Diisobutylaluminiumhydrid (20%ig in Toluol) und rührt 30 Minuten nach. Anschließend werden vorsichtig 3 ml Isopropanol zugetropft und 10 Minuten gerührt. Nun wird mit Wasser versetzt. 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-30% Ether erhält man 2.1 g des Allylalkohols als farbloses Öl.
IR (Film): 3317, 2928, 2840, 2222, 1665, 1598, 1490, 1462, 1360, 1254, 1100, 1057, 993, 835, 775, 754, 691, 525 $cm^{-1}$

Zu einer Lösung von 2,1 g des vorstehend beschriebenen Alkohols in 22 ml Toluol gibt man 3,36 ml Bromessigsäure-tert.-butylester gefolgt von 14 ml 25%iger Natronlauge und 98.8 mg Tetrabutylammoniumhydrogensulfat. Nun wird 16 Stunden unter Stickstoff bei Raumtemperatur gerührt. Anschließend wird mit Ether verdünnt, mit Wasser und gesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-20% Ether erhält man 2,3 g des Esters als farbloses Öl.
IR (Film): 2928, 2840, 1748, 1651, 1598, 1490, 1462, 1392, 1368, 1252, 1223, 1124, 1061, 994, 940, 836, 775, 755, 691 $cm^{-1}$

Zu einer Lösung von 755 mg des vorstehend beschriebenen Esters in 5 ml Tetrahydrofuran gibt man bei Raumtemperatur unter Stickstoff 770 mg Tetrabutylammoniumfluorid x 3 $H_2O$ und rührt 6 Stunden nach. Anschließend verdünnt man mit Ether, wäscht mit Wasser, trocknet die organische Phase über Natriumsulfat und engt nach Filtration im Vakuum ein. Den so erhaltenen Rückstand reinigt man durch Chromatographie an Kieselgel. Mit Hexan / 0-60% Ether erhält man 556 mg der Titelverbindung als farbloses Öl.
IR (Film): 3478, 2928, 2840, 1747, 1650, 1598, 1490, 1442, 1368, 1225, 1122, 994, 943, 845, 756, 693, 526, 466 $cm^{-1}$

Das Ausgangsmaterial für die obige Titelverbindung wird wie folgt hergestellt:

**1a)** (2S)-1-Ethylendioxy-2-hydroxymethyl-2-methyl-cyclohexan

Zu einer Lösung von 3,1 g (2R)-2-Methyl-2-methoxycarbonyl-1-oxo-cyclohexan ((a) K. Sakai et al., *Tetrahedron* **50**, 3315 (1994); b) K. Koga et al., *Tetrahedron* **49**, 1579 (1993)) in 20 ml Toluol gibt man 4,2 g Äthylenglycol gefolgt von 60 mg p-Toluolsulfonsäure und kocht die Mischung 5 Stunden mit einem Wasserabscheider am Rückfluß. Anschließend verdünnt man mit Ether, wäscht mit gesättigter Natriumhydrogencarbonat-Lösung und Wasser, trocknet über Natriumsulfat und engt nach Filtration im Vakuum ein. Den so erhaltenen Rückstand reinigt man durch Chromatographie an Kieselgel. Mit Hexan / Ether (8:2) erhält man 3,8 g des Äthylenketals als farbloses Öl.
IR ($CHCl_3$): 2980, 2930, 2885, 1718. 1460, 950 $cm^{-1}$

Zu einer Lösung von 22,5 g des Äthylenketals in 200 ml Toluol tropft man bei -30°C unter Stickstoff 218 ml Diisobutylaluminiumhydrid (20%ig in Toluol) und rührt 1 Stunde nach. Anschließend wird auf -70°C gekühlt und tropfenweise mit 100 ml Isopropanol versetzt. Nun werden vorsichtig 109 ml Wasser zum Reaktionsgemisch getropft, nach 30 Minuten das Kältebad entfernt und 2 Stunden kräftig gerührt. Der Niederschlag wird abgesaugt, gut mit Essigester gewaschen und das Filtrat im Vakuum eingeengt. Den so erhaltenen Rückstand reinigt man durch Chromatographie an Kieselgel. Mit Hexan / 0-60% Ether erhält man 19,5 g der Titelverbindung als farbloses Öl.
IR (Film): 3452. 2940, 2640, 1464, 1414, 1333, 1276, 1178, 1092, 1031, 949, 877, 799, 745 $cm^{-1}$

**1b)** 3-{(2S)-1,1-Ethylendioxy-2-methyl-cyclohex-2-yl}-(2E)-propen-1-al

Zu einer Lösung von 35,8 g Oxalylchlorid in 210 ml Methylenchlorid wird bei -70°C bis -65°C unter Stickstoff eine Lösung von 47,3 g Dimethylsulfoxid in 160 ml Methylenchlorid vorsichtig zugetropft und 10 Minuten gerührt. In dieses Reaktionsgemisch gibt man nun eine Lösung von 38,9 g des unter Beispiel 1a) hergestellten Alkohols

in 160 ml Methylenchlorid und rührt 2 Stunden bei -70°C nach. Anschließend tropft man 63.4 g Triethylamin zu, rührt 1 Stunde und gibt das Reaktionsgemisch auf Wasser. Nun wird mit Methylenchlorid extrahiert, die vereinigten organischen Phasen werden mit 5%iger Schwefelsäure, gesättigter Natriumhydrogencarbonat-Lösung und mit gesättigter Natriumchlorid-Lösung gewaschen, über Natriumsulfat getrocknet und nach Filtration im Vakuum eingeengt. Man erhält ohne weitere Reinigung 38.4 g des Aldehyds als farbloses Öl.

IR (Film): 2938, 1723, 1449, 1391, 1352, 1276, 1215, 1183, 1092, 1067, 1041, 1019, 949, 881, 789, 752 $cm^{-1}$

Zu 12,8 g Lithiumchlorid in 310 ml Acetonitril tropft man bei Raumtemperatur unter Stickstoff 65,4 g Phosphonoessigsäuretriethylester gefolgt von 39,7 g 1,8-Diazabicyclo
[5.4.0]undec-7-en und rührt 20 Minuten nach. Anschließend werden 38,4 g des vorstehend beschriebenen Aldehyds in 75 ml Acetonitnl zum Reaktionsgemisch getropft und 4,5 Stunden gerührt. Nun werden im Vakuum vorsichtig ca. 2/3 vom Lösungsmittel abdestilliert, der Rückstand auf Wasser gegeben, mit Ether extrahiert, die vereinigten organischen Phasen mit halbkonzentrierter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 42,2 g des Esters als farbloses Öl.

IR (Film): 2937, 2860, 1719, 1648, 1447, 1367, 1311, 1271, 1181, 1090, 1037, 950, 868 $cm^{-1}$

Zu einer Lösung von 42,2 g des vorstehend beschriebenen Esters in 360 ml Toluol tropft man bei -70°C unter Stickstoff 304 ml Diisobutylaluminiumhydrid (20%ig in Toluol) und rührt 45 Minuten nach. Anschließend werden 120 ml Isopropanol langsam zugetropft gefolgt von 150 ml Wasser und 2 Stunden kräftig gerührt. Der Niederschlag wird abgesaugt, gut mit Essigester gewaschen und das Filtrat im Vakuum eingeengt. Den so erhaltenen Rückstand reinigt man durch Chromatographie an Kieselgel. Mit Hexan / 0-60% Ether erhält man 33 g des Alkohols als farbloses Öl.

IR (Film): 3416, 2934, 2865, 1447, 1372, 1180, 1126, 1091. 1023, 960, 880$cm^{-1}$

Zu einer Lösung von 33 g des vorstehend beschriebenen Alkohols in 450 ml Toluol gibt man portionsweise 108 g Mangan(IV)oxid und rührt 4,5 Stunden bei Raumtemperatur unter Stickstoff. Anschließend wird über Celite abgesaugt, mit Essigester nachgewaschen und das Filtrat im Vakuum eingeengt. Man erhält ohne weitere Reinigung 31,4 g der Titelverbindung als schwach gelbgefärbtes Öl.

IR (Film): 2937, 2866, 2728, 1690, 1631, 1462, 1374, 1275, 1181, 1130, 1090, 1022, 962, 881, 797, 733, 595 $cm^{-1}$

**1c)** (5S)-5-Acetoxy-1-{(2S)-1,1-Ethylendioxy-2-methyl-cyclohexyl-2-yl}-6,6-trimethylen-9-phenyl-(1E,3E)-1,3-nonadien-8-in

Zu einer Suspension von 6,8 g Natriumhydrid (65%ig in Mineralöl) in 200 ml Ethylenglycoldimethylether tropft man bei 0°C unter Stickstoff eine Lösung aus 55 g 2-Oxo-3,3-trimethylen-6-phenyl-hex-5-in-phosphonsäuredimethylester in 250 ml Ethylenglycoldimethylether und rührt 1 Stunde nach. Nun tropft man eine Lösung aus 31,4 g des in Beispiel 1b) hergestellten Aldehyds in 250 ml Ethylenglycoldimethylether zu, rührt 30 Minuten bei 0°C und 20 Stunden bei Raumtemperatur. Anschließend wird gesättigte Ammoniumchlorid-Losung zugegeben, mit Ether extrahiert und die vereinigten organischen Phasen mit konzentrierter Natriumchlorid-Lösung gewaschen. Man trocknet über Natriumsulfat und nach Filtration wird im Vakuum eingeengt. Den so erhaitenen Rückstand reinigt man durch Chromatographie an Kieselgel. Mit Hexan / 0-30% Ether erhält man 56 g des Ketons als farbloses Öl.

IR (Film): 2934, 2880, 1680, 1625, 1592, 1490, 1442, 1343, 1180, 1137, 1088, 1010, 962, 880, 756, 692, 527 $cm^{-1}$

Eine Lösung von 56 g des vorstehend beschriebenen Ketons in 650 ml Methylenchlorid und 300 ml Tetrahydrofuran versetzt man bei -60°C unter Stickstoff mit 7,7 g Certrichlorid Heptahydrat und rührt 30 Minuten bei dieser Temperatur. Nun werden 7,8 g Natriumborhydrid hinzu gegeben und 1 Stunde nachgerührt. Anschließend tropft man 100 ml Aceton hinzu, rührt 30 Minuten, stellt den pH-Wert mit Eisessig auf pH7 ein und engt im Vakuum bei 30°C ein. Der Rückstand wird mit Wasser/Ether 1:1 verdünnt, mit Ether extrahiert und die vereinigten organischen Phasen mit halbkonzentrierter Natriumchlorid-Lösung gewaschen. Man trocknet über Natriumsulfat und nach Filtration wird im Vakuum eingeengt. Den so erhaltenen Rückstand trennt man durch mehrfache Chromatographie an Kieselgel. Mit Hexan / 0-40% Ether erhält man zunächst 23,8 g des unpolaren β-konfigurierten Alkohol (5S)-5-Hydroxy-1-{(2S)-1,1-Ethylendioxy-2-methyl-cyclohexyl-2-yl}-6,6-trimethylen-9-phenyl-(1E,3E)-1,3-nonadien-8-in als farbloses Öl, sowie 26 g des polaren α-konfigurierten Alkohols (5R)-5-Hydroxy-1-{(2S)-1,1-ethylendioxy-2-methyl-cyclohexyl-2-yl}-6,6-trimethylen-9-phenyl-(1E,3E)-13-nonadien-8-in als farbloses Öl.

IR (Film): (unpolarer Alkohol) 2463, 2933, 2830, 1598, 1490, 1442, 1180, 1089, 994, 950, 880, 756, 692, 526 $cm^{-1}$

Zu einer Lösung von 23.3 g des vorstehend hergestellten unpolaren Alkohols in 60 ml Pyridin gibt man 30 ml Essigsäureanhydrid und rührt unter Stickstoff 16 Stunden bei Raumtemperatur. Anschließend wird mehrmals im Vakuum unter Toluol Zusatz eingeengt. Den so erhaltenen Rückstand reinigt man durch Chromatographie an Kieselgel. Mit Hexan / 0-30% Ether erhält man 23.95 g der Titelverbindung als farbloses Öl.

IR (Film): 2934, 2880, 1732, 1653, 1598, 1490, 1442, 1370, 1236, 1180, 1090, 1020, 995, 962, 880, 797, 757, 692, 526 $cm^{-1}$

**1d)** (5S)-5-Tert.-butyl-dimethylsilyloxy-1-{(2S)-1-oxo-2-methyl-cyclohexyl-2-yl}-6,6-trimethylen-9-phenyl-(1E.3E)-1.3-nonadien-8-in

21 g des unter Beispiel 1c) hergestellten Acetates werden in 64 ml Tetrahydrofuran und 500 ml Gemisch (65:35:10 / Essigsäure : Wasser : Tetrahydrofuran) gelöst und unter Stickstoff bei 45°C 3.5 Stunden gerührt. Anschließend wird das Reaktionsgemisch im Eisbad gekühlt, vorsichtig mit 600 ml eiskalter 8 molarer Natronlauge auf pH8 gebracht und 30 Minuten nachgerührt. Nun wird mit Ether extrahiert. die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-20% Ether erhält man 15,4 g des Ketons als farbloses Öl.

IR (Film): 2934, 2880, 1735, 1708, 1490, 1442, 1371, 1235, 1118, 992, 757, 692 $cm^{-1}$

Zu einer Lösung von 8,3 g des vorstehend beschriebenen Ketons in 350 ml Methylenchlorid gibt man 5,7 g Kaliumcarbonat und rührt unter Stickstoff bei Raumtemperatur 16 Stunden. Anschließend wird mit Ether verdünnt, mit Wasser und gesättigter Natriumchlorid-Lösung gewaschen, die organischen Phase über Natriumsulfat getrocknet und nach Filtration im Vakuum eingeengt. Den so erhaltenen Rückstand reinigt man durch Chromatographie an Kieselgel. Mit Hexan / 0-40% Ether erhält man 7,4 g des Alkohols als farbloses Öl.

IR (Film): 3478, 2933, 2862, 2362, 1704, 1598, 1570, 1490, 1443, 1372, 1242, 1090, 1045, 992, 911, 860, 802, 757, 692, 526 $cm^{-1}$

Zu einer Lösung von 7,4 g des vorgehend beschriebenen Alkohols in 60 ml N,N-Dimethylformamid gibt man bei O°C unter Stickstoff 5,6 g Imidazol gefolgt von 6,2 g tert-Butyldimethylsilylchlorid und rührt 15 Minuten bei 0°C und 16 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-20% Ether erhält man 9.3 g der Titelverbindung als farbloses Öl.

IR (Film): 2930, 2856, 2220, 1710, 1653, 1598, 1489, 1442, 1360, 1254, 1102, 1062, 991, 836, 776, 756, 691, 523 $cm^{-1}$

**Beispiel 2**

5-{(E)-(2S)-2-((1E,3E)-(5S)-5-Hydroxy-6,6-trimethylen-9-phenyl-1,3-nonadien-8-inyl)-2-methyl-cyclohexyliden}-3-oxa-pentansäure

Zu einer Lösung von 546 mg 5-{(E)-(2S)-2-((1E,3E)-(5S)-5-Hydroxy-6,6-trimethylen-9-phenyl-1,3-nonadien-8-inyl)-2-methyl-cyclohexyliden}-3-oxa-pentansäure-tert.butylester (hergestellt in Beispiel 1) in 5,6 ml Tetrahydrofuran und 5,6 ml Methanol gibt man 5,6 ml 0.5 molare Lithiumhydroxid-Lösung gefolgt von 78 mg Lithiumhydroxid und rührt 18 Stunden bei Raumtemperatur. Anschließend wird mit Wasser verdünnt. mit 1 molarer Salzsäure auf pH5 angesauert. mit Essigester extrahiert, über Natriumsulfat getrocknet und nach Filtration im Vakuum eingeengt. Den so erhaltenen Rückstand reinigt man durch Chromatographie an Kieselgel. Mit Hexan / 0-70% Essigester erhält man 9,3 g der Titelverbindung als farbloses Öl. Diese Substanz ist die bevorzugte Ausführungsform. IR (Film): 3440, 2915, 2860, 1740, 1655, 1600, 1490, 1440, 1370, 1240, 1110, 990, 755, 690, 530 $cm^{-1}$

**Beispiel 3**

5-{(E)-(2S)-2-((1E,3E)-(5R)-5-Hydroxy-7.7-trimethylen-10-phenyl-1.3-decadien-9-inyl)-2-methyl-cyclohexyliden}-3-oxa-pentansäure-tert.-butylester

Zu einer Suspension von 488 mg Natriumhydrid (60%ig in Mineralöl) in 12 ml Ethytenglycoldimethylether tropft man bei O°C unter Stickstoff eine Lösung von 2,7 g Phosphonoessigsäuretriethylester in 12 ml Ethylenglycoldimethylether und rührt 1 Stunden nach. Nun wird eine Lösung von 3 g des nach Beispiel 3c) hergestellten Ketons in 12 ml Ethylenglycoldimethylether zum Reaktionsgemisch getropft und 24 Stunden bei 50°C gerührt. Anschließend gibt man gesättigte Ammoniumchlorid-Losung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 3,4 g des Esters als farbloses Öl.

IR (Film): 2930, 2860, 1720, 1640, 1490, 1480, 1470, 1460, 1380, 1250, 1180, 1130, 1070, 1040, 990, 940, 840, 780, 755, 690, 530 $cm^{-1}$

Zu einer Lösung von 3,5 g des vorstehend beschriebenen Esters in 37 ml Toluol tropft man bei -70°C unter Stickstoff 11,1 ml Diisobutylaluminiumhydrid (20%ig in Toluol) und rührt 30 Minuten nach. Anschließend werden vorsichtig 3,7 ml Isopropanol zugetropft und 5 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-70% Ether erhält man 3 g des Allylalkohols als farbloses Öl.
IR (Film): 3330, 2928, 2855, 1656, 1598, 1490, 1462, 1442, 1360, 1254, 1070, 993, 836, 809, 775, 755, 691 $cm^{-1}$

Zu einer Lösung von 2,6 g des vorstehend beschriebenen Alkohols in 24,6 ml Toluol gibt man 4,9 g Bromessigsäure-tert.-butylester gefolgt von 15 ml 25%iger Natronlauge und 119 mg Tetrabutylammoniumhydrogensulfat. Nun wird 24 Stunden unter Stickstoff bei Raumtemperatur gerührt. Anschließend wird mit Ether verdünnt, mit halbgesättigter Natriumchlorid-Lösung gewaschen die organische Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 3,8 g des Ethers als farbloses Öl.
IR (Film): 2929, 2860, 1749, 1368, 1297, 1256, 1124, 994, 836, 775, 756 $cm^{-1}$

Zu einer Lösung von 3,8 mg des vorstehend beschriebenen Ethers in 155 ml Tetrahydrofuran gibt man bei Raumtemperatur unter Stickstoff 9,7 g Tetrabutylammoniumfluorid x 3 $H_2O$ und rührt 4 Stunden nach. Anschließend verdünnt man mit Ether, wäscht mit halbgesättigter Natriumchlorid-Lösung, trocknet die organische Phase über Natriumsulfat und engt nach Filtration im Vakuum ein. Den so erhaltenen Rückstand reinigt man durch Chromatographie an Kieselgel. Mit Hexan / 0-70% Ether erhält man 2,3 g der Titelverbindung als farbloses Öl.
IR (Film): 3460, 2980, 2940, 2860, 1750, 1650, 1600, 1490, 1440, 1390, 1370, 1300, 1230, 1160, 1120, 990, 940, 850, 760, 690, 590, 530 $cm^{-1}$

Das Ausgangsmaterial für die obige Titelverbindung wird wie folgt hergestellt:

**3a)** <u>2-Oxo-4,4-trimethylen-7-phenyl-hept-6-in-phosphonsäuredimethylester</u>

Zu einer Lösung von 56,45 g 3,3-Trimethylen-6-phenyl-5-insäuremethylester (beschrieben in der Deutschen Offenlegungsschrift DE 4242390 A1) in 420 ml Toluol gibt man bei -70°C unter Stickstoff 412 ml Diisobutylaluminiumhydrid (20%ig in Toluol) und rührt 40 Minuten nach. Anschließend werden vorsichtig 105 ml Isopropanol zugetropft und 10 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-50% Ether erhält man 97,2 g des Alkohols als farbloses Öl.

Zu einer Lösung von 97.2 g des vorstehend beschriebenen Alkohols in 247 ml Pyridin gibt man bei 0°C unter Stickstoff 120,2 g p-Toluolsulfonsäurechlorid. rührt 4 Stunden nach und läßt 18 Stunden bei ca. 4°C stehen. Anschließend wird mit Eiswasser versetzt und 2 Stunden bei Raumtemperatur gerührt. Nun wind mit Ether verdünnt, mit Wasser, 10%iger Schwefelsäure, Wasser, gesättigter Natriumhydrogencarbonat-Lösung und mit gesättigter Natriumchlorid-Lösung gewaschen. Die organische Phase wird über Natriumsulfat getrocknet und nach Filtration im Vakuum eingeengt. Man erhält ohne weitere Reinigung 172 g des Tosylats als farbloses Öl.
IR (Film): 3020, 2980. 2960, 1600, 1490, 1360, 1190, 1175, 1100, 960, 840, 810, 690, 660 $cm^{-1}$

Zu einer Lösung von 172 g des vorstehend beschriebenen Tosylats in 922 ml Dimethylsulfoxid gibt man 28,5 g Natriumcyanid und rührt 2,5 Stunden bei 80°C. Nach abkühlen wird auf Eiswasser gegeben, mit Ether extrahiert, die vereinigten organischen Phasen mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-50% Ether erhält man 91.6 g des Nitrils als farbloses Öl.

Zu einer Lösung von 91.6 g des vorstehend beschriebenen Nitrils in 650 ml Toluol gibt man bei -70°C unter Stickstoff 547 ml Diisobutylaluminiumhydrid (20%ig in Toluol) und rührt 30 Minuten nach. Anschließend werden vorsichtig 55 ml Isopropanol zugetropft und 10 Minuten gerührt. Nun wird mit gesättigter Ammoniumchlorid-Lösung versetzt, 6 Stunden bei Raumtemperatur gerührt, der weiße Niederschlag abfiltriert und gut mit Essigester gewaschen. Das Filtrat wird im Vakuum eingeengt. Man erhält ohne weitere Reinigung 107 g des Aldehyds als farbloses Öl.
IR (Film): 2980, 2940, 2740, 1720, 1600, 1490, 1445, 1180, 1070, 915, 690, $cm^{-1}$

Zu einer Lösung von 53,5 g des vorstehend beschriebenen Aldehyds in 594 ml Aceton tropft man bei -5°C 141,5 ml Jones-Reagenz und rührt kräftig 1 Stunde nach. Anschließend werden 240 ml Isopropanol addiert und 30 Minuten bei Raumtemperatur gerührt. Nun wird über Celite abgesaugt und mit Ether nachgewaschen. Das Filtrat wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-50% Ether erhält man 60 g der Säure als farbloses Öl. Zu einer Lösung von 71 g der vorstehend beschriebenen Säure in 437 ml Aceton gibt man 96,7 g Kaliumcarbonat gefolgt von 43,3 ml Methyliodid und rührt 20 Stunden bei Raumtemperatur. Anschließend wird über Celite abgesaugt, mit Essigester nachgewaschen und im Vakuum eingeengt. Der Rückstand wird mit Ether verdünnt, mit Wasser, gesättigter Natriumchlorid-Lösung gewaschen, über Natriumsulfat getrocknet und nach Filtration im Vakuum eingeengt. Den so erhaltenen Rückstand

reinigt man durch Chromatographie an Kieselgel. Mit Hexan / 0-30% Ether erhält man 54,3 g des Esters als farbloses Öl.

19,6 ml Methanphosphonosäuredimethyester werden in 243 ml Tetrahydrofuran gelöst bei -70°C tropfenweise mit n-Butyllithium versetzt und 1 Stunde nachgerührt. Nun wird eine Lösung von 18 g des vorstehend beschriebenen Esters in 44 ml Tetrahydrofuran zum Reaktionsgemisch getropft und 5 Stunden nachgerührt. Anschließend werden 13,3 ml Essigsäure addiert und das Reaktionsgemisch über Nacht bei ca. 6°C stehen gelassen. Nach Einengen im Vakuum wird mit Wasser versetzt, mit Methylenchlorid extrahiert, die organische Phase über Natriumsulfat getrocknet und nach Filration im Vakuum eingeengt. Den so erhaltenen Rückstand reinigt man durch Chromatographie an Kieselgel. Mit Hexan / 0-100% Essigester erhält man 17,9 g des Phosphonats als farbloses Öl.

**3b)** (5R)-5-Benzoyloxy-1-{(2S)-1.1-ethylendioxy-2-methyl-cyclohexyl-2-yl}-7.7-trimethylen-10-phenyl-(1E,3E)-1,3-decadien-9-in

Zu einer Suspension von 2,1 g Natriumhydrid (60%ig in Mineralöl) in 65 ml Ethylenglycoldimethylether tropft man bei O°C unter Stickstoff eine Lösung von 17,88 g des vorstehend beschriebenen Phosphonats in 98 ml Ethylenglycoldimethylether und rührt 1 Stunde nach. Nun wird eine Lösung von 9,78 g des in 1b) beschriebenen Aldehyds in 98 ml Ethytenglycoldimethylether zum Reaktionsgemisch getropft, 30 Minuten bei 0°C und 20 Stunden bei Raumtemperatur gerührt. Anschließend gibt man gesättigte Ammoniumchlorid-Lösung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-40% Ether erhält man 17,4 g des Ketons als farbloses Öl.
IR (Film): 2940, 2860, 1680, 1655, 1630, 1590, 1490, 1440, 1355, 1180, 1090, 950, 880, 755, 690, 525 $cm^{-1}$

Zu einer Lösung von 17,4 g des vorstehend beschriebenen Ketons in 330 ml Methanol und 33 ml Tetrahydrofuran gibt man bei -70°C unter Stickstoff 2.3 g Certrichlorid Heptahydrat und rührt 30 Minuten nach. Nun werden 2,4 g Natriumborhydrid hinzu gegeben und 1 Stunde nachgerührt. Anschließend tropft man 20 ml Aceton hinzu, rührt 15 Minuten bei Raumtemperatur, stellt den pH-Wert mit Eisessig auf pH7 ein und engt im Vakuum bei 30°C ein. Der Rückstand wird mit Wasser/Ether 1:1 verdünnt, mit Ether extrahiert und die vereinigten organischen Phasen mit Wasser und gesättigter Natriumchlorid-Lösung gewaschen. Man trocknet über Natriumsulfat und engt nach Filtration im Vakuum ein. Den so erhaltenen Rückstand trennt man durch mehrfache Chromatographie an Kieselgel. Mit Hexan / 0-40% Ether-Gemischen erhält man zunächst 7,5g des unpolaren β-konfigurierten Alkohol (5R)-5-Hydroxy-1-{(2S)-1,1-ethylendioxy-2-methyl-cyclohexyl-2-yl}-7,7-trimethylen-10-phenyl-(1E,3E)-1,3-decadien-9-in sowie 8,2 g des polareren α-konfigurierten Alkohols (5S)-5-Hydroxy-1-{(2S)-1,1-ethylendioxy-2-methylcyclohexyl-2-yl}-7,7-trimethylen-10-phenyl-(1E,3E)-1,3-decadien-9-in als farbloses Öl.
IR (Film): (unpolarer Alkohol) 3460, 2940, 2865, 1600, 1490, 1440, 1275, 1180, 1090, 995, 950, 880, 760, 695, 530 $cm^{-1}$
IR (Film): (polarer Alkohol) 3450, 2940, 2860, 1600, 1480, 1440, 1275, 1180, 1090, 995, 880, 760, 695, 530 $cm^{-1}$

Zu einer Lösung von 7,5 g des vorstehend beschriebenen unpolaren Alkohols in 32,6 ml Pyridin gibt man bei 0°C 4,2 ml Benzoylchlorid und rührt 18 Stunden bei Raumtemperatur nach. Anschließend wird mit Eiswasser versetzt, 2 Stunden gerührt, mit Ether verdünnt. mit Wasser, 10%iger Schwefelsäure, Wasser, gesättigter Natriumhydrogencarbonat-Lösung und Wasser gewaschen. Nach Trocknung über Natriumsulfat und Filtration wird im Vakuum eingeengt. Den so erhaltenen Rückstand reinigt man durch Chromatographie an Kieselgel. Mit Hexan / 0-35% Ether erhält man 8,8 g des Benzoats als farbloses Öl.
IR (Film): 2933, 1716, 1600, 1490, 1450, 1314, 1268, 1178, 1109, 1070, 1026, 994, 962, 890, 757, 712, 692, 526 $cm^{-1}$

**3c)** (5R)-5-Tert.-butyl-dimethylsilyloxy-1-{(2S)-1-oxo-2-methyl-cyclohexyl-2-yl}-7,7-trimethylen-10-phenyl-(1E,3E)-1,3-decadien-9-in

3,9 g des unter Beispiel 3b) hergestellten Esters werden in 15,6 ml Tetrahydrofuran und 156 ml Gemisch (65:35:10 / Essigsäure : Wasser : Tetrahydrofuran) gelöst und unter Stickstoff bei 40°C 24 Stunden gerührt. Anschließend wird das Reaktionsgemisch im Eisbad gekühlt, vorsichtig mit 218 ml eiskalter 8 molarer Natronlauge auf pH8 gebracht und 30 Minuten bei Raumtemperatur nachgerührt. Nun wird mit Ether verdünnt, mit Wasser gewaschen, über Natriumsulfat getrocknet und nach Filtration im Vakuum eingeengt. Man erhält ohne weitere Reinigung 3.9 g des Ketons als farbloses Öl. Zur einer Lösung von 3,9 g des vorstehend beschriebenen Ketons in 117 ml Methanol gibt man 2,3 g Kaliumcarbonat und rührt 24 Stunden bei Raumtemperatur. Anschließend wird mit Ether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-70% Ether erhält man 2,45 g des Alkohols als farbloses Öl.
IR (Film): 3440, 2925, 2860, 1710, 1600, 1490, 1450, 990, 910, 755, 690, 550 $cm^{-1}$

Zu einer Lösung von 2.79 g des vorstehend beschriebenen Alkohols in 26,8 ml N,N-Dimethylformamid gibt

man bei O°C unter Stickstoff 2 g Imidazol gefolgt von 2,2 g tert-Butyldimethylsilylchlorid, rührt 15 Minuten bei 0°C und 24 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-20% Ether erhält man 3.2 g der Titelverbindung als farbloses Öl.

IR (Film): 2929, 2856, 2361, 1711, 1598, 1490, 1462, 1360, 1255, 1070, 991, 836, 775, 756, 692 $cm^{-1}$

**Beispiel 4**

5-{(E)-(2S)-2-((1E,3E)-(5R)-5-Hydroxy-7,7-trimethylen-10-phenyl-1,3-decadien-9-inyl)-2-methyl-cyclohexyliden}-3-oxa-pentansäure

Zu einer Lösung von 2,1 g des nach Beispiel 3) hergestellten Esters in 21 ml Methanol gibt man 20,3 ml 1 molare Natronlauge und rührt 24 Stunden bei Raumtemperatur nach. Anschließend wird mit 10%iger Schwefelsäure auf pH5 angesäuert, mit Essigester extrahiert, die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-20% Ether erhält man 3,2 g der Titelverbindung als farbloses Öl.

IR (Film): 3445, 2930, 2860, 1740, 1600, 1490, 1445, 1375, 1240, 1115, 1050, 760, 690, 550 $cm^{-1}$

**Beispiel 5**

5-{(E)-(2S)-2-((1E,3E)-(5S)-5-Hydroxy-7.7-trimethylen-10-phenyl-1.3-decadien-9-inyl)-2-methyl-cyclohexyliden}-3-oxa-pentansäure-tert.-butylester

Zu einer Suspension von 252 mg Natriumhydrid (60%ig in Mineralöl) in 6,3 ml Ethylenglycoldimethylether tropft man bei O°C unter Stickstoff eine Lösung von 1,4 g Phosphonoessigsäuretriethylester in 6,3 ml Ethylenglycoldimethylether und rührt 1 Stunde nach. Nun wird eine Lösung von 1,55 g des nach Beispiel 5b) hergestellten (5S)-5-Tert-butyl-dimethylsilyloxy-1-{(2S)-1-oxo-2-methyl-cyclohexyl-2-yl}-77-trimethylen-10-phenyl-(1E,3E)-1,3-decadien-9-in in 6,3 ml Ethylenglycoldimethylether zum Reaktionsgemisch getropft und 24 Stunden bei 50°C gerührt. Anschließend gibt man gesättigte Ammoniumchlorid-Lösung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 1,5 g des Esters als farbloses Öl.

IR (Film): 2930, 2860, 1720, 1635, 1600, 1490, 1440, 1380, 1250, 1175, 990, 835, 775, 755, 690, 550 $cm^{-1}$

Zu einer Lösung von 1,5 g des vorstehend beschriebenen Esters in 16 ml Toiuol tropft man bei -70°C unter Stickstoff 4,9 ml Diisobutylaluminiumhydrid (20%ig in Toluol) und rührt 30 Minuten nach. Anschließend werden vorsichtig 1,6 ml Isopropanol zugetropft und 5 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-70% Ether erhält man 1,38 g des Allylalkohols als farbloses Öl.

IR (Film): 3331, 2928, 2855, 1656, 1598, 1490, 1462, 1442, 1360, 1255, 1070, 994, 836, 809, 775, 755, 691 $cm^{-1}$

Zu einer Lösung von 1,38 g des vorstehend beschriebenen Alkohols in 13 ml Toluol gibt man 2,6 g Bromessigsäure-tert.-butylester gefolgt von 8 ml 25%iger Natronlauge und 63 mg Tetrabutylammoniumhydrogensulfat. Nun wird 24 Stunden unter Stickstoff bei Raumtemperatur gerührt, Anschließend, mit Ether verdünnt, mit halbgesättigter Natriumchlorid-Lösung gewaschen. die organische Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 1,82 g des Ethers als farbloses Öl.

IR (Film): 2928, 2856, 1749, 1590, 1472, 1392, 1368, 1297, 1255, 1223, 1124, 994, 939, 836, 775, 756, 692 $cm^{-1}$

Zu einer Lösung von 1.82 g des vorstehend beschriebenen Ethers in 73 ml Tetrahydrofuran gibt man bei Raumtemperatur unter Stickstoff 4,5 g Tetrabutylammoniumfluorid x 3 $H_2O$ und rührt 4 Stunden nach. Anschließend verdünnt man mit Ether, wäscht mit halbgesättigter Natriumchlorid-Lösung, trocknet die organische Phase über Natriumsulfat und engt. nach Filtration, im Vakuum ein. Den so erhaltenen Rückstand reinigt man durch Chromatographie an Kieselgel. Mit Hexan / 0-70% Ether erhält man 1,25 g der Titelverbindung als farbloses Öl.

IR (Film): 3470, 2930, 2860, 1750, 1650, 1600, 1450, 1370, 1225, 1160, 1120, 990, 845, 760, 690, 550 $cm^{-1}$

Das Ausgangsmaterial für die obige Titelverbindung wird wie folgt hergestellt:

**5a)** (5S)-5-Benzoyloxy-1-{(2S)-1.1-ethylendioxy-2-methyl-cyclohexyl-2-yl}7,7-trimethylen-10-phenyl-(1E,3E)-1,3-decadien-9-in

Zu einer Lösung von 8,24 g des in Beispiel 5) hergestellten polaren Alkohols in 35,8 ml Pyridin gibt man bei 0°C 4,6 ml Benzoylchlorid und rührt 24 Stunden bei Raumtemperatur nach. Anschließend wird mit Eiswasser versetzt, 2 Stunden gerührt, mit Ether verdünnt, mit Wasser, 10%iger Schwefelsäure, Wasser, gesättigter Natriumhydrogencarbonat-Lösung und Wasser gewaschen. Nach Trocknung über Natriumsulfat und Filtration wird im Vakuum eingeengt. Den so erhaltenen Rückstand reinigt man durch mehrmalige Chromatographie an Kieselgel. Mit Hexan / 0-20% Ether erhält man 4,05 g des Benzoats als farbloses Öl.

**5b)** (5S)-5-Tert.-butyl-dimethylsilyloxy-1-{(2S)-1-oxo-2-methyl-cyclohexyl-2-yl}-7,7-trimethylen-10-phenyl-(1E, 3E)-1,3-decadien-9-in

21 g des unter Beispiel 5a) hergestellten Benzoats werden in 12 ml Tetrahydrofuran und 118 ml Gemisch (65: 35:10 / Essigsäure : Wasser : Tetrahydrofuran) gelöst und unter Stickstoff bei 40°C 24 Stunden gerührt. Anschließend wird das Reaktionsgemisch im Eisbad gekühlt, vorsichtig mit 165 ml eiskalter 8 molarer Natronlauge auf pH8 gebracht und 30 Minuten nachgerührt. Nun wird mit Ether extrahiert, die vereinigten organischen Phasen mit gesättigter Natriumchlorid-Lösung gewaschen, über Natriumsulfat getrocknet und nach Filtration im Vakuum eingeengt. Man erhält ohne weitere Reinigung 2.95 g des Ketons als farbloses Öl.

Zur einer Lösung von 2,95 g des vorstehend beschriebenen Ketons in 89 ml Methanol gibt man 1,7 g Kaliumcarbonat und rührt 24 Stunden bei Raumtemperatur. Anschließend wird mit Ether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-70% Ether erhält man 1,12 g des Alkohols als farbloses Öl.

IR (Film): 3450, 2930, 2860, 1740, 1710, 1600, 1490, 1440, 1425, 1370, 1310, 1240, 1120, 1060, 990, 910, 760, 690. 530 $cm^{-1}$

Zu einer Lösung von 1,5 g des vorstehend beschriebenen Alkohols in 14 ml N,N-Dimethylformamid gibt man bei O°C unter Stickstoff 1,1 g Imidazol gefolgt von 1,2 g tert.-Butyldimethylsilylchlorid und rührt 15 Minuten bei 0°C und 24 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-20% Ether erhält man 1,55 g der Titelverbindung als farbloses Öl.

IR (Film): 2929, 2856, 2361, 1711, 1598, 1490, 1462, 1443, 1360, 1255, 1070, 992, 836, 775, 755, 692 $cm^{-1}$

**Beispiel 6**

5-{(E)-(2S)-2-((1E,3E)-(5S)-5-Hydroxy-7.7-trimethylen-10-phenyl-1.3-decadien-9-inyl)-2-methyl-cyclohexyliden}-3-oxa-pentansäure

Zu einer Lösung von 1,15 g des nach Beispiel 5) hergestellten Esters in 12 ml Methanol und 12 ml Tetrahydrofuran gibt man 11 ml 1 molare Natronlauge und rührt 24 Stunden bei Raumtemperatur nach. Anschließend wird mit 10%iger Schwefelsäure auf pH5 eingestellt, mit Essigester extrahiert, die vereinigten organischen Phasen mit gesättigter Natriumchlorid-Lösung gewaschen, über Natnumsulfat getrocknet und nach Filtration im Vakuum eingeengt. Den so erhaltenen Rückstand reinigt man durch Chromatographie an Kieselgel. Mit Hexan / 0-50% Essigester erhält man 1 g der Titetverbindung als farbloses Öl.

IR (Film): 3440, 2930, 2855, 1735, 1655, 1600, 1490, 1440, 1370, 1240, 1115, 1050, 990, 755, 690 $cm^{-1}$

**Beispiel 7**

5-{(E)-(2S)-2-2((1E,3E)-(5-R)-5-Hydroxy-8.8,trimethylen-11-phenyl-1,3-undecadien-10-inyl)-2-methyl-cyclohexyliden}-3-oxy-pentansäure-tert.-butylester

Zu einer Suspension von 411 mg Natriumhydrid (60%ig in Mineralöl) in 10 ml Ethylenglycoldimethylether tropft man bei O°C unter Stickstoff eine Lösung von 2.3 g Phosphonoessigsäuretriethylester in 10 ml Ethylenglycoldimethylether und rührt 1 Stunde

bei Raumtemperatur nach. Nun wird eine Lösung von 2,6 g des nach Beispiel 7c) hergestellten Ketons in 10 ml Ethylenglycoldimethylether zum Reaktionsgemisch getropft und 24 Stunden bei 50°C gerührt. Anschließend gibt man auf gesättigte Ammoniumchlorid-Lösung und verdünnt mit Ether. Die organische Phase wird mit halbgesättigter Natriumchlorid-Lösung gewaschen, über Natriumsulfat getrocknet und nach Filtration im Vakuum eingeengt. Den so erhaltenen

Rückstand reinigt man durch Chromatographie an Kieselgel. Mit Hexan / 0-10% Ether erhält man 2,74 g des Esters als farbloses Öl.

IR (Film): 2930, 2850, 1715, 1630, 1600, 1490, 1440, 1375, 1250, 1170, 990, 835, 775, 755, 690 $cm^{-1}$

Zu einer Lösung von 2,39 g des vorstehend beschriebenen Esters in 25 ml Toluol tropft man bei -70°C unter Stickstoff 7,6 ml Diisobutylaluminiumhydrid (20%ig in Toluol) und rührt 30 Minuten nach. Anschließend werden vorsichtig 2.5 ml Isopropanol zugetropft und 5 Minuten gerührt. Nun wird mit Wasser versetzt, 2 Stunden bei Raumtemperatur gerührt. der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-70% Ether erhält man 1,95 g des Allylalkohols als farbloses Öl.

IR (Film): 3260, 2928, 2855, 1490, 1442, 1254, 1070, 993, 835, 775, 755, 691 $cm^{-1}$

Zu einer Lösung von 1.95 g des vorstehend beschriebenen Alkohols in 18,5 ml Toluol gibt man 3,6 g Bromessigsäure-tert.-butylester gefolgt von 11,4 ml 25%iger Natronlauge und 87 mg Tetrabutylammoniumhydrogensulfat. Nun wird 24 Stunden unter Stickstoff bei Raumtemperatur gerührt. Anschließend wird mit Ether verdünnt, mit halbgesättigter Natriumchlorid-Lösung gewaschen. die organische Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 3,73 g des Ethers als farbloses Öl.

IR (Film): 2930, 2856, 1748, 1490, 1456, 1393, 1369, 1297, 1256, 1161, 994, 951, 836, 775, 756, 692 $cm^{-1}$

Zu einer Lösung von 3.73 g des vorstehend beschriebenen Ethers in 145 ml Tetrahydrofuran gibt man bei Raumtemperatur unter Stickstoff 9.1 g Tetrabutylammoniumfluorid x 3 $H_2O$ und rührt 4 Stunden nach. Anschließend verdünnt man mit Ether, wäscht mit halbgesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-70% Ether erhält man 1,62 g der Titelverbindung als farbloses Öl.

IR (Film): 3460, 2930, 2855, 1745, 1650, 1600, 1490, 1440, 1370, 1225. 1160, 1120, 990, 845, 755, 690 $cm^{-1}$

Das Ausgangsmaterial für die obige Titelverbindung wird wie folgt hergestellt:

**7a)** 2-Oxo-5,5-trimethylen-8-phenyl-oct-6-in-phosphonsäuredimethylester

Zu einer Lösung von 36,25 g 3.3-Trimethylen-6-phenyl-5-insäuremethylester (beschrieben in der deutschen Offenlegungsschrift DE 4242390 A1) in 267 ml Toluol tropft man bei -70°C unter Stickstoff 261 ml Diisobutylaluminiumhydrid (20%ig in Toluol) und rührt 30 Minuten nach. Anschließend werden vorsichtig 67 ml Isopropanol zugetropft und 5 Minuten gerührt. Nun wird mit Wasser versetzt, 3 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-80% Ether erhält man 30,4 g des Alkohols als farbloses Öl.

Zu einer Lösung von 30,4 g des vorstehend beschriebenen Alkohols in 88 ml Pyridin gibt man bei 0°C 40,6 g p-Toluolsulfonsäurechlorid, rührt 4 Stunden nach und läßt 20 Stunden bei ca. 6°C stehen. Anschließend wird mit Eiswasser versetzt und 2 Stunden bei Raumtemperatur gerührt. Nun wird mit Ether verdünnt, mit Wasser. 10%iger Schwefelsäure. Wasser, gesättigter Natriumhydrogencarbonat-Lösung und mit gesättigter Natriumchlorid-Lösung gewaschen. Die organische Phase wird über Natriumsulfat getrocknet und nach Filtration im Vakuum eingeengt. Man erhält ohne weitere Reinigung 49 g des Tosylats als farbloses Öl.

Zu einer Lösung von 49 g des vorstehend beschriebenen Tosylats in 254 ml Dimethylsulfoxid gibt man 7,8 g Natriumcyanid und rührt 3,5 Stunden bei 80°C. Nach abkühlen wird auf Eiswasser gegeben, mit Ether extrahiert, die vereinigten organischen Phasen mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-50% Ether erhält man 28,3 g des Nitrils als farbloses Öl.

IR (Film): 3060, 2990, 2930, 2250, 1600, 1570, 1490, 1440, 1425, 1385, 1070, 1030, 915, 760, 690, 525 $cm^{-1}$

Zu einer Lösung von 28,3 g des vorstehend beschriebenen Nitrils in 270 ml Toluol tropft man bei -70°C unter Stickstoff 165,8 ml Diisobutylaluminiumhydrid (20%ig in Toluol) und rührt 40 Minuten nach. Anschließend werden vorsichtig 20 ml Isopropanol zugetropft und 10 Minuten gerührt. Nun wird mit gesättigter Ammoniumchlorid-Lösung versetzt, 6 Stunden bei Raumtemperatur gerührt, der weiße Niederschlag abfiltriert und gut mit Essigester gewaschen. Das Filtrat wird im Vakuum eingeengt. Man erhält ohne weitere Reinigung 32 g des Aldehyds als farbloses Öl.

Zu einer Lösung von 32 g des vorstehend beschriebenen Aldehyds in 400 ml Aceton tropft man bei 0°C 79,4 ml Jones-Reagenz und rührt kräftig 1 Stunden nach. Anschließend werden 120 mf Isopropanol addiert und 30 Minuten bei Raumtemperatur gerührt. Nun wird über Celite abgesaugt und mit Ether nachgewaschen. Das Filtrat wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Ether erhält man 13,87 g der Säure als farbloses Öl.

Zu einer Lösung von 18,18 g der vorstehend beschriebenen Säure in 106 ml Aceton gibt man 23.33 g Kaliumcarbonat gefolgt von 10,5 ml Methyliodid und rührt 24 Stunden bei Raumtemperatur. Anschließend wird über Celite abgesaugt, mit Essigester nachgewaschen und im Vakuum eingeengt. Der Rückstand wird mit Ether verdünnt, mit Wasser, gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-40% Ether erhält man 14,53 g des Esters als farbloses Öl.

15 ml Methanphosphonosäuredimethylester werden in 186 ml Tetrahydrofuran gelöst bei -70°C, tropfenweise mit 80 ml n-Butyllithium versetzt und 1 Stunde nachgerührt. Nun wird eine Lösung von 14,53 g des vorstehend beschriebenen Esters in 34 ml Tetrahydrofuran zum Reaktionsgemisch getropft und 5 Stunden nachgerührt. Anschließend werden 10,1 ml Essigsäure addiert und das Reaktionsgemisch über Nacht bei ca. 6°C stehen gelassen. Nach Einengen im Vakuum wird mit Wasser versetzt, mit Methylenchlorid extrahiert, die organische Phase über Natriumsulfat getrocknet und nach Filration im Vakuum eingeengt. Den so erhaltenen Rückstand reinigt man durch Chromatographie an Kieselgel. Mit Hexan / 0-100% Essigester erhält man 12.08 g der Titelverbindung als farbloses Öl.

IR (Film): 3288, 2945, 2893, 2360, 2102, 1725, 1675, 1598, 1558, 1506, 1496, 1442, 1424, 1191, 1113, 1078, 1030, 967, 946, 757, 692. 668, 526 $cm^{-1}$

**7b)** (5R)-5-Benzoyloxy-1-{(2S)-1,1-ethylendioxy-2-methyl-cyclohexyl-2-yl}-8,8-trimethylen-11-phenyl-(1E,3E)-1,3-undecadien-10-in

Zu einer Suspension von 1.38 g Natriumhydrid (60%ig in Mineralöl) in 42 ml Ethylenglycoldimethylether tropft man bei O°C unter Stickstoff eine Lösung von 12,08 g des unter Beispiel 7a) hergestellten Phosphonats in 63 ml Ethylenglycoldimethylether und rührt 1 Stunden nach. Nun wird eine Lösung von 6,34 g des in 1b) beschriebenen Aldehyds in 63 ml Ethylenglycoldimethylether zum Reaktionsgemisch getropft, 30 Minuten bei 0°C und 24 Stunden bei Raumtemperatur gerührt. Anschließend gibt man gesättigte Ammoniumchlorid-Lösung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-40% Ether erhält man 11,7 g des Ketons als farbloses Öl.

IR (Film): 2940, 2860, 1690, 1660, 1630, 1595, 1440, 1370, 1335, 1290, 1190, 1090, 1005, 965, 950, 800, 760, 695, 560 $cm^{-1}$

Zu einer Lösung von 11,7 g des vorstehend beschriebenen Ketons in 215 ml Methanol und 22 ml Tetrahydrofuran gibt man bei -70°C unter Stickstoff 1,5 g Certrichlorid Heptahydrat und rührt 30 Minuten nach. Nun werden 1.5 g Natriumborhydrid hinzu gegeben und 1 Stunde nachgerührt. Anschließend tropft man 13 ml Aceton hinzu, rührt 15 Minuten bei Raumtemperatur, stellt den pH-Wert mit Eisessig auf pH7 ein und engt im Vakuum bei 30°C ein. Der Rückstand wird mit Wasser/Ether 1:1 verdünnt, mit Ether extrahiert und die vereinigten organischen Phasen mit Wasser und gesättigter Natriumchlorid-Lösung gewaschen. Man trocknet über Natriumsulfat und nach Filtration wird im Vakuum eingeengt. Den so erhaltenen Rückstand trennt man durch mehrfache Chromatographie an Kieselgel. Mit Hexan / 0-100% Ether-Gemischen erhält man zunächst 5,35 g des unpolaren β-konfigurierten Alkohol sowie 5,63 g des polareren α-konfigurierten Alkohols.

Zu einer Lösung von 5,35 g des vorstehend beschriebenen unpolaren Alkohols in 22,6 ml Pyridin gibt man bei 0°C 3 ml Benzoylchlorid und rührt 24 Stunden bei Raumtemperatur nach. Anschließend wird mit Eiswasser versetzt, 2 Stunden gerührt, mit Ether verdünnt, mit Wasser, 10%iger Schwefelsäure, Wasser, gesättigter Natriumhydrogencarbonat-Lösung und Wasser gewaschen Nach Trocknung über Natriumsulfat und Filtration wird im Vakuum eingeengt. Den so erhaltenen Rückstand reinigt man durch Chromatographie an Kieselgel. Mit Hexan / 0-35% Ether erhält man 5,8 g des Benzoats als farbloses Öl.

IR (Film): 2930, 2860, 1720, 1655, 1600, 1585, 1496, 1450, 1315, 1270, 1180, 1110, 1080, 1030, 995, 960, 950, 890, 880, 760, 715, 690, 530 $cm^{-1}$

**7c)** (5-R)-5-Tert.-butyl-dimethylsilyloxy-1-{(2S)-1-oxy-2-methyl-cyclohexyl-2-yl}-8,8-trimethylen-11-phenyl-(1E. 3E)-1.3-undecadien-10-in

2,3 g des unter Beispiel 7b) hergestellten Esters werden in 9,2 ml Tetrahydrofuran und 92 ml Gemisch (65: 35:10 / Essigsäure : Wasser: Tetrahydrofuran) gelöst und unter Stickstoff bei 40°C 24 Stunden gerührt. Anschließend wird das Reaktionsgemisch im Eisbad gekühlt, vorsichtig mit 129 ml eiskalter 8 molarer Natronlauge auf pH12 gebracht und 30 Minuten bei Raumtemperatur nachgerührt. Nun wird mit Ether verdünnt, mit Wasser gewaschen, über Natriumsulfat getrocknet und nach Filtration im Vakuum eingeengt. Man erhält ohne weitere Reinigung 2,3 g des Ketons als farbloses Öl.

Zur einer Lösung von 2,3 g des vorstehend beschriebenen Ketons in 69 ml Methanol gibt man 1,28 g Kaliumcarbonat und rührt 24 Stunden bei Raumtemperatur. Anschließend wird mit Ether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-100% Ether erhält man 2,68 g des Alkohols als farbloses Öl.

IR (Film): 3440, 2930, 2860, 1740, 1705, 1600, 1490, 1370, 1240, 990, 755, 690 $cm^{-1}$

Zu einer Lösung von 3,6 g des vorstehend beschriebenen Alkohols in 34,6 ml N,N-Dimethylformamid gibt man bei O°C unter Stickstoff 2,5 g imidazol gefolgt von 2.8 g tert.-Butyldimethylsilylchlorid und rührt 15 Minuten bei 0°C und 24 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-35% Ether erhält man 4,1 g der Titelverbindung als farbloses Öl.

IR (Film): 2929, 2856, 2360, 1710, 1598, 1490, 1462, 1360, 1255. 1088, 991, 835, 775, 755, 691, 666 $cm^{-1}$

**Beispiel 8**

5-{(E)-(2S)-2-((1E,3E)-(5R)-5-Hydroxy-8,8-trimethylen-11-phenyl-1,3-undecadien-10-inyl)-2-methyl-cyclohexyliden}-3-oxy-pentansäure

Zu einer Lösung von 1,5 g des nach Beispiel 7) hergestellten Esters in 15 ml Methanol und 15 ml Tetrahydrofuran gibt man 14 ml 1 molare Natronlauge und rührt 24 Stunden bei Raumtemperatur nach. Anschließend wird mit 10%iger Schwefelsäure auf pH5 angesäuert, mit Essigester extrahiert, die vereinigten organischen Phasen mit gesättigter Natriumchlorid-Lösung gewaschen, über Natriumsulfat getrocknet und nach Filtration im Vakuum eingeengt Den so erhaltenen Rückstand reinigt man durch Chromatographie an Kieselgel. Mit Hexan / 0-100% Essigester erhält man 1,2 g der Titelverbindung als farbloses Öl.

IR (Film): 3440, 2930, 2855, 1740, 1650, 1600, 1490, 1440, 1370, 1240, 1115, 1045, 990, 755, 690 $cm^{-1}$

**Beispiel 9**

5-{(E)-(2S)-2-((1E,3E)-(5S)-5-Hydroxy-8,8-trimethylen-11-phenyl-1,3-undecadien-10-inyl)-2-methyl-cyclohexyliden}-3-oxy-pentansäure-tert.-butylester

Zu einer Suspension von 474 mg Natriumhydrid (60%ig in Mineralöl) in 12 ml Ethylenglycoldimethylether tropft man bei O°C unter Stickstoff eine Lösung von 2.67 g Phosphonoessigsäuretriethylester in 12 ml Ethylenglycoldimethylether und rührt 1 Stunde bei Raumtemperatur nach. Nun wird eine Lösung von 3 g des unter Beispiel 9b) hergestellten Ketons in 12 ml Ethylenglycoldimethylether zum Reaktionsgemisch getropft und 24 Stunden bei 50°C gerührt. Anschließend gibt man auf gesättigte Ammoniumchlorid-Lösung und verdünnt mit Ether. Die organische Phase wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 2,74 g des Esters als farbloses Öl.

IR (Film): 2930, 2860, 1720, 1635, 1490, 1440, 1380, 1250, 1175, 995, 885, 775, 755, 690 $cm^{-1}$

Zu einer Lösung von 2,94 g des vorstehend beschriebenen Esters in 30,6 ml Toluol tropft man bei -70°C unter Stickstoff 9,4 ml Diisobutylaluminiumhydrid (20%ig in Toluol) und rührt 30 Minuten nach. Anschließend werden vorsichtig 3 ml Isopropanol zugetropft und 5 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-70% Ether erhält man 2,56 g des Allylalkohols als farbloses Öl.

IR (Film): 3355, 2928, 2855, 1490, 1442, 1360, 1254, 1070, 993, 835, 775, 755, 691 $cm^{-1}$

Zu einer Lösung von 2,56 g des vorstehend beschriebenen Alkohols in 24 ml Toluol gibt man 4,7 g Bromessigsäure-tert.-butylester gefolgt von 14,9 ml 25%iger Natronlauge und 114 mg Tetrabutylammoniumhydrogensulfat. Nun wird 24 Stunden unter Stickstoff bei Raumtemperatur gerührt. Anschließend wird mit Ether verdünnt, mit halbgesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 3,72 g des Ethers als farbloses Öl.

IR (Film): 2929, 2855, 1749, 1368, 1296, 1256, 1161, 994, 836, 775, 756 $cm^{-1}$

Zu einer Lösung von 3,72 g des vorstehend beschriebenen Ethers in 145 ml Tetrahydrofuran gibt man bei Raumtemperatur unter Stickstoff 9,1 g Tetrabutylammoniumfluorid x 3 $H_2O$ und rührt 4 Stunden nach. Anschließend verdünnt man mit Ether, wäscht mit halbgesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-70% Ether erhält man 2.23 g der Titelverbindung als farbloses Öl.

IR (Film): 3460, 2920, 2850, 1750, 1650, 1600, 1490, 1440, 1370, 1225, 1160, 1120, 990, 845, 755, 690 $cm^{-1}$

Das Ausgangsmaterial für die obige Titelverbindung wird wie folgt hergestellt:

**9a)** (5S)-5-Benzoyloxy-1-{(2S)-1,1-ethylendioxy-2-methyl-cyclohexyl-2-yl}-8,8-trimethylen-11-phenyl-(1E,3E)-1,3-undecadien-10-in

Zu einer Lösung von 5,63 g des in Beispiel 7b) beschriebenen polaren Alkohols in 23,8 ml Pyridin gibt man bei 0°C 3,1 ml Benzoylchlorid und rührt 24 Stunden bei Raumtemperatur nach. Anschließend wird mit Eiswasser versetzt, 2 Stunden gerührt, mit Ether verdünnt, mit Wasser, 10%iger Schwefelsäure, Wasser, gesättigter Natriumhydrogencarbonat-Lösung und Wasser gewaschen. Nach Trocknung über Natriumsulfat und Filtration wird im Vakuum eingeengt. Den so erhaltenen Rückstand reinigt man durch Chromatographie an Kieselgel. Mit Hexan / 0-35% Ether erhält man 6,14 g des Benzoats als farbloses Öl.

IR (Film): 2930, 2860, 1720, 1655, 1600, 1585, 1490, 1450, 1315, 1270, 1180, 1110, 1070, 1025, 995, 960, 950, 890, 880, 760, 710, 690, 530 $cm^{-1}$

**9b)** (5S)-5-Tert.-butyl-dimethylsilyloxy-1-{(2S)-1-oxy-2-methyl-cyclohexyl-2-yl}-8,8-trimethylen-11-phenyl-(1E,3E)-1,3-undecadien-10-in

5 g des unter Beispiel 9a) hergestellten Esters werden in 20 ml Tetrahydrofuran und 200 ml Gemisch (65:35:10 / Essigsäure : Wasser : Tetrahydrofuran) gelöst und unter Stickstoff bei 40°C 24 Stunden gerührt. Anschließend wird das Reaktionsgemisch im Eisbad gekühlt, vorsichtig mit 280 ml eiskalter 8 molarer Natronlauge auf pH12 gebracht und 30 Minuten bei Raumtemperatur nachgerührt. Nun wird mit Ether verdünnt, mit Wasser gewaschen, über Natriumsulfat getrocknet und nach Filtration im Vakuum eingeengt. Man erhält ohne weitere Reinigung 5 g des Ketons als farbloses Öl.

Zu einer Lösung von 5 g des vorstehend beschriebenen Ketons in 150 ml Methanol gibt man 2,8 g Kaliumcarbonat und rührt 24 Stunden bei Raumtemperatur. Anschließend wird mit Ether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-70% Ether erhält man 3 g des Alkohols als farbloses Öl.

IR (Film): (DSC7/8) 3440, 2930, 2860, 1740, 1710, 1600, 1490, 1440, 1370, 1240, 1090, 1040, 990, 755, 690, 530 $cm^{-1}$

Zu einer Lösung von 4 g des vorstehend beschriebenen Alkohols in 38,4 ml N,N-Dimethylformamid gibt man bei O°C unter Stickstoff 2,8 g Imidazol gefolgt von 3,1 g tert-Butyldimethylsilylchlorid, rührt 15 Minuten bei 0°C und 24 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-35% Ether erhält man 4,52 g der Titelverbindung als farbloses Öl.

IR (Film): 2929, 2856, 2361, 1710, 1598, 1490, 1449, 1360, 1255, 1089, 991, 835, 775, 756, 692, 666 $cm^{-1}$

### Beispiel 10

5-{(E)-(2S)-2-((1E,3E)-(5S)-5-Hydroxy-8,8-trimethylen-11-phenyl-1,3-undecadien-1β-inyl)-2-methyl-cyclohexyliden}-3-oxa-pentansäure

Zu einer Lösung von 2,1 g des nach Beispiel 9) hergestellten Esters in 20,4 ml Methanol gibt man 19,7 ml 1 molare Natronlauge und rührt 24 Stunden bei Raumtemperatur nach. Anschließend wird mit 10%iger Schwefelsäure auf pH5 angesäuert, mit Essigester extrahiert, die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Ether erhält man 1,75 g der Titelverbindung als farbloses Öl.

IR (Film): 3440, 2930, 2855, 1740, 1655, 1600, 1490, 1440, 1370, 1240, 1115, 1045, 990, 755, 690 $cm^{-1}$

### Beispiel 11

5-{(E)-(2S)-2-((1E,3E)-(5S)-5-Hydroxy-6,6-trimethylen-9-phenyl-1,3-nonadienyl)-2-methyl-cyclohexyliden}-3-oxy-pentansäure-tert.-butylester

Zu einer Suspension von 299 mg Natriumhydrid (60%ig in Mineralöl) in 7,4 ml Ethylenglycoldimethylether tropft man bei O°C unter Stickstoff eine Lösung von 1,7 g Phosphonoessigsäuretriethylester in 12 ml Ethylenglycoldimethylether und rührt 1 Stunden bei Raumtemperatur nach. Nun wird eine Lösung von 1,8 g des unter Beispiel 11c) hergestellten Ketons in 12 ml Ethylenglycoldimethylether zum Reaktionsgemisch getropft und 24 Stunden bei 50°C gerührt. Anschließend gibt man auf gesättigte Ammoniumchlorid-Lösung und verdünnt mit Ether. Die organische Phase

wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 2,06 g des Esters als farbloses Öl.

Zu einer Lösung von 2.06 g des vorstehend beschriebenen Esters in 22.4 ml Toluol tropft man bei -70°C unter Stickstoff 6,9 ml Diisobutylaluminiumhydrid (20%ig in Toluol) und rührt 30 Minuten nach. Anschließend werden vorsichtig 2,3 ml Isopropanol zugetropft und 5 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-70% Ether erhält man 1,77 g des Allylalkohols als farbloses Öl.
IR (Film): 3320, 3020, 2920, 2850, 1650, 1600, 1495, 1460, 1360, 1250. 1100, 1050, 990, 835, 770, 745, 700 $cm^{-1}$

Zu einer Lösung von 1,77 g des vorstehend beschriebenen Alkohols in 17 ml Toluol gibt man 3,4 g Bromessigsäure-tert.-butylester gefolgt von 10,5 ml 25%iger Natronlauge und 83 mg Tetrabutylammoniumhydrogensulfat. Nun wird 24 Stunden unter Stickstoff bei Raumtemperatur gerührt. Anschließend wird mit Ether verdünnt, mit halbgesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 3,01 g des Ethers als farbloses Öl.
IR (Film): 2930, 2856, 1748, 1456, 1393, 1369, 1296, 1256, 1161, 1054, 994, 951, 836, 774, 699 $cm^{-1}$

Zu einer Lösung von 3,01 g des vorstehend beschriebenen Ethers in 123 ml Tetrahydrofuran gibt man bei Raumtemperatur unter Stickstoff 7,7 g Tetrabutylammoniumfluorid x 3 $H_2O$ und rührt 24 Stunden nach. Anschließend verdünnt man mit Ether, wäscht mit halbgesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-80% Ether erhält man 1.51 g der Titelverbindung als farbloses Öl.
IR (Film): 3480, 2920, 2840, 1740, 1650, 1600, 1495, 1450, 1390, 1360, 1300, 1220, 1160, 1120, 990, 940, 840, 745, 700 $cm^{-1}$

Das Ausgangsmaterial für die obige Titelverbindung wird wie folgt hergestellt:

**11a)** <u>2-Oxo-3,3-trimethylen-6-phenyl-hexan-phosphonsäure-dimethylester</u>

Zu einer Lösung von 1 g 2-Oxo-3,3-trimethylen-6-phenyl-hex-5-inphosphonsäuredimethylester in 10 ml Essigester gibt man bei Raumtemperatur unter Wasserstoff 100 mg Palladium/Kohle und rührt 6 Stunden nach. Anschließend wird abgesaugt, der Rückstand gut mit Essigester nachgewaschen und das Filtrat im Vakuum eingeengt. Den so erhaltenen Rückstand reinigt man durch Chromatographie an Kieselgel. Mit Hexan / 0-100% Essigester erhält man 900 mg der Titelverbindung als farbloses Öl.

**11b)** <u>(5S)-5-Benzoyloxy-1-{(2S)-ethylendioxy-2-methyl-6,6-trimethylen-9-phenyl-(1E,3E)-1,3-nonadien</u>

Zu einer Suspension von 1,1 g Natriumhydrid (60%ig in Mineralöl) in 33 ml Ethylenglycoldimethylether tropft man bei O°C unter Stickstoff eine Lösung von 8,9 g des unter Beispiel 11a) hergestellten Phosphonats in 50 ml Ethylenglycoldimethylether und rührt 1 Stunden nach. Nun wird eine Lösung von 5 g des in 1b) beschriebenen Aldehyds in 50 ml Ethylenglycoldimethylether zum Reaktionsgemisch getropft, 30 Minuten bei 0°C und 24 Stunden bei Raumtemperatur gerührt. Anschließend gibt man gesättigte Ammoniumchlorid-Lösung hinzu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-40% Ether erhält man 8,1 g des Ketons als farbloses Öl.
IR (Film): 3030, 2930, 2860, 1680, 1625, 1595, 1500, 1450, 1340, 1180, 1130, 1090, 1010, 960, 950, 880, 750, 700 $cm^{-1}$

Zu einer Lösung von 8,1 g des vorstehend beschriebenen Ketons in 158 ml Methanol und 16 ml Tetrahydrofuran gibt man bei -70°C unter Stickstoff 1,1 g Certrichlorid Heptahydrat und rührt 30 Minuten nach. Nun werden 1,1 g Natriumborhydrid hinzu gegeben und 1 Stunde nachgerührt. Anschließend tropft man 12 ml Aceton hinzu, rührt 15 Minuten bei Raumtemperatur, stellt den pH-Wert mit Eisessig auf pH7 ein und engt im Vakuum bei 30°C ein. Der Rückstand wird mit Wasser/Ether 1:1 verdünnt, mit Ether extrahiert und die vereinigten organischen Phasen mit Wasser und gesättigter Natriumchlorid-Lösung gewaschen. Man trocknet über Natriumsulfat und nach Filtration wird im Vakuum eingeengt. Den so erhaltenen Rückstand trennt man durch mehrfache Chromatographie an Kieselgel. Mit Hexan / 0-50% Ether-Gemischen erhält man zunächst 3,62 g des unpolaren β-konfigurierten Alkohol sowie 4.25 g des polareren α-konfigurierten Alkohols.
IR (Film): (unpolarer Alkohol) 3440, 3020, 2920, 1680, 1600, 1495, 1450, 1270, 1180, 1085, 990, 960, 880, 745, 695 $cm^{-1}$
IR (Film): (polarer Alkohol) 3480, 3020, 2930, 2680, 1680, 1660, 1600, 1500, 1450, 1180, 1090, 995, 960, 880, 800, 750, 700, 655 $cm^{-1}$
Zu einer Lösung von 3.62 g des vorstehend beschriebenen unpolaren Alkohols in 16,2 ml Pyridin gibt man bei

0°C 2,1 ml Benzoylchlorid und rührt 24 Stunden bei Raumtemperatur nach. Anschließend wird mit Eiswasser versetzt, 3 Stunden gerührt, mit Ether verdünnt, mit Wasser, 10%iger Schwefelsäure. Wasser, gesättigter Natriumhydrogencarbonat-Lösung und Wasser gewaschen. Nach Trocknung über Natriumsulfat und Filtration wird im Vakuum eingeengt. Den so erhaltenen Rückstand reinigt man durch Chromatographie an Kieselgel. Mit Hexan / 0-35% Ether erhält man 4,26 g der Titelverbindung als farbloses Öl.

**11c)** (5S)-5-Tert.-butyl-dimethylsilyloxy-1-{(2S)-1-oxo-2-methyl-cyclohexyl-2-yl}-6.6-trimethylen-9-phenyl-(1E, 3E)-1,3-nonadien

4,26 g des unter Beispiel 11b) hergestellten Benzoats werden in 17 ml Tetrahydrofuran und 170 ml Gemisch (65:35:10 / Essigsäure : Wasser : Tetrahydrofuran) gelöst und unter Stickstoff bei 45°C 6 Stunden gerührt. Anschließend wird das Reaktionsgemisch im Eisbad gekühlt, vorsichtig mit 239 ml eiskalter 8 molarer Natronlauge auf pH12 gebracht und 30 Minuten bei Raumtemperatur nachgerührt. Nun wird mit Ether verdünnt, mit Wasser gewaschen, über Natriumsulfat getrocknet und nach Filtration im Vakuum eingeengt Den so erhaltenen Rückstand reinigt man durch Chromatographie an Kieselgel. Mit Hexan / 0-50% Ether erhält man 2 g des Ketons als farbloses Öl.

IR (Film): 3030, 2930, 2860, 1710, 1600, 1500, 1450, 1315, 1270, 1185, 1110, 1070, 990, 940, 750, 710 $cm^{-1}$

Zur einer Lösung von 2 g des vorstehend beschriebenen Ketons in 61 ml Methanol gibt man 1,2 g Kaliumcarbonat und rührt 48 Stunden bei Raumtemperatur. Anschließend wird mit Ether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 0-70% Ether erhält man 1,5 g des Alkohols als farbloses Öl.

IR (Film): 3454. 2933. 2860. 1707, 1496. 1452. 1089. 992. 699 $cm^{-1}$

Zu einer Lösung von 1,5 g des vorstehend beschriebenen Alkohols in 15 ml N.N-Dimethylformamid gibt man bei O°C unter Stickstoff 1,1 g Imidazol gefolgt von 1.2 g tert.-Butyldimethylsilylchlorid. rührt 15 Minuten bei 0°C und 24 Stunden bei Raumtemperatur. Anschließend wird mit Ether verdünnt, mit Wasser. 5%iger Schwefelsäure, Wasser, gesättigter Natriumhydrogencarbonat-Lösung und Wasser gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-10% Ether erhält man 1.8 g der Titelverbindung als farbloses Öl.

IR (Film): 3020, 2920, 2850, 1710, 1590, 1460, 1360, 1250, 1100, 990, 830, 770, 700 $cm^{-1}$

**Beispiel 12**

5-{(E)-(2S)-2-((1E,3E)-(5S)-5-Hydroxy-6,6-trimethylen-9-phenyl-1,3-nonadienyl)-2-methyl-cyclohexyliden}-3-oxa-pentansäure

Zu einer Lösung von 1,36 g des nach Beispiel 11) hergestellten Esters in 13,4 ml Methanol gibt man 13,4 ml 1 molare Natronlauge und rührt 24 Stunden bei Raumtemperatur nach. Anschließend wird mit 10%iger Schwefelsäure auf pH5 angesäuert. mit Essigester extrahiert. die vereinigten organischen Phasen mit gesättigter Natriumchlorid-Lösung gewaschen. über Natriumsulfat getrocknet und nach Filtration im Vakuum eingeengt. Den so erhaltenen Rückstand reinigt man durch Chromatographie an Kieselgel. Mit Hexan / 0-100% Essigester erhält man 580 mg der Titelverbindung als farbloses Öl.

IR (Film): 3420, 3020, 2920, 2850, 1725, 1650, 1490, 1450, 1400, 1240, 1195, 1110, 990, 745, 700 $cm^{-1}$

**Beispiel 13**

5-{(E)-(2S)-2-((1E,3E)-(5R,8S)-5-Hydroxy-12,12-dimethyl-8-methyl-1,3,11-dodecatrienyl)-2-methyl-cyclohexyliden}-3-oxa-pentansäure-tert.-butylester

Zu einer Suspension von 101 mg Natriumhydrid (60%ig in Mineralöl) in 3.2 ml Ethylenglycoldimethylether tropft man bei O°C unter Stickstoff eine Lösung von 942 mg Phosphonoessigsäuretriethylester in 3,2 ml Ethylenglycoldimethylether und rührt 1 Stunden bei Raumtemperatur nach. Nun wird eine Lösung von 938 mg des unter Beispiel 13c) hergestellten Ketons in 3,2 ml Ethylenglycoldimethylether zum Reaktionsgemisch getropft und 26 Stunden bei 50°C gerührt. Anschließend gibt man auf gesättigte Ammoniumchlorid-Lösung und verdünnt mit Ether. Die organische Phase wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 1.04 g des Esters als farbloses Öl.

IR (Film): 2960, 2915, 2860, 1720, 1685, 1460, 1440, 1380, 1310, 1250, 1180, 1130, 1090, 1040, 990, 840, 810, 775 $cm^{-1}$

Zu einer Lösung von 1 g des vorstehend beschriebenen Esters in 10 ml Toluol tropft man bei -70°C unter

Stickstoff 3.6 ml Diisobutylaluminiumhydrid (20%ig in Toluol) und rührt 30 Minuten nach. Anschließend werden vorsichtig 1,5 ml Isopropanol zugetropft und 10 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-30% Ether erhält man 725 mg des Alkohols als farbloses Öl.

IR (Film): 3320, 2960, 2930, 2860, 1650. 1470, 1460, 1375, 1360, 1250, 1070, 990, 885, 805, 775 $cm^{-1}$

Zu einer Lösung von 690 mg des vorstehend beschriebenen Alkohols in 10 ml Toluol gibt man 1,4 g Bromessigsäure-tert.-butylester gefolgt von 4,4 ml 25%iger Natronlauge und 22 mg Tetrabutylammoniumhydrogensulfat. Nun wird 28 Stunden unter Stickstoff bei Raumtemperatur gerührt. Anschließend wird mit Ether verdünnt, mit halbgesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-10% Ether erhält man 730 mg des Ethers als farbloses Öl.

IR (Film): 2960, 2930,2860, 1750, 1730,1650, 1460, 1390, 1370, 1300, 1250, 1220, 1120. 990, 940, 865, 770 $cm^{-1}$

Zu einer Lösung von 690 mg des vorstehend beschriebenen Ethers in 20 ml Tetrahydrofuran gibt man bei Raumtemperatur unter Stickstoff 1,9 g Tetrabutylammoniumfluorid x 3 $H_2O$ und rührt 5 Stunden nach. Anschließend verdünnt man mit Ether, wäscht mit halbgesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-30% Ether erhält man 500 mg der Titelverbindung als farbloses Öl.

IR (Film): 3440, 2960, 2920, 2850, 1750, 1650, 1460, 1390, 1370, 1300, 1250, 1220, 1160, 1120, 990, 940, 850, 740 $cm^{-1}$

Das Ausgangsmaterial für die obige Titelverbindung wird wie folgt hergestellt:

**13a)** 5-{(2S)-1,1-Ethylendioxy-2-methyl-cyclohex-2-yl}-(2E,4E)-pentadien-1-al

Zu 4,4 g Lithiumchlorid in 300 ml Acetonitril tropft man bei Raumtemperatur unter Stickstoff 23,1 g Phosphonoessigsäuretriethylester gefolgt von 14 g 1,8-Diazabicyclo[5.4.0]undec-7-en und rührt 15 Minuten nach. Anschließend werden 15,5 g des in Beispiel 1b) beschriebenen Aldehyds in 50 ml Acetonitril zum Reaktionsgemisch getropft und 2 Stunden gerührt. Nun wurde mit Ether verdünnt mit halbkonzentrierter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 13,84 g des Esters als farbloses Öl.

IR (Film): 2980, 2940, 2860, 1710, 1640, 1610, 1460, 1445, 1370, 1330, 1310, 1260, 1240, 1180, 1150, 1140, 1090, 1045, 1005, 960, 950, 880, 800, 750, 720 $cm^{-1}$

Zu einer Lösung von 13,8 g des vorstehend beschriebenen Esters in 250 ml Toluol tropft man bei -70°C unter Stickstoff 90,2 ml Diisobutylaluminiumhydrid (20%ig in Toluol) und rührt 40 Minuten nach. Anschließend werden 25 ml Isopropanol langsam zugetropft gefolgt von 45 ml Wasser und 2 Stunden kräftig gerührt. Der Niederschlag wird abgesaugt, gut mit Essigester gewaschen und das Filtrat im Vakuum eingeengt. Den so erhaltenen Rückstand reinigt man durch Chromatographie an Kieselgel. Mit Hexan / 0-30% Ether erhält man 11,7 g des Alkohols als farbloses Öl.

IR (Film): 3420, 3020, 2940, 2860, 1660, 1460, 1445, 1370, 1340, 1290, 1275, 1180, 1140, 1090, 990, 950, 880, 800, 750, 650 $cm^{-1}$

Zu einer Lösung von 11,66 g des vorstehend beschriebenen Alkohols in 500 ml Toluol gibt man portionsweise 42,5 g Mangan(IV)oxid und rührt 3 Stunden bei Raumtemperatur unter Stickstoff. Anschließend wird über Celite abgesaugt, mit Toluol nachgewaschen und das Filtrat im Vakuum eingeengt. Man erhält ohne weitere Reinigung 10,3 g der Titelverbindung als schwach gelbgefärbtes Öl.

**13b)** (5R,8S)-5-Acetoxy-1-{(2S)-Ethylendioxy-2-methyl-cyclohexyl-2-yl}-12,12-dimethyl-8-methyl-(1E,3E)-1,3,11-dodecatrien

7,2 g Magnesium werden in 30 ml Tetrahydrofuran vorgelegt, auf 60°C erhitzt und mit Jod versetzt. Anschließend tropft man 32,9 g (S)-(+)-Citronellylbromid in 30 ml Tetrahydrofuran dazu. Nach ca. 10 ml ist die Reaktion angesprungen, die Restmenge wird zugetropft und 30 Minuten nachgerührt. Nun werden 62 ml Tetrahydrofuran addiert und auf Raumtemperatur abgekühlt.

Zu einer Lösung von 10,3 g des nach Beispiel 13a) hergestellten Aldehyds in 90 ml Tetrahydrofuran tropft man bei -70°C unter Stickstoff 130 ml der vorstehend beschriebenen Grignard-Lösung. Es wird 1 Stunde nachgerührt. Anschließend wird mit gesättigter Ammoniumchlorid-Lösung versetzt und mit Ether verdünnt. Die organische Phase wird mit halbgesättigter Natriumchlorid-Lösung gewaschen, über Natriumsulfat getrocknet und nach Filtration im Vakuum eingeengt. Man erhält ohne weitere Reinigung 6,04 g des unpolaren β-konfigurierten Alkohols sowie 8,58 g des polareren α-konfigurierten Alkohols

IR (Film): (unpolarer Alkohol) 3440, 3040, 2940, 2860, 1460, 1445, 1375, 1275, 1180, 1090, 1050, 1020, 990, 960, 950, 890, 880, 800, 750 $cm^{-1}$

IR (Film): (polarer Alkohol) 3440, 3030, 2930. 1460, 1450, 1380, 1275, 1180, 1090, 1050, 990, 960, 950, 890, 880 $cm^{-1}$

Zu einer Lösung von 6 g des vorstehend hergestellten unpolaren Alkohols in 22,7 ml Pyridin gibt man 9 ml Essigsäureanhydrid und rührt unter Stickstoff 24 Stunden bei Raumtemperatur. Anschließend wird mehrmals im Vakuum mit Toluol eingeengt. Den so erhaltenen Rückstand reinigt man durch Chromatographie an Kieselgel. Mit Hexan / 0-50% Ether erhält man 6,6 g der Titelverbindung als farbloses Öl.

IR (Film): 3040, 2930, 2860, 1735, 1650, 1460, 1460, 1445, 1370, 1230, 1180, 1090, 1020, 990, 960, 950, 880, 730 $cm^{-1}$

**13c)** (5R,8S)-5-Tert.-butyl-dimethylsilyloxy-1-{(2S)-1-oxo-2-methyl-cyclohexyl-2-yl} -12,12-dimethyl-8-methyl(1E, 3E)-1,3,11-dodecatrien

6,1 g des nach Beispiel 13b) hergestellten Acetats werden in 22 ml Tetrahydrofuran und 219 ml Gemisch (65: 35:10 / Essigsäure : Wasser : Tetrahydrofuran) gelöst und unter Stickstoff bei Raumtemperatur 12 Stunden gerührt. Anschließend wird mehrmals mit Toluol im Vakuum eingeengt. Den so erhaltenen Rückstand reinigt man durch Chromatographie an Kieselgel. Mit Hexan / 0-50% Ether erhält man 2.5 g des Keto/Acetat als farbloses Öl.

IR (Film): 2940, 2860, 1740, 1710, 1660, 1450, 1375, 1240, 1090, 1020, 990, 610 $cm^{-1}$

Zur einer Lösung von 2,8 g des vorstehend beschriebenen Keto-Acetates in 84 ml Methanol gibt man 2,1 g Kaliumcarbonat und rührt 24 Stunden bei Raumtemperatur. Anschließend wird mit Ether verdünnt, mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-50% Ether erhält man 2,45 g des Alkohols als farbloses Öl.

IR (Film): 3440, 2940, 2860, 1710, 1450, 1380, 1315, 1180, 1090, 1060, 990 cm-1

Zu einer Lösung von 1.85 g des vorstehend beschriebenen Alkohols in 20 ml N,N-Dimethylformamid gibt man bei O°C unter Stickstoff 1,1 g Imidazol gefolgt von 1,2 g tert-Butyldimethylsilylchlorid, rührt 15 Minuten bei 0°C und 23 Stunden bei Raumtemperatur. Anschließend wird mit Ether verdünnt, mit Wasser. 5%iger Schwefelsäure, Wasser, gesättigter Natriumhydrogencarbonat-Lösung und halbgesättigte Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-20% Ether erhält man 2.04 g der Titelverbindung als farbloses Öl.

IR (Film): 2960, 2930, 2860, 1710, 1470, 1460, 1450, 1380, 1360, 1250, 1090, 990, 940, 830, 770, 680 $cm^{-1}$

**Beispiel 14**

5-{(E)-(2S)-2-((1E.3E)-(5R.8S)-5-Hydroxy-12.12-dimethyl-8-methyl-1,3,11-dodecatrienyl)-2-methyl-cyclohexyliden}-3-oxa-pentansäure

Zu einer Lösung von 250 mg des nach Beispiel 13) hergestellten Esters in 5,3 ml Methanol und 2,7 ml Tetrahydrofuran gibt man 5,3 ml 0,5 molare Natronlauge und rührt 24 Stunden bei Raumtemperatur nach. Anschließend wird mit 10%iger Schwefelsäure auf pH5 angesäuert, mit Essigester extrahiert, die vereinigten organischen Phasen mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Essigester / 0-20% Isopropanol erhält man 167 mg der Titelverbindung als farbloses Öl.

IR (Film): 3440, 2960, 2920, 2840, 1730, 1650, 1650, 1600, 1440, 1375, 1230, 1120, 990. 950 $cm^{-1}$

**Beispiel 15**

5-{(E)-(2S)-2-((1E,3E)-(5S.8S)-5-Hydroxy-12.12-dimethyl-8-methyl-1,3,11-dodecatrienyl)-2-methyl-cyclohexyliden}-3-oxa-pentansäure-tert.-butylester

Zu einer Suspension von 147 mg Natriumhydrid (60%ig in Mineralöl) in 3 ml Ethylenglycoldimethylether tropft man bei O°C unter Stickstoff eine Lösung von 897 mg Phosphonoessigsäuretriethylester in 3 ml Ethylenglycoldimethylether und rührt 1 Stunden bei Raumtemperatur nach. Nun wird eine Lösung von 894 mg des unter Beispiel 15b) hergestellten Ketons in 3 ml Ethylenglycoldimethylether zum Reaktionsgemisch getropft und 26 Stunden bei 50°C gerührt. Anschließend gibt man auf gesättigte Ammoniumchlorid-Lösung und verdünnt mit Ether. Die organische Phase wird mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 1,03 g des Esters als farbloses Öl.

Zu einer Lösung von 1 g des vorstehend beschriebenen Esters in 10 ml Toluol tropft man bei -70°C unter

Stickstoff 3.6 ml Diisobutylaluminiumhydrid (20%ig in Toluol) und rührt 30 Minuten nach. Anschließend werden vorsichtig 1 ml Isopropanol zugetropft und 10 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-20% Ether erhält man 760 mg des Alkohols als farbloses Öl.

IR (Film): 3320, 2960, 2920, 2860, 1650, 1470, 1460, 1380, 1360, 1250, 1080, 990, 830, 800, 770, 670 $cm^{-1}$

Zu einer Lösung von 730 mg des vorstehend beschriebenen Alkohols in 11 ml Toluol gibt man 1,5 g Bromessigsäure-tert.-butylester gefolgt von 4,6 ml 25%iger Natronlauge und 23 mg Tetrabutylammoniumhydrogensulfat. Nun wird 28 Stunden unter Stickstoff bei Raumtemperatur gerührt. Anschließend wird mit Ether verdünnt, mit halbgesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-10% Ether erhält man 796 mg des Ethers als farbloses Öl.

IR (Film): 2960, 2920, 2860, 1750, 1730, 1650, 1470, 1460, 1390, 1370, 1300, 1250, 1220, 1160, 1120, 1070, 990, 940, 890, 770, 730, 670 $cm^{-1}$

Zu einer Lösung von 400 mg des vorstehend beschriebenen Ethers in 12 ml Tetrahydrofuran gibt man bei Raumtemperatur unter Stickstoff 1,1 g Tetrabutylammoniumfluorid x 3 $H_2O$ und rührt 5 Stunden nach. Anschließend verdünnt man mit Ether, wäscht mit halbgesättigter Natriumchlorid-Lösung, trocknet die organische Phase über Natriumsulfat und engt nach Filtration im Vakuum ein. Den so erhaltenen Rückstand reinigt man durch Chromatographie an Kieselgel. Mit Hexan / 0-30% Ether erhält man 310 mg der Titelverbindung als farbloses Öl.

IR (Film): 3460, 2966, 2920, 2860, 1750, 1650, 1460, 1390, 1370, 1300, 1250, 1220, 1160, 1120, 990, 940, 840, 740 $cm^{-1}$

Das Ausgangsmaterial für die obige Titelverbindung wird wie folgt hergestellt:

**15a)** <u>(5S,8S)-5-Acetoxy-1-{(2S)-Ethylendioxy-2-methyl-cyclohexyl-2-yl}-12,12-dimethyl-8-methyl-(1E,3E)-1,3,11-dodecatrien</u>

Zu einer Lösung von 8,5 g des in Beispiel 13b) beschriebenen polaren Alkohols in 32 ml Pyridin gibt man 13,8 ml Essigsäureanhydrid und rührt unter Stickstoff 24 Stunden bei Raumtemperatur. Anschließend wird mehrmals im Vakuum unter Toluol Zusatz eingeengt. Den so erhaitenen Rückstand reinigt man durch Chromatographie an Kieselgel. Mit Hexan / 0-40% Ether erhält man 7.46 g der Titelverbindung als farbloses Öl.

IR (Film): 3020, 2920, 2860, 1730, 1650, 1460, 1445, 1370, 1240, 1180, 1090, 1020, 990, 960, 950, 880, 800, 750, 610 $cm^{-1}$

**15b)** <u>(5S,8S)-5-Tert.-butyl-dimethylsilyloxy-1-{(2S)-1-oxo-2-methyl-cyclohexyl-2-yl} -12,11-dimethyl-8-methyl-(1E,3E)-1,3,11-dodecatrien</u>

7 g des unter Beispiel 15a) hergestellten Acetates werden in 25 ml Tetrahydrofuran und 250 ml Gemisch (65:35:10 / Essigsäure . Wasser Tetrahydrofuran) gelöst und unter Stickstoff bei Raumtemperatur 12 Stunden gerührt. Anschließend wird das Reaktionsgemisch mehrmal mit Toluol im Vakuum eingeengt. Den so erhaltenen Rückstand reinigt man durch Chromatographie an Kieselgel. Mit Hexan / 0-20% Ether erhält man 2,05 g des Ketons als farbloses Öl.

IR (Film): 2940, 2860, 1740, 1710, 1450, 1370, 1320, 1240, 1120, 1090, 1060, 1020, 990, 860, 700, 610 $cm^{-1}$

Zu einer Lösung von 2 g des vorstehend beschriebenen Ketons in 60 ml Methanol gibt man 1,5 g Kaliumcarbonat und rührt unter Stickstoff bei Raumtemperatur 24 Stunden. Anschließend wird mit Ether verdünnt, mit halbgesattigter Natriumchlorid-Lösung gewaschen, die organischen Phase über Natriumsulfat getrocknet und nach Filtration im Vakuum eingeengt. Den so erhaltenen Rückstand reinigt man durch Chromatographie an Kieselgel. Mit Hexan / 0-50% Ether erhält man 1,56 g des Alkohols als farbloses Öl.

IR (Film): 3440, 2940, 2860, 1710, 1450, 1380, 1340, 1310, 1120, 1090. 1060. 990, 910, 860, 700 $cm^{-1}$

Zu einer Lösung von 1,5 g des vorgehend beschriebenen Alkohols in 17 ml N.N-Dimethylformamid gibt man bei O°C unter Stickstoff 1,2 g Imidazol gefolgt von 1,4 g tert.-Butyldimethylsilylchlorid, rührt 15 Minuten bei 0°C und 26 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-20% Ether erhält man 1.9 g der Titelverbindung als farbloses Öl.

IR (Film): 2960, 2920, 2860, 1710, 1470, 1460, 1450, 1380, 1360, 1310, 1250, 1010, 990, 940, 840, 780, 680 $cm^{-1}$

**Beispiel 16**

5-{(E)-(2S)-2-((1E.3E)-(5S.8S)-5-Hydroxy-12.12-dimethyl-8-methyl-1,3,11-dodecatrienyl)-2-methyl-cyclohexyliden-3-oxa-pentansäure

Zu einer Lösung von 216 mg des nach Beispiel 15) hergestellten Esters in 4.6 ml Methanol und 2.3 ml Tetrahydrofuran gibt man 4,6 ml 0,5 molare Natronlauge und rührt 24 Stunden bei Raumtemperatur nach. Anschließend wird mit 10%iger Schwefelsäure auf pH3 angesäuert. mit Essigester extrahiert. die vereinigten organischen Phasen mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-60% Ether erhält man 193 mg der Titelverbindung als farbloses Öl.
IR (Film): 3440, 2960, 2920, 2860, 1730, 1650, 1460, 1380, 1230, 1120, 990, 940, 680 $cm^{-1}$

**Beispiel 17**

5-{(E)-(2S)-2-((1E,3E)-(5S)-5-hydroxy-6,6-trimethylen-10-phenyl-1,3-decadien-9-inyl)-2-methyl-cyclohexyliden}-3-oxa-pentansäure-tert.-butylester

Zu einer Suspension von 119 mg Natnumhydrid (60%ig in Mineralöl) in 2.4 ml Ethylenglycoldimethylether tropft man bei O°C unter Stickstoff eine Lösung von 722 mg Phosphonoessigsäuretriethylester in 2.4 ml Ethylenglycoldimethylether und rührt 2 Stunden nach. Nun wird eine Lösung von 790 mg des nach Beispiel 17b) hergestellten Ketons in 2,4 ml Ethylenglycoldimethylether zum Reaktionsgemisch getropft und 24 Stunden bei 50°C gerührt. Anschließend gibt man gesättigte Ammoniumchlorid-Lösung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-5% Ether erhält man 804 mg des Esters als farbloses Öl.
IR (Film): 2390, 2857, 1716, 1635, 1490, 1443, 1377, 1251, 1174, 1125, 1040, 995, 836, 775, 756, 692, 526 $cm^{-1}$

Zu einer Lösung von 792 mg des vorstehend beschriebenen Esters in 7,5 ml Toluol tropft man bei -70°C unter Stickstoff 2,4 ml Diisobutylaluminiumhydrid (20%ig in Toluol) und rührt 30 Minuten nach. Anschließend werden vorsichtig 0,4 ml Isopropanol zugetropft und 10 Minuten gerührt. Nun wird mit Wasser versetzt. 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-20% Ether erhält man 656 mg des Allylalkohols als farbloses Öl.
IR (Film): 2254, 2928, 2856, 2360, 1653, 1598, 1490, 1462, 1442, 1360, 1251, 1103, 1053, 994, 836, 775, 755, 691, 526 $cm^{-1}$

Zu einer Lösung von 632 mg des vorstehend beschriebenen Alkohols in 9,5 ml Toluol gibt man 1,2 g Bromessigsäure-tert.-butylester gefolgt von 3,8 ml 25%iger Natronlauge und 21 mg Tetrabutylammoniumhydrogensulfat. Nun wird 24 Stunden unter Stickstoff bei Raumtemperatur gerührt. Anschließend wird mit Ether und Wasser verdünnt, mit 10%iger Zitronensäure angesäuert, mit Ether extrahiert, die vereinigten organischen Phasen mit halbgesättigter Natriumchlorid-Lösung gewaschen. über Natriumsulfat getrocknet und nach Filtration im Vakuum eingeengt. Den so erhaltenen Rückstand reinigt man durch Chromatographie an Kieselgel. Mit Hexan / 0-10% Ether erhält man 716 mg des Esters als farbloses Öl.
IR (Film): 2928, 2856, 1749, 1657, 1598, 1490, 1462, 1392, 1367, 1251, 1223, 1120, 1054, 994, 939, 836, 775, 756, 692. 526 $cm^{-1}$

Zu einer Lösung von 703 mg des vorstehend beschriebenen Esters in 4,6 ml Tetrahydrofuran gibt man bei Raumtemperatur unter Stickstoff 701 mg Tetrabutylammoniumfluorid x 3 $H_2O$ und rührt 18 Stunden nach. Anschließend verdünnt man mit Ether, wäscht mit halbgesättigter Natriumchlorid-Lösung, trocknet die organische Phase über Natriumsulfat und engt, nach Filtration, am Vakuum ein. Den so erhaltenen Rückstand reinigt man durch Chromatographie an Kieselgel. Mit Hexan / 0-20% Ether erhält man 485 mg der Titelverbindung als farbloses Öl.
IR (Film): 2470, 2929, 2360, 1747, 1657, 1598, 1490, 1442, 1368, 1225, 1120, 995. 943, 845, 756, 692, 668, 527 $cm^{-1}$

Das Ausgangsmaterial für die obige Titelverbindung wird wie folgt hergestellt:

**17a)** (5R)-5-Benzoyloxy-1-{(2S)-1.1-ethylendioxy-2-methyl-cyclohexyl-2-yl}-6.6-trimethylen-10-phenyl-(1E.3E)-decadien-9-in

Zu einer Suspension von 1,1 g Natriumhydrid (60%ig in Mineralöl) in 55 ml Ethylenglycoldimethylether tropft man bei O°C unter Stickstoff eine Lösung von 9,9 g 2-Oxo-3.3-trimethylen-7-phenyl-hept-6-in-phosphonsäuredimethylester (beschrieben in DE-OS.....) in 55 ml Ethylenglycoldimethylether und rührt 30 Minuten nach. Nun wird eine Lösung von 5,42 g des in 1b) beschriebenen Aldehyds in 55 ml Ethylenglycoldimethylether zum Reaktionsgemisch getropft und 4 Stunden bei 50°C gerührt. Anschließend gibt man gesättigte Ammoniumchlorid-Lösung zu und extrahiert mit Ether. Die vereinigten organischen Phasen werden mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-35% Ether erhält man 8.59 g des Ketons als farbloses Öl.
IR (Film): 2934, 1678, 1625, 1591, 1490, 1442, 1350, 1227, 1180, 1135, 1089, 1055, 1019, 962, 880, 795, 757, 692, 528 $cm^{-1}$

Zu einer Lösung von 5 g des vorstehend beschriebenen Ketons in 100 ml Methanol und 10 ml Tetrahydrofuran gibt man bei -60°C unter Stickstoff 691 mg Certrichlorid Heptahydrat und rührt 30 Minuten nach. Nun werden 702 mg Natriumborhydrid hinzu gegeben und 1.5 Stunden nachgerührt. Anschließend tropft man 20 ml Aceton hinzu, rührt 15 Minuten bei Raumtemperatur, stellt den pH-Wert mit Eisessig auf pH7 ein und engt im Vakuum bei 30°C ein Der Rückstand wird mit Wasser/Ether 1:1 verdünnt, mit Ether extraniert und die vereinigten organischen Phasen mit halbgesättigter Natriumchlorid-Lösung gewaschen. Man trocknet über Natriumsulfat und nach Filtration wird im Vakuum eingeengt. Den so erhaltenen Rückstand trennt man durch mehrfache Chromatographie an Kieselgel. Mit Hexan / 0-25% Ether-Gemischen erhält man zunächst 1,54g des unpolaren Alkohols sowie 2,63 g des polareren Alkohols als farbloses Öl.

Zu einer Lösung von 5,57 g des vorstehend beschriebenen unpolaren Alkohols in 21,5 ml Pyridin gibt man bei 0°C 3,1 ml Benzoylchlorid, rührt 1 Stunde bei 0°C und 18 Stunden bei Raumtemperatur nach. Anschließend wird mit Eiswasser versetzt, 1 Stunde gerührt, mit Ether verdünnt, mit halbgesättigter Natriumchlorid-Lösung gewaschen. Nach Trocknung über Natriumsulfat und Filtration wird im Vakuum eingeengt. Den so erhaltenen Rückstand reinigt man durch Chromatographie an Kieselgel. Mit Hexan / 0-10% Ether erhält man 6,46 g des Benzoats als farbloses Öl.
IR (Film): 2920, 2235, 1712, 1650, 1600, 1584, 1491, 1450, 1274, 1093, 880, 802, 757, 714, 618, 526 $cm^{-1}$

**17b)** (5R)-5-Tert-butyl-dimethylsilyloxy-1-{(2S)-1-oxo-2-methyl-2-yl}-6,6-trimethylen-10-phenyl-(1E,3E)-13-decadien-9-in

6,45 g des unter Beispiel 17a) hergestellten Benzoats werden in 185 ml Gemisch (65:35:10 / Essigsäure : Wasser : Tetrahydrofuran) gelöst und unter Stickstoff bei 45°C 20 Stunden Stunden gerührt. Anschließend wird das Reaktionsgemisch mehrmals mit Toluol im Vakuum eingeengt. Den so erhaltenen Rückstand reinigt man durch mehrmalige Chromatographie an Kieselgel. Mit Hexan / 0-10% Essigester erhält man 3,09 g des Ketons als farbloses Öl.
IR (Film): 2934, 2862, 2234, 1713, 1599, 1584, 1490, 1450, 1314, 1272, 1176, 1111, 1069, 1026, 992, 941, 859, 804, 757, 712, 692, 526 $cm^{-1}$

Zu einer Lösung von 3,08 g des vorstehend beschriebenen Ketons in 50 ml Methanol gibt man 1.33 g Kaliumcarbonat und rührt unter Stickstoff bei Raumtemperatur 18 Stunden. Anschließend wird mit Wasser verdünnt, mit Essigester extrahiert, die vereinigten organischen Phasen mt halbgesättigter Natriumchlorid-Lösung gewaschen, über Natriumsulfat getrocknet und nach Filtration im Vakuum eingeengt Den so erhaltenen Rückstand reinigt man durch Chromatographie an Kieselgel. Mit Hexan / 0-30% Ether erhält man 2.13 g des Alkohols als farbloses Öl.
IR (Film): 3460, 2933, 2862, 1707, 1598, 1490, 1442, 1373, 1312, 1118, 992, 757, 692, 527 $cm^{-1}$
Zu einer Lösung von 2.12 g des vorstehend beschriebenen Alkohols in 29 mi N.N-Dimethylformamid gibt man bei Raumtemperatur unter Stickstoff 1,4 g Imidazol gefolgt von 1,5 g tert.-Butyldimethylsilylchlorid und rührt 20 Stunden bei Raumtemperatur. Anschließend wird mit Ether/Hexan 1:1 verdünnt, mit Wasser und halbgesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-5% Ether erhält man 2.39 g der Titelverbindung als farbloses Öl.
IR (Film): 2931, 2840, 1711, 1598, 1490, 1462, 1448, 1360, 1253, 1118, 1062, 992, 908, 836, 775, 756, 692, 526 $cm^{-1}$

**Beispiel 18**

5-{(E)-(2S)-2-((1E,3E)-(5S)-5-Hydroxy-6,6-trimethylen-10-phenyl-1,3-decadien-9-inyl)-2-methyl-cyclohexyliden}-3-oxa-pentansäure

Zu einer Lösung von 462 mg des in Beispiel 17 hergestellten Esters in 8,8 ml Tetrahydrofuran und 8,8 ml Methanol werden 4,5 ml 0,5 molare Lithiumhydroxid-Lösung gefolgt von 66 mg Lithiumhydroxid addiert und 18 Stunden bei Raumtemperatur gerührt. Anschließend wird mit Wasser verdünnt, mit 1 molarer Salzsäure auf pH5 gebracht, mit Essigester extrahiert, über Natriumsulfat getrocknet und nach Filtration im Vakuum eingeengt. Den so erhaltenen Rückstand reinigt man durch Chromatographie an Kieselgel. Mit Hexan / 0-90% Essigester erhält man 178 mg der Titelverbindung als farbloses Öl.
IR (Film): 3450, 2930, 2850, 1740, 1650, 1600, 1490, 1440. 1380, 1240, 1110, 1040, 990, 760, 690, 530 $cm^{-1}$

**Beispiel 19**

5-{(E)-(2S)-2-((1E.3E)-(5S)-5-Hydroxy-6.6-dimethyl-9-phenoxy-1.3-nonadienyl)-2-methyl-cyclohexyliden}-3-oxa-pentansäure-tert.-butylester

Zu einer Suspension von 159 mg Natriumhydrid (60%ig in Mineralöl) in 3 ml Ethylenglycoldimethylether tropft man bei O°C unter Stickstoff eine Lösung von 896 mg Phosphonoessigsäuretriethylester in 3 ml Ethylenglycoldimethylether und rührt 2 Stunden nach. Nun wird eine Lösung von 969 mg des nach Beispiel 19c) hergestellten Ketons in 5 ml Ethylenglycoldimethylether zum Reaktionsgemisch getropft und 16 Stunden bei 50°C gerührt. Durch die noch nicht vollständige Reaktion wird auf 0°C gekühlt und eine frisch bereitete Phosphonatlösung (448 mg Phosphonoessigsäuretriethylester/ 80 mg Natriumhydrid in 6 ml Ethylenglycoldimethylether) addiert und 5 Stunden bei 50°C gerührt. Anschließend gibt man gesättigte Ammoniumchlorid-Lösung zu und extrahiert mit Ether. Die vereinigten organischen Phasen werden mit Wasser und gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 1.03 g des Esters als farbloses Öl.
IR (Film): 2929. 2857, 1716, 1635, 1600, 1498, 1471, 1247, 1173, 1040, 995, 836, 775, 753, 691 $cm^{-1}$

Zu einer Lösung von 945 mg des vorstehend beschriebenen Esters in 10 ml Toluol tropft man bei -70°C unter Stickstoff 3,2 ml Diisobutylaluminiumhydrid (20%ig in Toluol) und rührt 1 Stunde nach. Anschließend werden vorsichtig 0,9 ml Isopropanol zugetropft und 10 Minuten gerührt. Nun wird mit Wasser versetzt, 2 Stunden bei Raumtemperatur gerührt, der weiße Niederschlag abfiltriert und gut mit Essigester gewaschen. Das Filtrat wird im Vakuum eingeengt. Den so erhaltenen Rückstand reinigt man durch Chromatographie an Kieselgel. Mit Hexan / 0-50% Ether erhält man 730 mg des Allylalkohols als farbloses Öl.
IR (Film): 3334, 2927, 2856, 1600, 1497, 1472, 1247, 1109, 1058, 994, 836, 774, 753, 691 $cm^{-1}$

Zu einer Lösung von 326 mg des vorstehend beschriebenen Alkohols in 3 ml Toluol gibt man 1,6 ml Bromessigsäure-tert.-butylester gefolgt von 1,8 ml 25%iger Natronlauge und 15 mg Tetrabutylammoniumhydrogensulfat. Nun wird 6 Stunden unter Stickstoff bei Raumtemperatur gerührt. Anschließend wird mit Ether verdünnt, mit Wasser und gesättigter Natriumchlorid-Lösung gewaschen, die organische Phase über Natriumsulfat getrocknet und nach Filtration im Vakuum eingeengt. Den so erhaltenen Rückstand reinigt man durch Chromatographie an Kieselgel. Mit Hexan / 0-10% Ether erhält man 350 mg des Esters als farbloses Öl.
IR (Film): 2929, 2856, 1749, 1497, 1472, 1368, 1247, 1119, 1062, 994, 836, 775, 753, 691 $cm^{-1}$

Das Ausgangsmaterial für die obige Titelverbindung wird wie folgt hergestellt:

**19a)** 2-Oxo-3,3-dimethyl-6-phenoxy-hexan-phosphonsäuredimethylester

Zu 99.3 ml n-Butyllithium werden bei 0°C unter Stickstoff 16,3 g Diisopropylamin in 120 ml Tetrahydrofuran getropft und 45 Minuten nachgerührt. Die Reaktionslösung wird auf -70° bis -65°C gekühlt, vorsichtig mit 14,3 g Isobuttersäure in 120 ml Tetrahydrofuran versetzt und 1 Stunde gerührt. 30,1 g 3-Phenoxypropylbromid werden in 200 ml 1,3-Dimethyltetrahydro-2-pyrimidinon gelöst und zum Reaktionsgemisch getropft. Es wird 30 Minuten bei -70°C, 1 Stunde bei -30°C und 1 Stunde bei 0°C gerührt. Anschließend wird auf gesättigte Ammoniumchlorid-Lösung gegeben, mit Ether extrahiert, die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan/ 0-10% Ether erhält man 24,3 g des Esters als farbloses Öl.
IR (Film): 2951, 2360, 1731, 1600, 1586, 1498, 1474, 1390, 1246, 1197, 1147, 1080, 1048, 990, 755, 692 $cm^{-1}$

Zu 131 ml n-Butyllithium werden bei -40°C unter Stickstoff 33 g Diisopropylamin in 50 ml Tetrahydrofuran gegeben und 15 Minuten gerührt. Nun wird das Reaktionsgemisch auf -70°C gekühlt mit 24,9 g Methanphosphorsäuredimethylester in 50 ml Tetrahydrofuran (30 Minuten nachgerührt) gefolgt von 22 g des vorstehend beschriebenen Esters in 50 ml Tetrahydrofuran versetzt, langsam auf O°C erwärmt und 2 Stunden gerührt. Anschließend wird mit 10%iger Schwefelsäure auf pH2 angesäuert, mit Essigester extrahiert, die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Essigester erhält man 28,1 g der Titelverbindung als farbloses Öl.
IR (Film): 2956, 2359, 1704, 1600, 1586, 1497, 1471, 1390, 1247, 1174, 1032, 869, 806, 757, 693 $cm^{-1}$

**19b)** (5R)-5-Benzoyloxy-1-{(2S)-1,1-ethylendioxy-2-methyl-cyclohexyl-2-yl}-6,6-dimethyl-9-phenoxy-(1E.3E)-1.3-nonadien

Zu einer Suspension von 2,73 g Natriumhydrid (60%ig in Mineralöl) in 90 ml 1.2-Dimethoxyethan tropft man bei O°C unter Stickstoff eine Lösung von 22.63 g des unter Beispiel 19a) hergestellten Phosphonats in 150 ml 1,2-Dimethoxyethan und rührt 1 Stunden nach. Nun wird eine Lösung von 12,6 g des unter Beispiel 1b) hergestellten Aldehyds in 150 ml 1,2-Dimethoxyethan zum Reaktionsgemisch getropft, 30 Minuten bei 0°C und 64 Stunden bei Raumtemperatur gerührt. Anschließend gibt man gesättigte Ammoniumchlorid-Lösung zu und extrahiert mit Ether. Die vereinigten organischen Phasen werden mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 22,7 g des Ketons als farbloses Öl. IR (Film): 2934, 1679, 1625, 1589, 1497, 1470, 1340, 1245, 1179, 1085, 1038, 962, 880, 795, 754, 692 $cm^{-1}$

Zu einer Lösung von 22,5 g des vorstehend beschriebenen Ketons in 240 ml Methanol und 180 ml Tetrahydrofuran gibt man bei -55°C unter Stickstoff 3.1 g Certrichlorid Heptahydrat und rührt 30 Minuten nach. Nun werden 3.1 g Natriumborhydrid hinzu gegeben und 3 Stunden nachgerührt. Anschließend tropft man 33 ml Aceton hinzu, rührt 30 Minuten bei Raumtemperatur, stellt den pH-Wert mit Eisessig auf pH7 ein und engt im Vakuum bei 30°C ein. Der Rückstand wird mit Wasser/Ether 1:1 verdünnt, mit Ether extrahiert und die vereinigten organischen Phasen mit Wasser und gesättigter Natriumchlorid-Lösung gewaschen. Man trocknet über Natriumsulfat und engt nach Filtration im Vakuum ein. Den so erhaltenen Rückstand trennt man durch mehrfache Chromatographie an Kieselgel. Mit Hexan / 0-50% Ether-Gemischen erhält man zunächst 6.8 g des unpolaren β-konfigurierten Alkohols sowie 7,7 g des polareren α-konfigunerten Alkohols als farbloses Öl.

IR (Film): (unpolarer Alkohol) 3484, 2947, 2868, 1600, 1586, 1498, 1470, 1365, 1246, 1179, 1088, 993, 961, 880, 754, 692 $cm^{-1}$

IR (Film): (polarer Alkohol) 3478, 2947, 2867, 1600, 1586, 1497, 1471, 1365, 1246, 1178, 1088, 993, 961, 880, 754, 692 $cm^{-1}$

Zu einer Lösung von 4,56 g des vorstehend beschriebenen unpolaren Alkoho's in 1 ml Pyridin gibt man bei 0°C 1,5 ml Benzoylchlorid und rührt 24 Stunden bei Raumtemperatur nach. Anschließend wird mit Eiswasser versetzt, 2 Stunden gerührt, mit Ether verdünnt, mit Wasser, 10%iger Schwefelsäure, Wasser, gesättigter Natriumhydrogencarbonat-Lösung und Wasser gewaschen. Nach Trocknung über Natriumsuffat und Filtration wird im Vakuum eingeengt. Den so erhaltenen Rückstand reinigt man durch mehrmalige Chromatographie an Kieselgel. Mit Hexan / 0-30% Ether erhält man 5,8 g des Benzoats als farbloses Öl.

IR (Film): 2947, 2359, 1716, 1600, 1586, 1497, 1269, 1176, 1109, 1026, 949, 754, 712, 692 $cm^{-1}$

**19c)** (5R)-5-Tert.-butyl-dimethylsilyloxy-1-{(2S)-1-oxo-2-methyl-cyclohexyl-2-yl}-6,6-dimethyl-9-phenoxy-(1E. 3E)-1.3-nonadien

5,6 g des unter Beispiel 19b) hergestellten Ethers werden in 110 ml Tetrahydrofuran und 150 ml Gemisch (65: 35:10 / Essigsäure : Wasser : Tetrahydrofuran) gelöst und unter Stickstoff bei 50°C 20 Stunden gerührt. Anschließend wird das Reaktionsgemisch im Eisbad gekühlt, vorsichtig mit eiskalter 8 molarer Natronlauge auf pH8 gebracht und 30 Minuten nachgerührt. Nun wird mit Ether extrahiert, die vereinigten organischen Phasen mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-20% Ether erhält man 3.5 g des Ketons als farbloses Öl.

IR (Film): 2932, 1713, 1600, 1586, 1495, 1450, 1268, 1173, 1110, 992, 835, 755, 712, 692 $cm^{-1}$

Zu einer Lösung von 3,4 g des vorstehend beschriebenen Ketons in 120 ml Methanol gibt man 2,1 g Kaliumcarbonat und rührt unter Stickstoff bei Raumtemperatur 16 Stunden. Anschließend wird mit Ether verdünnt, mit Wasser und gesättigter Natriumchlorid-Lösung gewaschen, die organischen Phase über Natriumsulfat getrocknet und nach Filtration im Vakuum eingeengt. Den so erhaltenen Rückstand reinigt man durch Chromatographie an Kieselgel. Mit Hexan / 0-30% Ether erhält man 2.51 g des Alkohols als farbloses Öl.

IR (Film): 3485, 2934, 2866, 1707, 1600, 1497, 1470, 1246, 1171, 1036, 992, 755, 692 $cm^{-1}$

Zu einer Lösung von 2,4 g des vorgehend beschriebenen Alkohols in 25 ml N,N-Dimethylformamid gibt man bei O°C unter Stickstoff 1,8 g Imidazol gefolgt von 2 g tert.-Butyldimethylsilylchlorid und rührt 15 Minuten bei 0°C und 16 Stunden bei Raumtemperatur. Anschließend wird mit Ether verdünnt, mit Wasser, 5%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-10% Ether erhält man 2,7 g der Titelverbindung als farbloses Öl.

IR (Film): 2956, 2857, 1711, 1600, 1498, 1471, 1247, 1110, 1063, 992, 836, 775, 753, 691 $cm^{-1}$

**Beispiel 20**

5-{(E)-(2S)-2-((1E,3E)-(5S)-5-Hydroxy-6.6-dimethyl-9-phenoxy-1.3-nonadienyl)-2-methyl-cyclohexyliden}-3-oxa-pentansäure

Zu einer Lösung von 170 mg des in Beispiel 19 hergestellten Esters in 2 ml Tetrahydrofuran und 2 ml Methanol werden 1,9 ml 1 molare Natriumhydroxid-Lösung addiert und 16 Stunden bei Raumtemperatur gerührt. Anschließend wird mit Eiswasser gekühlt, mit 1 molarer Schwefelsäure auf pH4 angesäuert und mit Essigester extrahiert. Die vereinigten organischen Phasen werden mit Natriumchlorid-Lösung gewaschen über Natriumsulfat getrocknet und nach Filtration im Vakuum eingeengt. Den so erhaltenen Rückstand reinigt man durch Chromatographie an Kieselgel. Mit Methylenchlorid / 0-10% Methanol erhält man 79 mg der Titelverbindung als farbloses Öl.
IR (Film): 3440, 2960, 2930, 2860, 1620, 1600, 1500, 1470, 1430, 1340, 1250, 1180, 1080, 1040, 990, 950, 760, 690 600 510 $cm^{-1}$

**Beispiel 21**

7-{(E)-(2S)-2-((1E,3E)-(5S)-5-Hydroxy-6.6-trimethylen-9-phenyl-1,3-nonadien-8-inyl)-2-methyl-cyclohexyliden}-5-oxa-heptansäure

Zu einer Lösung von 125 mg des in Beispiel 1 hergestellten Allylalkohols in 1,9 ml Toluol gibt man 85 mg 4-Bromorthobuttersäuretrimethylester gefolgt von 0,75 ml 25%iger Natronlauge und 3,6 mg Tetrabutylammoniumhydrogensulfat. Nun wird 24 Stunden unter Stickstoff bei Raumtemperatur gerührt. Anschließend wird mit Ether und Wasser verdünnt, mit Ether extrahiert, die vereinigten organischen Phasen mit halbgesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-10% Ether erhält man 60,1 mg des Ethers als farbloses Öl.
IR (Film): 2929, 1738, 1657, 1598, 1442, 1360, 1252, 1103, 995, 836, 775, 756, 692, 526 $cm^{-1}$
Zu einer Lösung von 114,7 mg des vorstehend beschriebenen Esters in 0,8 ml Tetrahydrofuran gibt man bei Raumtemperatur unter Stickstoff 120 mg Tetrabutylammoniumfluorid x 3 $H_2O$ und rührt 5 Stunden nach. Anschließend verdünnt man mit Ether, wäscht mit Wasser, trocknet die organische Phase über Natriumsulfat und engt, nach Filtration, am Vakuum ein. Den so erhaltenen Rückstand reinigt man durch Chromatographie an Kieselgel. Mit Hexan / 0-30% Ether erhält man 62,5 mg des Alkohols als farbloses Öl.
IR (Film): 3450, 2930, 2860, 1740, 1650, 1600, 1490, 1440, 1365, 1260, 1200, 1170, 1100, 990, 805, 755, 690, 525 $cm^{-1}$
Zu einer Lösung von 67,5 mg des vorstehend beschriebenen Alkohols in 0,7 ml Tetrahydrofuran und 0,7 ml Methanol werden 0,7 ml 0,5 molare Lithiumhydroxid-Lösung addiert und 40 Stunden bei Raumtemperatur gerührt. Anschließend wird mit Wasser verdünnt, mit 1 molarer Salzsäure auf pH5 gebracht, mit Essigester extrahiert, über Natriumsulfat getrocknet und nach Filtration im Vakuum eingeengt. Den so erhaltenen Rückstand reinigt man durch Chromatographie an Kieselgel. Mit Hexan / 0-100% Essigester erhält man 28,4 mg der Titelverbindung als farbloses Öl.
IR (Film): 2960, 2920, 2850, 1710, 1360, 1115, 990, 755, 690, 620 $cm^{-1}$

**Beispiel 22**

5-{(E.Z)-(2S)-2-((1E,3E)-(5S)-5-Hydroxy-6,6-trimethylen-9-phenyl-1,3-nonadien-8-inyl)-2-methyl-cyclohexyliden-pentansäure

3,35 ml einer Lösung von Methansulfinylmethylnatrium in Dimethylsulfoxid (Herstellung: man löst 1g 50%ige Natriumhydridsuspension in 20 ml Dimethylsulfoxid während einer Stunde bei 70°C) tropft man bein 15°C zu einer Lösung von 776 mg 4-Carboxybutyltriphenylphosphoniumbromid in 3,5 ml Dimethylsulfoxid und rührt 30 Minuten bei Raumtemperatur. Zur roten Ylenlösung gibt man eine Lösung von 223 mg (5S)-5-Tert.-butyl-dimethylsilyloxy-1-{(2S)-1-Oxo-cyclohexyl-2-yl}-6,6-trimethylen-9-phenyl-(1E, 3E)-1,3-nonadien-8-in (hergestellt in Beispiel 1d) in 2 ml Dimethylsulfoxid und rührt 24 Stunden bei Raumtemperatur unter Stickstoff. Anschließend wird das Reaktionsgemisch auf Eiswasser gegeben. mit 10%iger Zitronensäure-Lösung auf pH4 angesäuert und mit Essigester extrahiert. Die organische Phase wird mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Ether erhält man 21 mg der Carbonsäure als farbloses Öl.
IR (Film): 3300, 2934, 2859, 2203, 1709, 992, 836 cm-1
Zu einer Lösung von 19 mg der vorstehend beschriebenen Carbonsäure in 0,2 ml Tetrahydrofuran gibt man bei Raumtemperatur unter Stickstoff 25 mg Tetrabutylammoniumfluorid und rührt 12 Stunden bei Raumtemperatur.

Anschließend verdünnt man mit Ether, wäscht mit Wasser, trocknet über Natriumsulfat und engt nach Filtration im Vakuum ein. Den so erhaltenen Rückstand reinigt man durch Chromatographie an Kieselgel. Mit Ether erhält man 7,3 mg der Titelverbindung als farbloses Öl.
IR (Film): 3400, 2934, 2859, 1709, 1490, 1439, 1246, 994, 756, 692 cm-1

**Beispiel 23**

2-{(E)-(2S)-2-((1E.3E)-(5S)-5-Hydroxy-6.6-trimethylen-9-phenyl-1.3-nonadien-8-inyl)-2-methyl-cyclohexyliden}-ethansäure

Zu einer Lösung von 400 mg des in Beispiel 1 beschriebenen Silylethers in 20 ml Tetrahydrofuran gibt man bei Raumtemperatur unter Stickstoff 1,2 g Tetrabutylammoniumfluorid x 3 $H_2O$ und rührt 4 Stunden nach. Anschließend verdünnt man mit Ether. wäscht mit Wasser und gesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-40% Ether erhält man 270 mg des Alkohols als farbloses Öl.
IR (Film): 3478, 2931, 2850, 1716, 1634, 1490, 1443, 1378, 1243, 1180, 1035, 994, 874, 836, 756, 692 $cm^{-1}$

Zu einer Lösung von 270 mg des vorstehend beschriebenen Alkohols in 5 ml Tetrahydrofuran und 4 ml Methanol werden 3 ml 1 molare Natronlauge gefolgt von 50 mg Lithiumhydroxid addiert und 5 Stunden bei 50°C, 20 stunden bei Raumtemperatur gerührt. Anschließend wird mit 1 normaler Schwefelsäure auf pH4 angesäuert, mit Ether extrahiert, die vereinigten organischen Phasen werden mit gesättigter Natriumchlorid-Lösung über Natriumsulfat getrocknet und nach Filtration im Vakuum eingeengt. Den so erhaltenen Rückstand reinigt man durch Chromatographie an Kieselgel. Mit Hexan / 0-40% Ether erhält man 400,7 mg der Titelverbindung als farbloses Öl.
IR (Film): 3400, 2940, 2860, 1690, 1630, 1490, 1440, 1250, 990, 760, 690, 530 $cm^{-1}$

**Beispiel 24**

5-{(E)-(2S)-2-((1E,3E)-(5S)-5-Hydroxy-6.6-trimethylen-9-phenyl-1,3-nonadien-8-inyl)-2-methyl-cyclohexyliden}-3-oxy-pentansäure-N-(5-tetraloxyl)-amid

Zu einer Lösung von 2,3 g des in Beispiel 1) hergestellten Esters in 18 ml Methanol und 20 ml Tetrahydrofuran gibt man 14.8 ml 1 molare Natronlauge und rührt 16 Stunden bei Raumtemperatur nach. Anschließend wird mit 10%iger Schwefelsäure auf pH5 eingestellt, mit Essigester extrahiert, die vereinigten organischen Phasen mit Wasser und gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Essigester / 0-5% Isopropanol erhält man 1,69 g der Säure als farbloses Öl.
IR (Film): 2930, 2359, 1732, 1651, 1598, 1490, 1462, 1442, 1360, 1250, 1106, 1060, 994, 835, 775, 755, 691 $cm^{-1}$

Zu einer Lösung von 281 mg der vorstehend beschnebenen Säure in 1.5 ml Tetrahydrofuran gibt man bei Raumtemperatur unter Stickstoff 58 mg 5-Aminotetrazol gefolgt von 117 mg N,N-Dicyclohexytcarbodiimid in 0.5 ml Tetrahydrofuran und rührt 20 Stunden bei Raumtemperatur. Anschließend wird der Niederschlag abgesaugt, mit Methylenchlorid gewaschen und im Vakuum eingeengt. Den so erhaltenen Rückstand reinigt man durch Chromatographie an Kieselgel. Mit Methylenchlorid / 0-20% Methanol erhält man 300 mg des Tetrazols als farbloses Öl.
IR (Film): 3200, 2920, 2850, 2160, 1690, 1610, 1580, 1520, 1480, 1400, 1350, 1240, 1100, 1050, 980, 830, 770, 750, 730, 680, 520

Zu einer Lösung von 280 mg des vorstehend beschriebenen Silylethers in 6 ml Tetrahydrofuran gibt man bei Raumtemperatur unter Stickstoff 900 mg Tetrabutylammoniumfluorid x 3 $H_2O$ und rührt 5 Stunden bei 40°C nach. Anschließend verdünnt man mit Essigester, wäscht mit Wasser und gesättigter Natriumchlorid-Lösung, trocknet die organische Phase über Natriumsulfat und engt. nach Filtration, im Vakuum ein. Den so erhaltenen Rückstand reinigt man durch Chromatograpnie an Kieselgel. Mit Methylenchlorid / 0-30% Methanol erhält man 184 mg der Titelverbindung als farbloses Öl.
IR (Film): 3400, 3200, 2930, 2860, 1700, 1620, 1590, 1490, 1400, 1100, 990, 760, 690 $cm^{-1}$

**Beispiel 25**

5-{(E)-(2S)-2-((1E,3E)-(5S)-5-Hydroxy-6.6-trimethylen-9-phenyl-1.3-nonadien-8-inyl)-2-methyl-cyclohexyliden}-3-oxa-pentansäure-methylester

Zu einer Lösung von 1 g der in Beispiel 4 beschriebenen Säure in 3.23 ml Aceton gibt man 673 mg Kaliumcarbonat gefolgt von 689 mg Methyljodid und rührt 24 Stunden bei Raumtemperatur. Anschließend wird mit Ether

verdünnt, mit gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-70% Ether erhält man 920 mg der Titelverbindung als farbloses Öl.
IR (Film): 3480, 2920, 2850, 1760, 1650, 1600, 1490, 1440, 1280, 1210, 1120, 990, 940, 920,. 760, 690, 520 $cm^{-1}$

**Beispiel 26**

5-{(E)-(2S)-2-((1E,3E)-(5S)-5-Hydroxy-6,6-trimethylen-9-phenyl-1,3-nonadien-8-inyl)-2-methyl-cyclohexyliden}-3-oxa-pentansäure-amid

Zu einer Lösung von 740 mg des in Beispiel 25 beschriebenen Alkohols in 5,6 ml N,N-Dimethylformamid gibt man bei O°C unter Stickstoff 423 mg Imidazol gefolgt von 470 mg tert.-Butyldimethylsilylchlorid. rührt 15 Minuten bei 0°C und 24 Stunden bei Raumtemperatur. Anschließend wird mit Ether verdünnt, mit Wasser, 10%iger Schwefelsäure, Wasser, gesättigter Natriumhydrogencarbonat-Lösung und gesättigter Natriumchlorid-Lösung gewaschen. Die organische Phase wird über Natriumsulfat getrocknet und nach Filtration im Vakuum eingeengt. Den so erhaltenen Rückstand reinigt man durch Chromatographie an Kieselgel. Mit Hexan / 0-30% Ether erhält man 890 mg der Titelverbindung als farbloses Öl.
IR (Film): 2928, 2855, 2360, 1757, 1684, 1653, 1540, 1472, 1362, 1362, 1254, 1205, 1122, 1070, 994, 938, 836, 775, 756, 691 $cm^{-1}$
Zu einer Lösung von 280 mg des vorstehend beschriebenen Esters in 2,5 ml Methanol und 2,5 Tetrahydrofuran gibt man 2.4 ml 1 molare Natronlauge und rührt 24 Stunden bei Raumtemperatur nach. Anschließend wird mit 10%iger Schwefelsäure auf pH5 eingestellt, mit Essigester extrahiert, die vereinigten organischen Phasen mit gesättigter Natnumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Essigester erhält man 250 mg der Säure als farbloses Öl.
IR (Film): 2928, 2855, 1740, 1490, 1462, 1442, 1372, 1249, 1110, 1101, 994, 938, 836, 809, 775, 756, 691, 666 $cm^{-1}$
Zu einer Lösung von 250 mg der vorstehend beschriebenen Säure in 3 ml Tetrahydrofuran gibt man bei 0°C unter Stickstoff 41µl Chlorameisensäureethylester gefolgt von 60 µl Triethylamin und rührt 10 Minuten nach. Nun werden 162 µl 25%ige Ammoniumhydroxid-Lösung addiert und 1 Stunde gerührt. Anschließend wird im Vakuum eingeengt, der Rückstand mit Essigester verdünnt, mit 10%iger Zitronensäure und gesättigter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-100% Essigester erhält man 260 mg des Amids als farbloses Öl.
IR (Film): 2928, 2855, 2360, 1634, 1338, 1254, 1071, 994, 836, 776, 756, 691, 668 $cm^{-1}$
Zu einer Lösung von 260 mg des vorstehend beschriebenen Silylethers in 12 ml Tetrahydrofuran gibt man bei Raumtemperatur unter Stickstoff 712 mg Tetrabutylammoniumfluorid x 3 $H_2O$ und rührt 6 Stunden bei Raumtemperatur nach. Anschließend verdünnt man mit Essigester, wäscht mit gesättigter Natriumchlorid-Lösung, trocknet die organische Phase über Natriumsulfat und engt, nach Filtration, im Vakuum ein. Den so erhaltenen Rückstand reinigt man durch Chromatographie an Kieselgel. Mit Hexan / 0-100% Essigester / 0-20% Methanol erhält man 158 mg der Titelverbindung als farbloses Öl.
IR (Film): 3470, 3400, 2920, 2860, 1740, 1680, 1600, 1490, 1440, 1370, 1340, 1240, 1100, 1050, 1000, 760, 690, 530 $cm^{-1}$
In den Figuren werden die Sturkturformeln und die wesentlichen Herstellungsparameter zusätzlich zu den Beispielen erläutert.

**In vivo Testsystem**

(i). Herstellung von humanen polymorphnuklenaren Leukozyten (PMN)

PMNs von gesunden Freiwilligen werden aus heparinisiertem venösem Blut durch Dextran - Sedimentation und anschließender Zentrifugation über Ficoll-Histopaque® isoliert. Die verbleibenden Erytrozyten werden durch hypotonische Lyse in 0,2 - prozentiger Natriumchlorid - Lösung beseitigt. Die PMNs werden in Hank's balanced salt solution (HBSS) resuspendiert und mit Ei - Albumin von Hühnern (OVA) oder Rinder - Serum - Albumin (BSA) versetzt.

(ii). LTB4 - Rezeptor - Kompetitions - Bindungsversuch

Humane PMNs werden mit Tritium markiertem Leukotrien $B_4$ ($LTB_4$) in Gegenwart oder Abwesenheit von den getesteten Substanzen in Konzentrationen von 10 µmol/l bis 0.05 mmol/l in HBSS zusammen mit OVA inkubiert. Zell - gebundenes. tntiummarkiertes $LTB_4$ wird von den freien Liganden durch Vakuum - Filtration durch einen Glasfaser -

Filter getrennt und in einem Szintillations - Meßgerät gemessen. Die nicht spezifische Bindung von tritiummarkiertem $LTB_4$ wird in Gegenwart von einem Überschuß an unmarkiertem $LTB_4$ (500 nmol/l) bestimmt. Der Kompetitionsfaktor (CF) wird aus dem Verhältnis von der Konzentration der Substanz zu der Konzentration des LTB4 errechnet, was zu einer 50 - prozentigen Reduktion der tritiummarkierten $LTB_4$ Rezeptor Bindung führt.

(iii). $LTB_4$ - induzierter Chemotaxis - Versuch

Der Chemotaxis - Versuch wird mit modifizierten Boyden - Kammern ausgeführt. die aus Transwell® Modulen mit polyvinylpyrrolidon - ummantelten Polykarbon - Filtem mit einer Porengrößen von 3 µm besteht. Der obere Kammerteil enthält die humanen PMNs in HBSS, welches mit BSA oder OVA ergänzt ist. Der untere Kammerteil wird allein mit Puffer oder mit dem chemotaktisch aktiven Leukotrien $B_4$ ($LTB_4$) in einer Konzentration im Rahmen von 1 nmol/l bis 100 nmol/l in Gegenwart oder Abwesenheit von der Testsubstanz hinzugegeben. Die Kammer wird für 60 Minuten in wassergesättigter Atmosphäre mit 5 % Kohlendioxid inkubiert. Die Anzahl der PMNs. die in den unteren Kammerteil gewandert sind, wird durch die Messung der Aktivität des Enzyms Myeloperoxidase (MPO) in einem kalibrierten Versuch bestimmt. Die Enzymaktivität wird spektrometrisch (450 nm) durch Bestimmung der Rate an $H_2O_2$ - abhängiger Oxidation vom aromatischen Amin 3,3,',5.5'-Tetramethylbenzidin (TMB) gemessen.

Der $EC_{50}$ - Wert wird graphisch durch die nicht lineare Regressionkurve bestimmt. Der $K_B$ - Wert beschreibt das Vermögen des kompetitiven Antagonisten. Der $K_B$ - Wert wird als die Antagonisten - Konzentration ermittelt, die erforderlich ist. den $EC_{50}$ - Wert des Agonisten um den Faktor 2 anzuheben.

Der $K_B$ - Wert wird wie folgt berechnet:

$$K_B = [LTB_4 \text{ - Rezeptor - Antagonist}] / (DR\text{-}1)$$

(DR = ist das Verhältnis von der $LTB_4$ Konzentration, die für die halb - maximale Stimulation in Gegenwart des Antagonisten benötigt wird. zu der $LTB_4$ Konzentration, die für die halb - maximale Stimulation in Abwesenheit des Antagonisten benötigt wird.)

(iv). $LTB_4$/Iloprost - induzierte Hautentzündung in den Ohren von Mäusen

Weibliche NMRI - Mäuse mit einem Gewicht von 26 bis 28 g und einem Alter von 5 bis 6 Wochen werden für dieses *in vivo* Experiment verwendet. Zehn Tiere pro Gruppe werden nach dem Zufallsprinzip in die verschiedenen Behandlungsgruppen eingeteilt und einzeln gehalten. Die Tiere hatten freien Zugang zum Futter und Wasser. Um die orale Aufnahme von topisch zu applizierendem $LTB_4$/Iloprost Lösungen zu vermeiden, werden Sperr - Halsbänder um den Hals der Tiere unter Ether - Narkose kurz vor der topischen Applikation befestigt.

Leukotrien B4 ($LTB_4$) und das stabilen Prostacyclin - Derivat Iloprost wird in Ethanol/Isopropylmyristat (95 + 5 v/v) bei einer Konzentration von 0,003 % (w/v) gelöst. 10 µl der $LTB_4$/Iloprost - Lösung wird topisch auf der äußeren Oberfläche eines jeden Ohres (Fläche etwa 1 $cm^2$/Ohr) verabreicht. Dieses entspricht einer Dosis von 0.3µg pro Ohr oder etwa 0.3 µg pro $cm^2$. Tiere, die allein mit $LTB_4$/Iloprost - Lösung behandelt werden, entwickeln die typischen Merkmale einer entzündeten Haut unter Bildung von Ödemen und Infiltration von Neutrophilen. Diese Tiere dienen als Positiv - Kontrolle. Tiere, die allein mit Ethanol/Isopropylmyristat (95 + 5 v/v) auf der äußeren Oberfläche eines jeden Ohres (Fläche etwa 1 $cm^2$/Ohr) behandelt werden, dienen als Negativ - Kontrolle.

Der Effekt des LTB4 - Rezeptor - Antagonisten auf die $LTB_4$/Iloprost induzierte Entzündungsreaktion wird entweder bei einer topischen oder bei einer intragastralen Verabreichung von der Testsubstanz bestimmt.

Für die topische Applikation wird die Testsubstanz in einer LTB4/Iloprost - Lösung in verschiedenen Konzentration gelöst. 10 µl dieser Lösung wird topisch auf die äußere Oberfläche des Ohrs aufgetragen.

Für die intragastrale Applikation wird der LTB4 - Rezeptor - Antagonist in Ethanol gelöst. Direkt nach der topische Verabreichung mit LTB4/Iloprost wird der LTB4 - Rezeptor - Antagonist oder nur das Lösungsmittel intragastral bei verschiedenen Dosen mit Hilfe einer Sonde verabreicht Die höchste Endkonzentration von Ethanol ist 3 %. Die Menge an Ethanol nimmt mit weiteren Verdünnungsschritten ab.

Die Tiere werden 24 Stunden nach dem Setzen der entzündlichen Reaktion getötet. Die Ohren werden abgetrennt. gewogen, schock - gefroren und für weitere Untersuchungen gelagert. Die Peroxidase Aktivität wird spektrometrisch in dem Homogeriat von der Ohrhaut bestimmt. Das Gewebe wird in HTAB-Puffer (0,5% Hexadecyltrimethylammoniumbromid (w/v) in $10^{-3}$ mol/l 3-[Nmorpholino]propansulfonsäure mit pH 7.0) für 20 Sekunden mit einem Polytron® PT 3000 (Kinematica AG. Schweiz) bei einer Rotation von 30000 Umdrehungen pro Minute homogenisiert. Das Homogenat wird für 20 Minuten bei 10 °C und bei 14500 Umdrehungen pro Minute (20000g) in einer Sorvall RC2-B Zentrifuge (SM-24 Rotor) zentrifugiert. Der wäßrige Uberstand wird abgesaugt und auf seine Peroxidase Aktivität bei einer Verdünnung von 1 zu 50 in HTAB-Puffer ausgetestet. Die Peroxidase Aktivität wird durch photomethrische Messung der Rate an $H_2O_2$ - abhängigen Oxidation des aromatischen Amins 3,3',5,5'-Tetramethylbenzidin (TMB) bestimmt. In einer 96 - Loch - Mikrotiterplatte werden die verdünnten Überstände mit TMB - Lösung und Hydrogenperoxid inkubiert (Lösung von 6.5 mg 3,3',5,5'-Tetramethylbenzidindihydrochlorid in 1 ml Dimethylsulfoxid (DMSO): 1: 100 (v/v) gelöst mit 0.1 mol/l Natrium - Acetat - Zitrat - Puffer. pH 6.0. endgültige Konzentration in der Inkubationsmixtur: $1{,}57 \cdot 10^{-4}$ mol/l) (Hydrogenperoxid 30% $H_2O_2$ 1 : 16860 (v/v) gelöst mit 0.1 mol/l Natrium - Acetat - Zitrat - Puffer, pH 6,0, endgültige Konzentration in der Inkubationsmixtur: $4.93 \cdot 10^{-5}$ mol/l). Nach 30 Minuten bei Raumtemperatur wird die Reaktion durch Zugabe von 0,5 mol/l Schwefelsäure gestoppt. Die Extinktion wird bei 450 nm (maximale Absorption) in einem Mikrotiter - Platten Meßgerät bestimmt.

## Beispiel 1

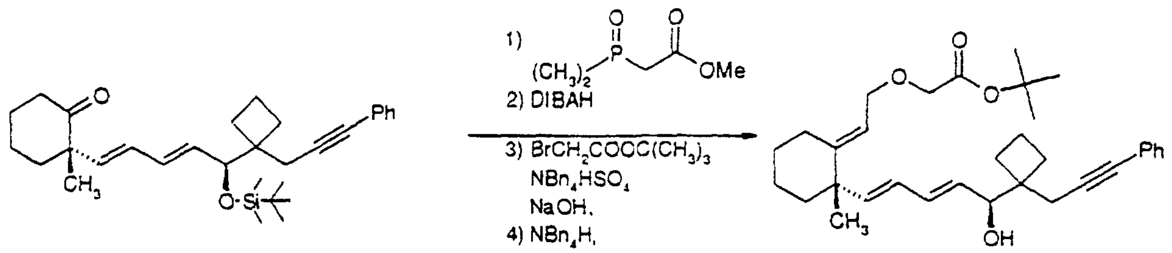


### 1a)

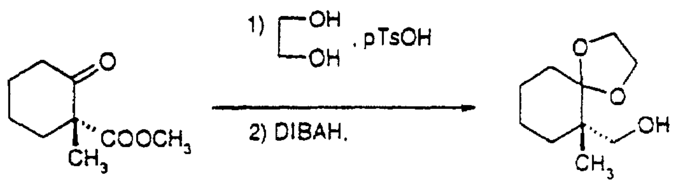


### 1b)

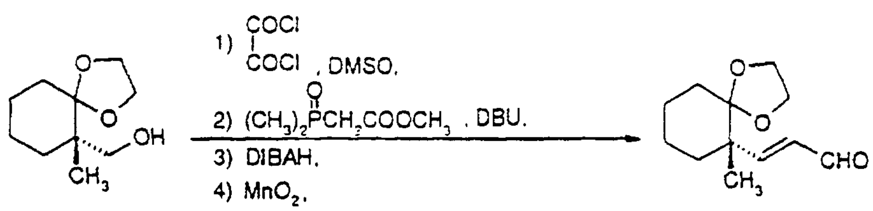


### 1c)

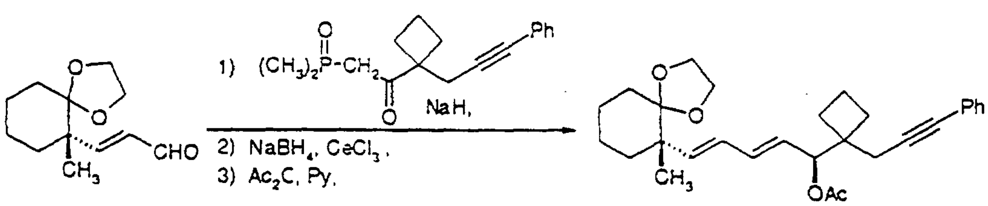

1d)

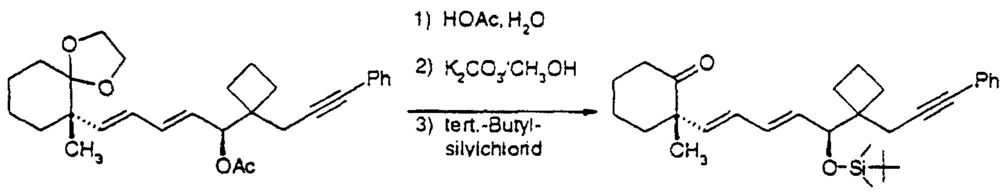


## Beispiel 2

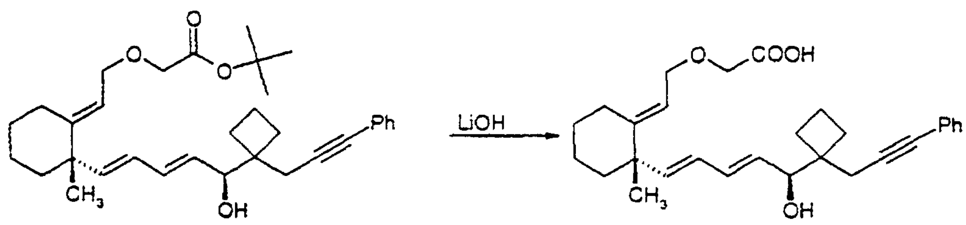


## Beispiel 3

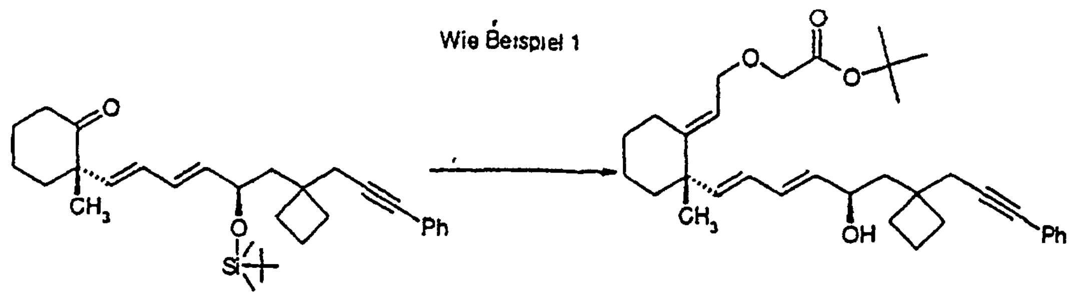


3a)

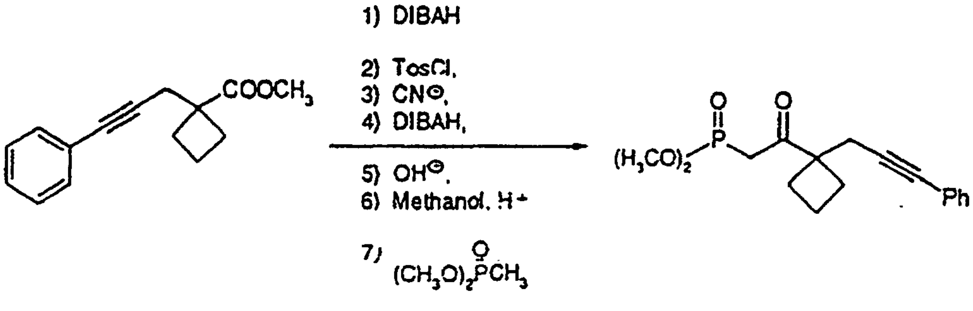

**3b)**

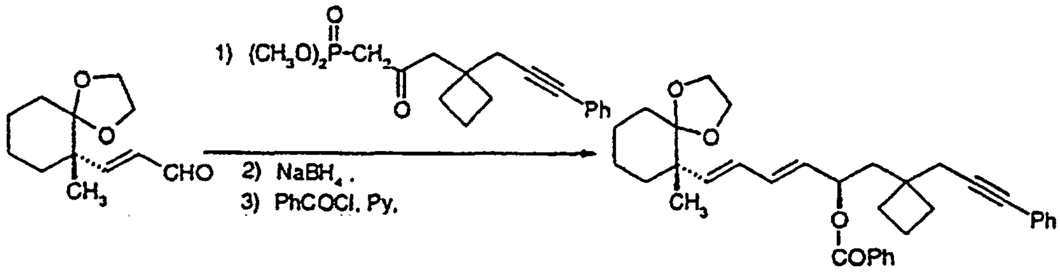


**3c)**

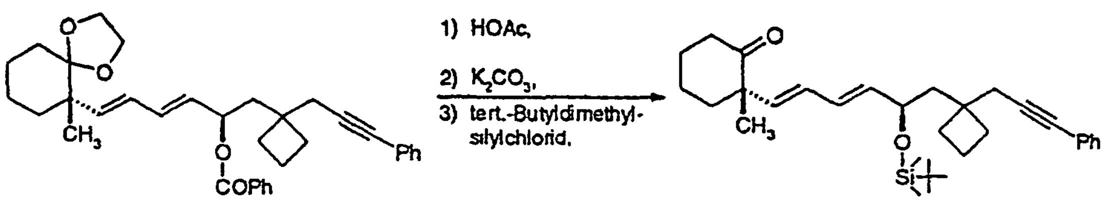


**Beispiel 4**

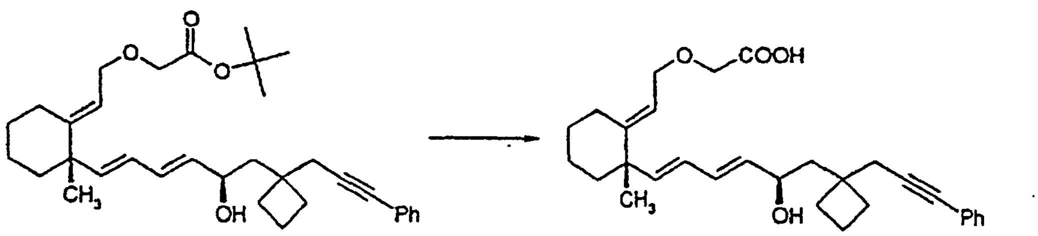

(optisch aktiv)

**Beispiel 5** racemisch

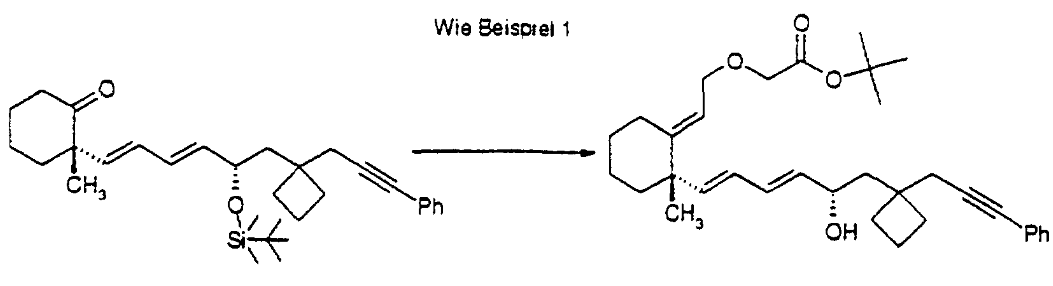

**5a)**

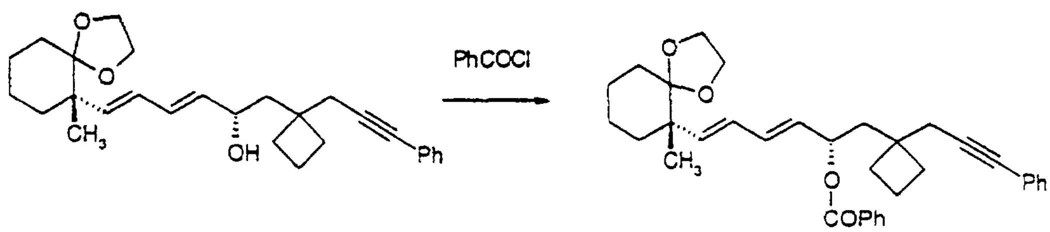


aus Beispiel 3b)

**5b)**

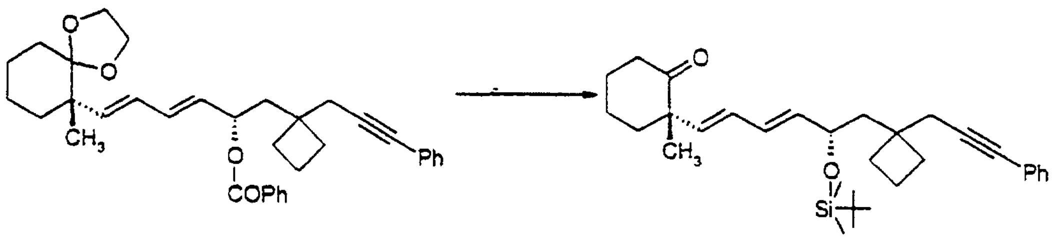

**Beispiel 6**

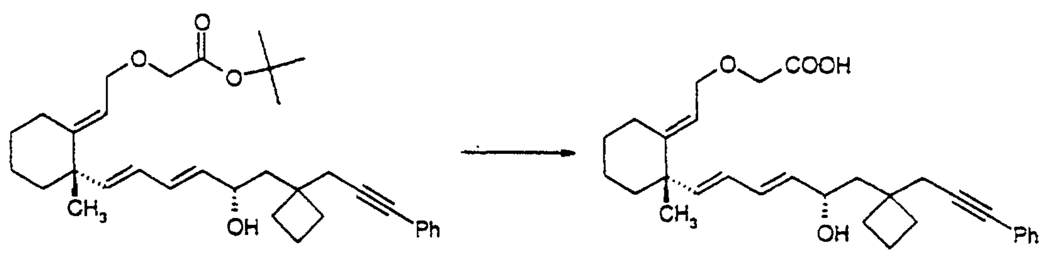

(optisch aktiv)

**Beispiel 7**

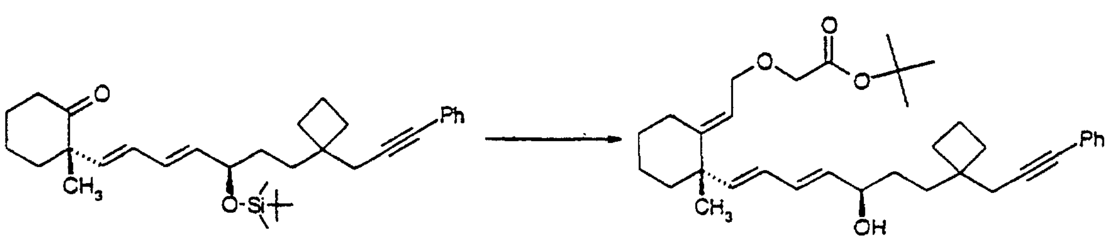

7a)

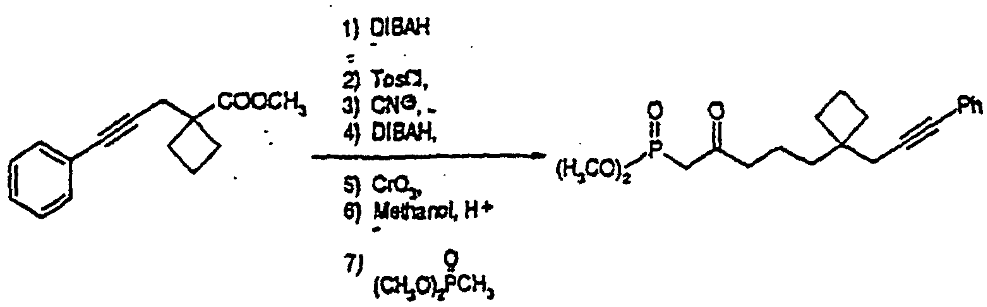


aus Beispiel 3a)

7b)

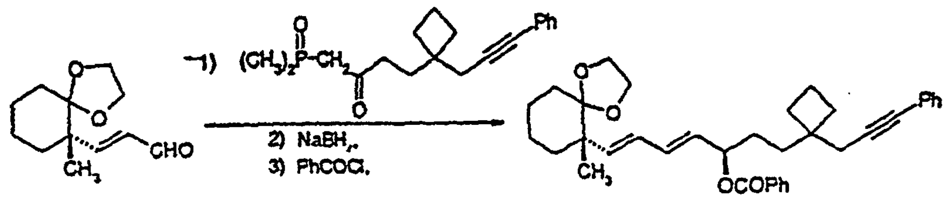


7c)

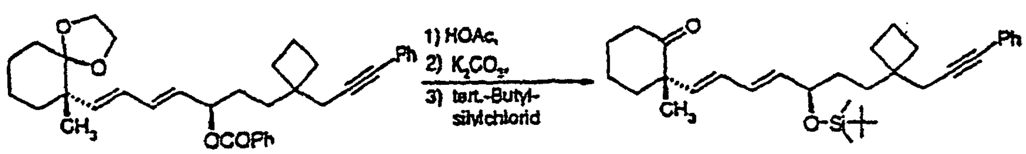


**Beispiel 8**

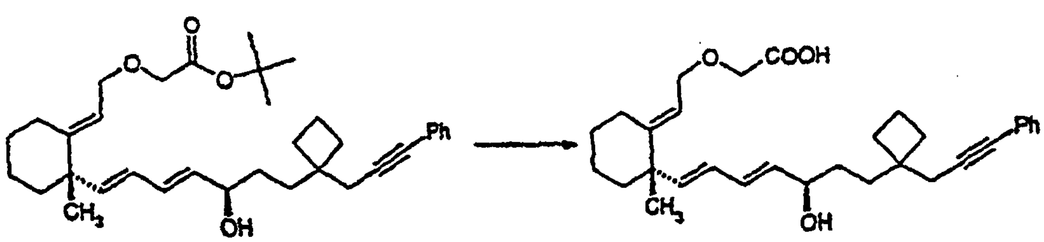

## Beispiel 9

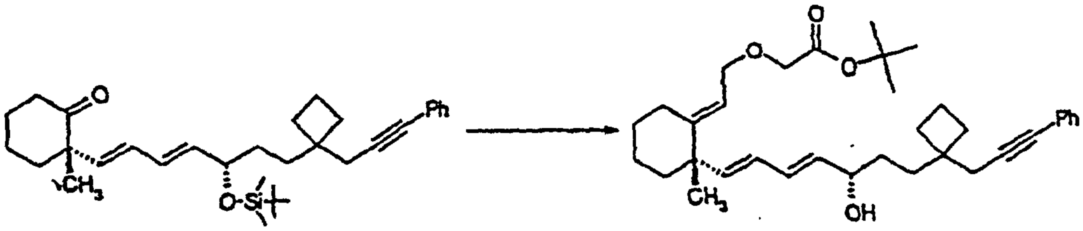

### 9a)

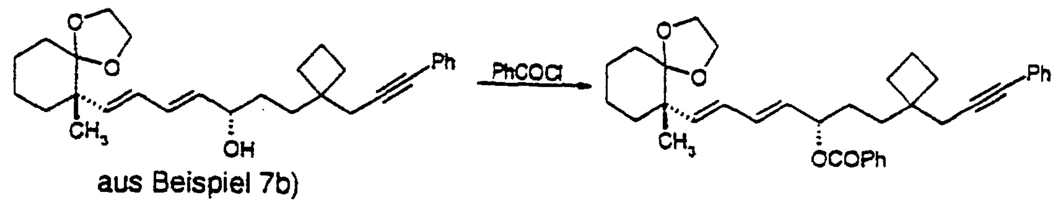


aus Beispiel 7b)
polarer Alkohol

### 9b)

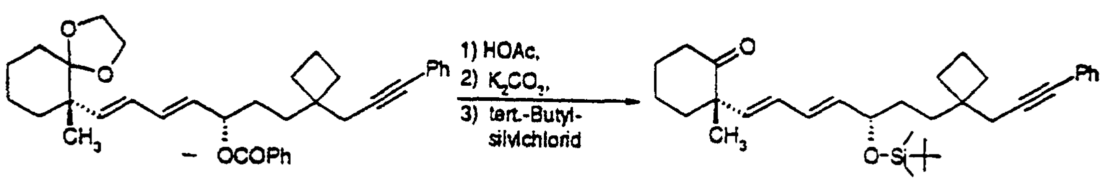


## Beispiel 10

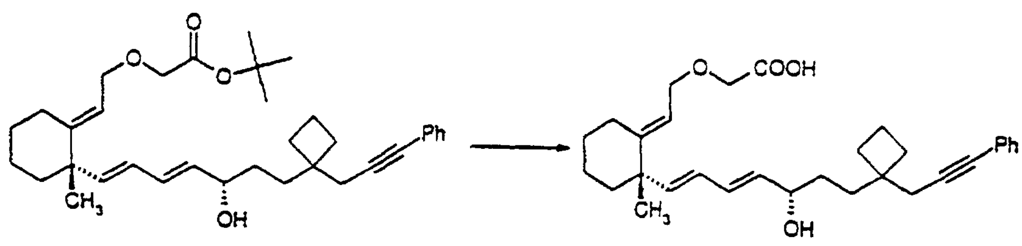

## Beispiel 11

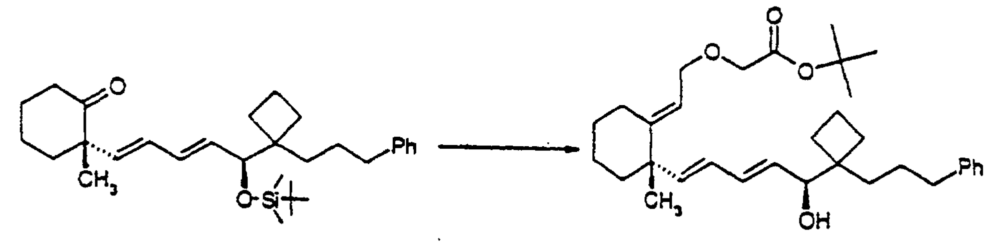

### 11a)

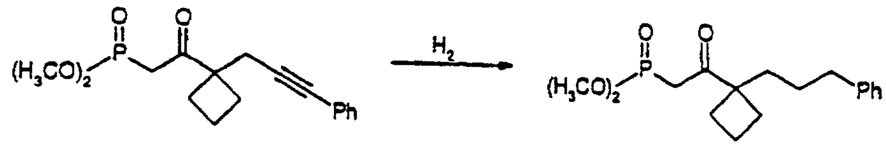


### 11b)

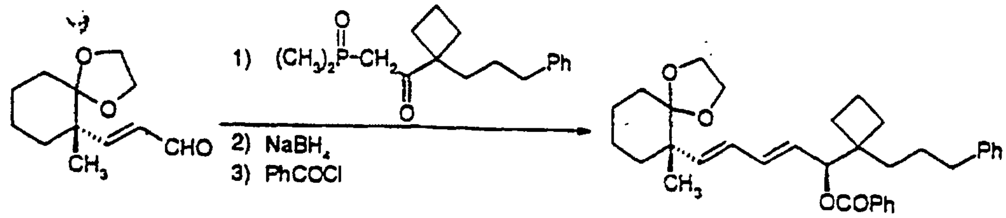


### 11c)

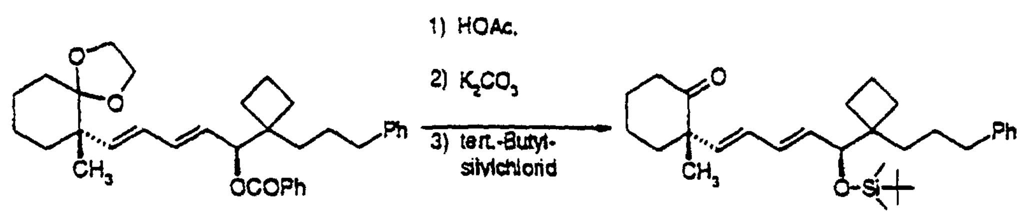

## Beispiel 12

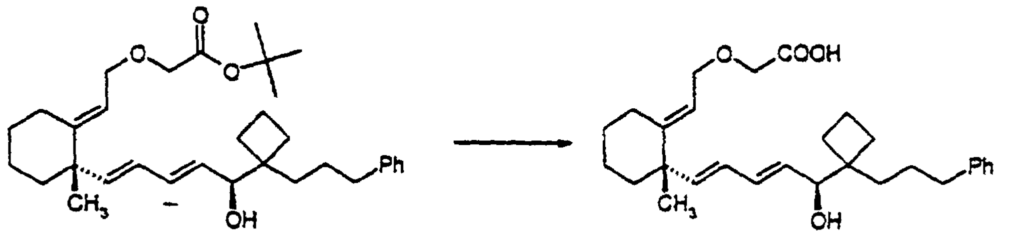

## Beispiel 13

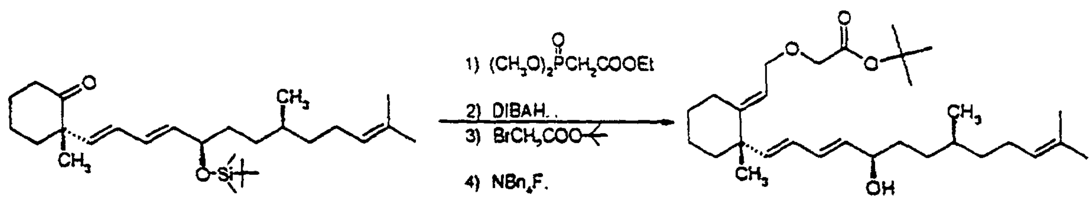


## 13a)

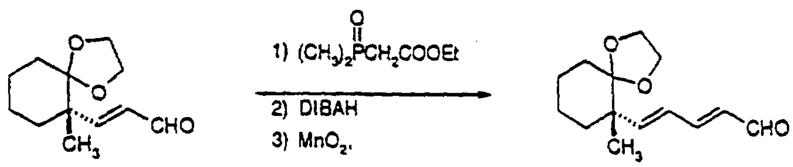


## 13b)

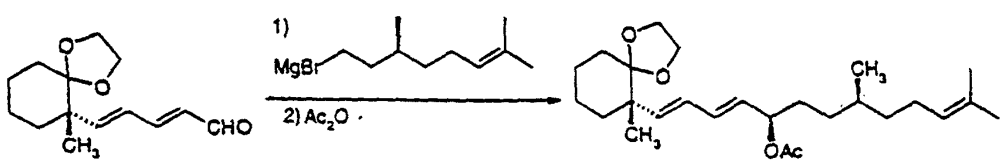


## 13c)

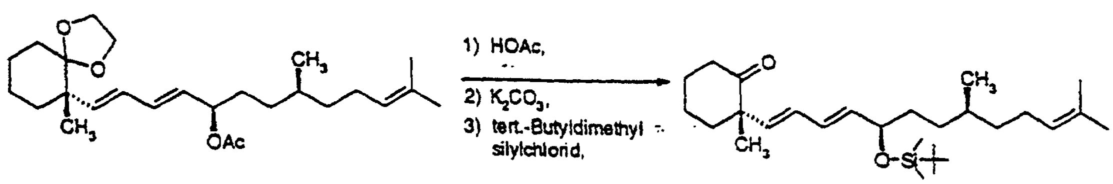

## Beispiel 14

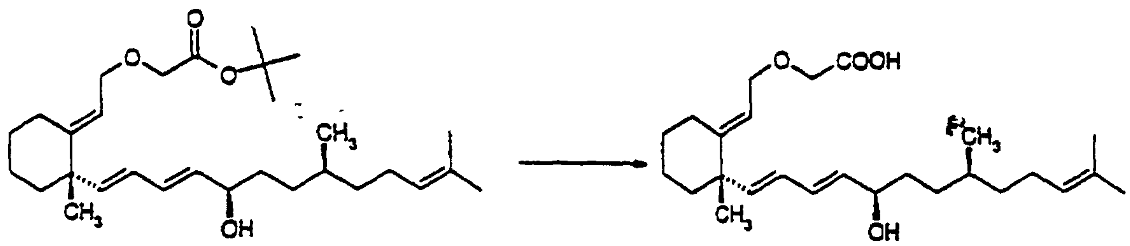

## Beispiel 15

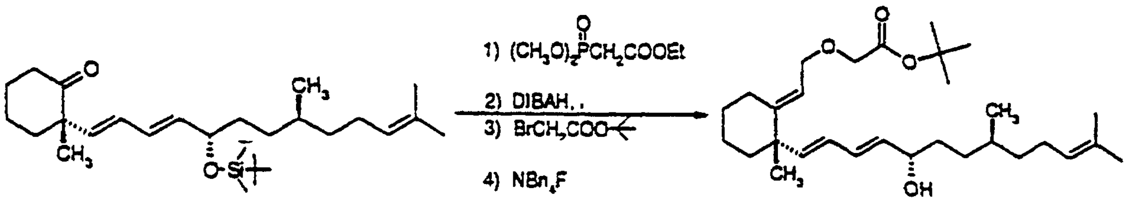


**15a)**

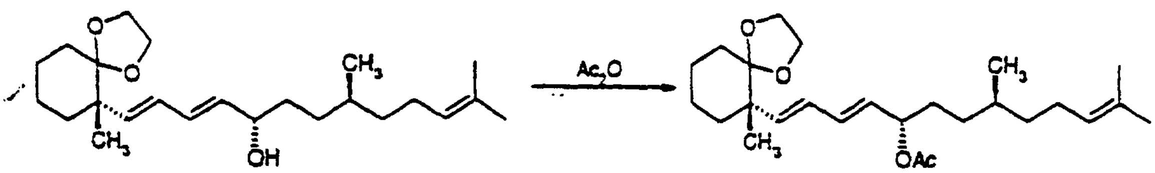


aus 13b) polarer Alkohol

**15b)**

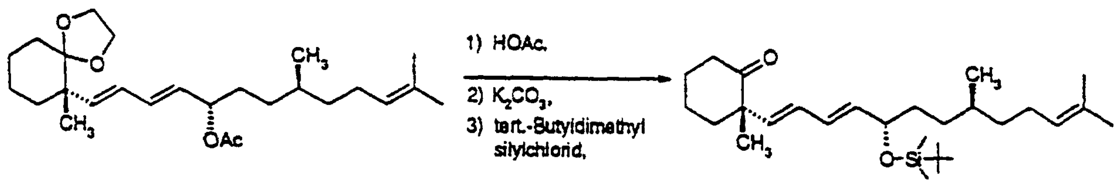

## Beispiel 16

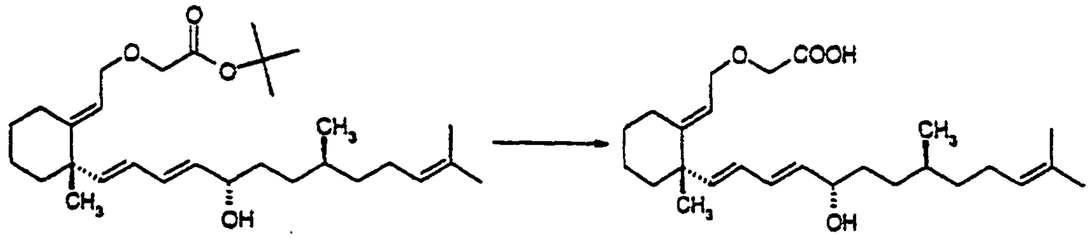

## Beispiel 17

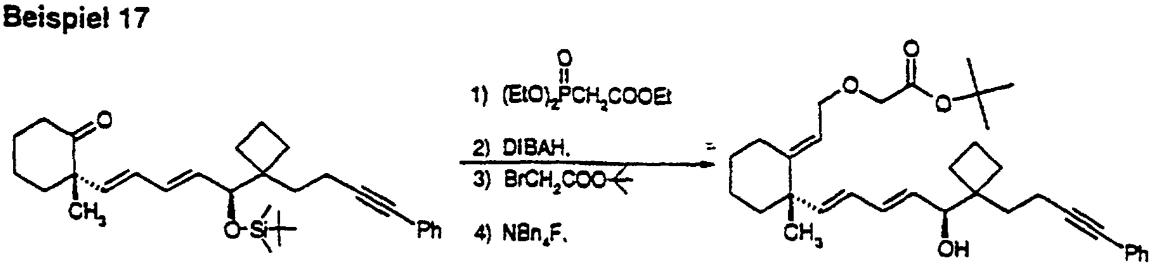


## 17a)

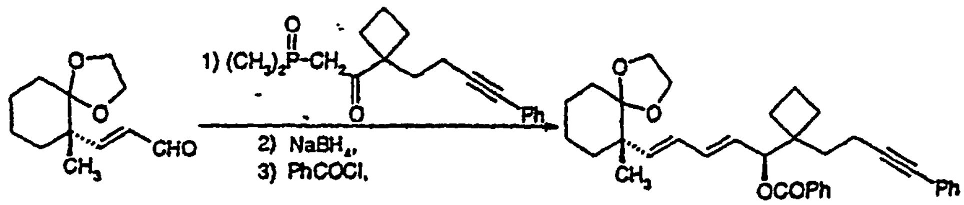


## 17b)

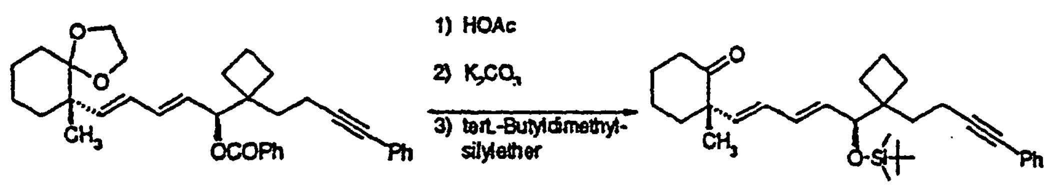

## Beispiel 18

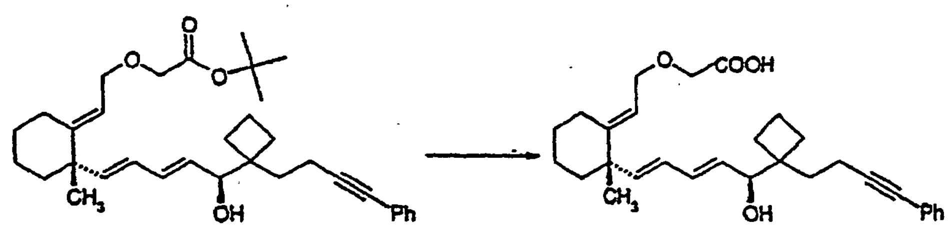

## Beispiel 19

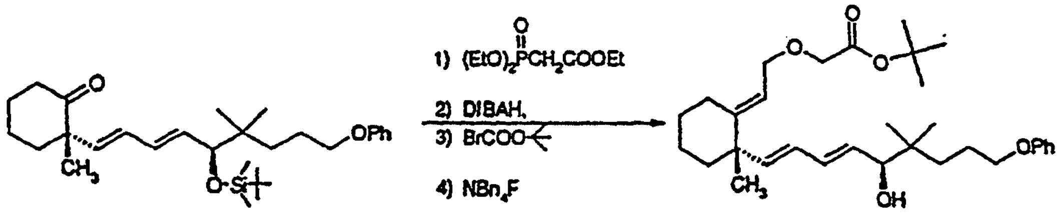


### 19a)

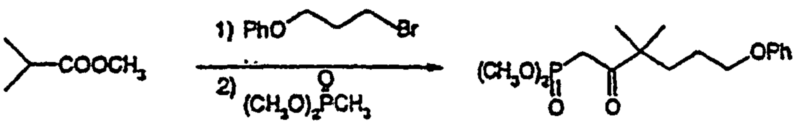


### 19b)

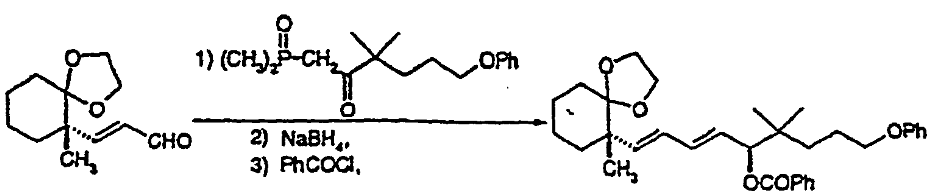


### 19c)

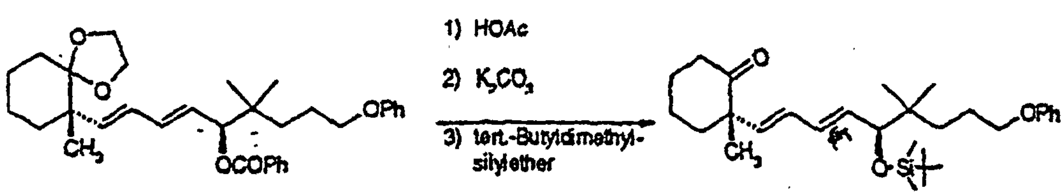

**Beispiel 20**

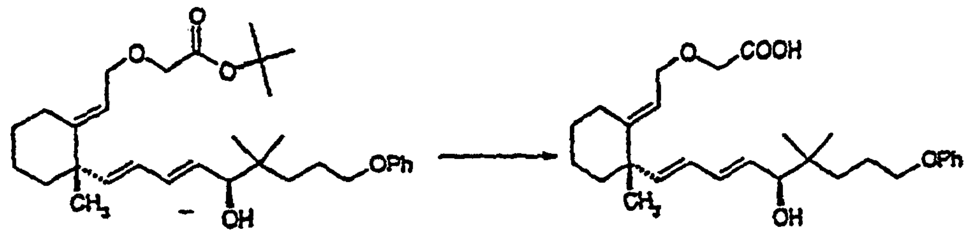

**Beispiel 21**

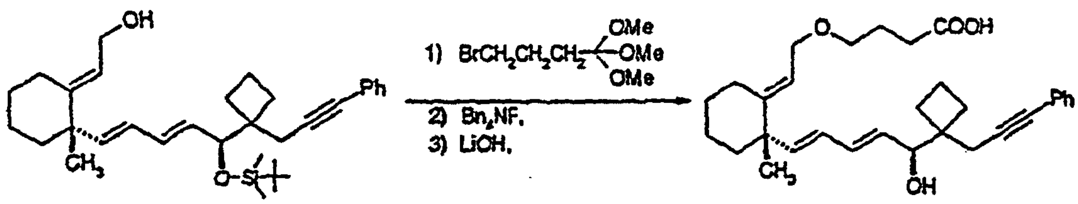


aus Beispiel 1)

**Beispiel 22**

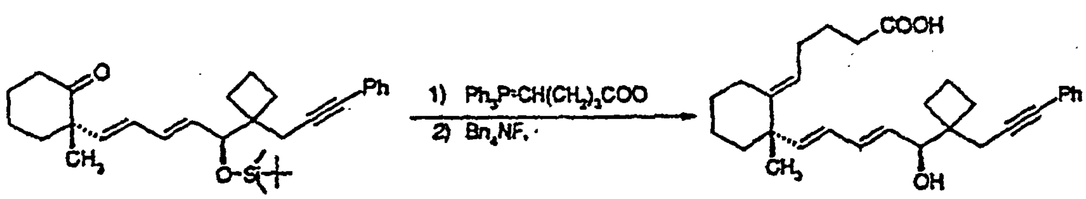


**Beispiel 23**

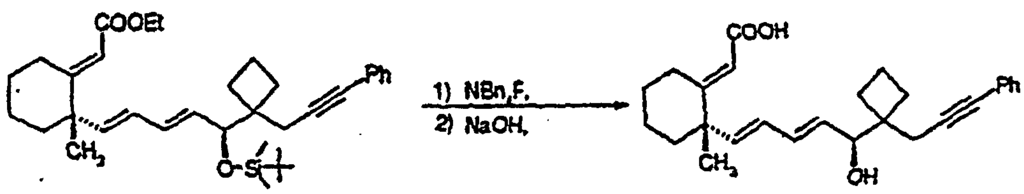


aus Beispiel 1)

**Beispiel 24**

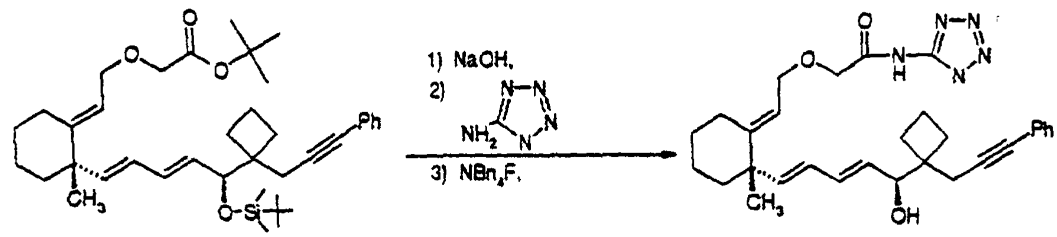


**Beispiel 25**

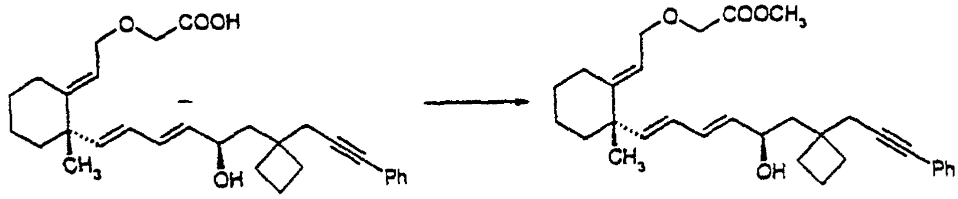

aus Beispiel 6)

**Beispiel 26**

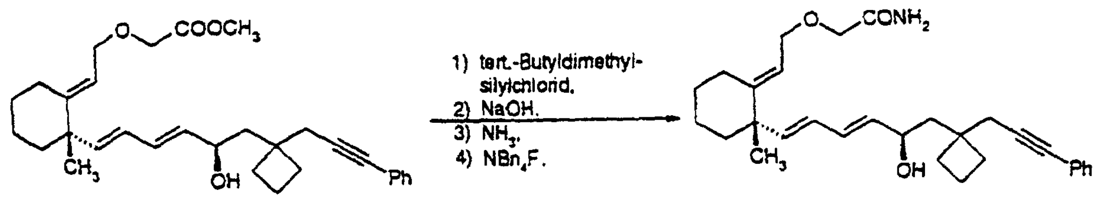


**Patentansprüche**

1. Leukotrien-$B_4$-Derivate der allgemeinen Formel I,

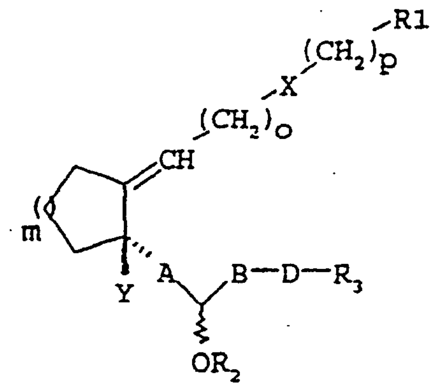

(I)

worin

| | |
|---|---|
| $R_1$ | $CH_2OH$, $CH_3$, $CF_3$, $COOR_4$, $CONR_5R_6$, und |
| $R_2$ | H oder einen organischen, Säurerest mit 1-15 C-Atomen darstellt, |
| $R_3$ | H, gegebenenfalls einfach oder mehrfach substituiertes $C_1$-$C_{14}$-Alkyl. $C_3$-$C_{10}$-Cycloalkyl, einen gegebenenfalls unabhängig voneinander einfach oder mehrfach durch Halogen, Phenyl, $C_1$-$C_4$-Alkyl, $C_1$-$C_4$-Alkoxy, Fluormethyl, Chlormethyl, Trifluormethyl, Carbonyl, Carboxyl oder Hydroxy substituierten $C_6$-$C_{10}$-Arylrest oder einen 5-6 gliedrigen aromatischen heterocyclischen Ring mit wenigstens 1 Heteroatom symbolisiert, |
| $R_4$ | Wasserstoff, $C_1$-$C_{10}$-Alkyl, $C_3$-$C_{10}$-Cycloalkyl, einen gegebenenfalls durch 1-3 Halogen, Phenyl, $C_1$-$C_4$-Alkyl, $C_1$-$C_4$-Alkoxy, Fluormethyl, Chlormethyl, Trifluormethyl, Carboxyl oder Hydroxy substituierten $C_6$-$C_{10}$-Arylrest, $CH_2$-CO-($C_6$-$C_{10}$) Aryl oder einen 5-6 gliedrigen Ring mit wenigstens 1 Heteroatom bedeutet, |
| A | eine trans, trans-CH=CH-CH=CH. eine $-CH_2CH_2-CH=CH-$ oder eine Tetramethylengruppe, |
| B | eine $C_1$-$C_{10}$- gerad- oder verzweigtkettige Alkylengruppe, die gegebenenfalls durch Fluor substituiert sein kann oder die Gruppe |

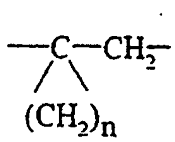 oder 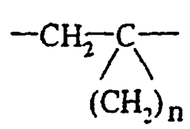

| | |
|---|---|
| | symbolisiert, |
| D | eine Direktbindung, Sauerstoff. Schwefel, -C≡C-, $-CH=CR_7$, oder gemeinsam mit |
| B | auch eine Direktbindung bedeuten kann, |
| $R_5$ und $R_6$ | gleich oder verschieden sind und H oder gegebenenfalls durch Hydroxygruppen substituiertes $C_1$-$C_4$-Alkyl oder $R_6$ H und $R_5$ $C_1$-$C_{15}$-Alkanoyl oder $R_8SO_2$- darstellen, |
| $R_7$ | H, $C_1$-$C_5$-Alkyl, Chlor, Brom bedeutet, |
| $R_8$ | die gleiche Bedeutung wie $R_3$ besitzt, |
| m | 1-3 bedeutet, |
| o | 0-5 bedeutet, |
| p | 0-5 bedeutet, |
| X | eine Direktbindung, Sauerstoff, Schwefel, |
| Y | ein gegebenenfalls einfach oder mehrfach substituiertes $C_1$-$C_8$-Alkyl, $C_3$-$C_{10}$-Cycloalkyl, gegebenenfalls substituiert durch Aryl, und |
| n | 2-5 ist sowie, wenn $R_4$ Wasserstoff bedeutet, deren Salze mit physiologisch verträglichen Basen und deren Cyclodextrinclathrate. |

2. Pharmazeutische Präparate **gekennzeichnet durch** einen Gehalt an Leukotrien-$B_4$-Derivaten der allgemeinen Formel I gemäß Patentanspruch 1.

3. Verfahren zur Herstellung von Leukotrien-$B_4$-Derivaten der allgemeinen Formel I, gemäß Patentanspruch 1, **dadurch gekennzeichnet, daß** man ein Keton der Formel II

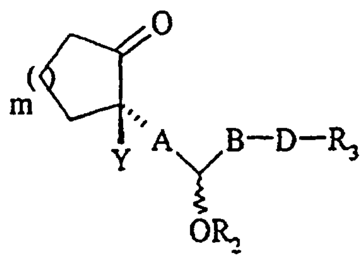

(II)

worin A, B, D, $R_2$, $R_3$ und Y die oben angegebene Bedeutung haben, gegebenenfalls nach Schutz freier Hydroxygruppen in $R_2$ mit einem Olefinierungsreagenz der allgemeinen Formel III

$$E\text{-}CH_2\text{-}(CH_2)_o\text{-}X\text{-}(CH_2)_p\text{-}R_1 \qquad \text{(III)}$$

wobei E einen

$\overset{(+)}{Ph_3P}$— oder 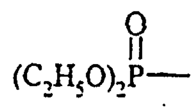 oder 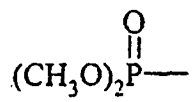 Rest

darstellt und o, X, p, $R_1$ die oben angegebene Bedeutung aufweisen, in Gegenwart einer Base umsetzt und gegebenenfalls anschließend in beliebiger Reihenfolge Isomere trennt, geschützte Hydroxygruppen freisetzt und/ oder eine freie Hydroxygruppe verethert und/ oder die 1-Hydroxygruppe zur Carbonsäure oxidiert und/ oder reduziert und/ oder eine Carboxylgruppe verestert und/ oder eine freie Carboxylgruppe in ein Amid überführt oder eine Carboxylgruppe mit einer physiologisch verträglichen Base in ein Salz überführt.

## Claims

1. Leukotriene $B_4$ derivatives of the general formula I

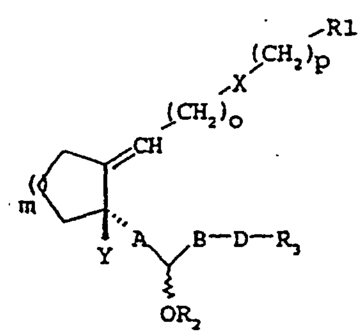

(I)

in which

| | |
|---|---|
| $R_1$ | is $CH_2OH$, $CH_3$, $CF_3$, $COOR_4$, $CONR_5R_6$, and |
| $R_2$ | is H or an organic acid residue with 1-15 C atoms, |
| $R_3$ | is H, optionally mono- or multisubstituted $C_1$-$C_{14}$-alkyl, $C_3$-$C_{10}$-cycloalkyl, a $C_6$-$C_{10}$-aryl radical which is optionally substituted independently of one another one or more times by halogen, phenyl, $C_1$-$C_4$- |

alkyl, $C_1$-$C_4$-alkoxy, fluoromethyl, chloromethyl, trifluoromethyl, carbonyl, carboxyl or hydroxyl, or a 5-6-membered aromatic heterocyclic ring with at least one heteroatom,

$R_4$ is hydrogen, $C_1$-$C_{10}$-alkyl, $C_3$-$C_{10}$-cycloalkyl, a $C_6$-$C_{10}$-aryl radical which is optionally substituted by 1-3 halogen, phenyl, $C_1$-$C_4$-alkyl, $C_1$-$C_4$-alkoxy, fluoromethyl, chloromethyl, trifluoromethyl, carboxyl or hydroxyl, or $CH_2$-CO-($C_6$-$C_{10}$)-aryl or a 5-6-membered ring with at least one heteroatom,

A is a trans, trans-CH=CH-CH=CH, a $-CH_2CH_2$-CH=CH- or a tetramethylene group,

B is a $C_1$-$C_{10}$ straight- or branched-chain alkylene group which may optionally be substituted by fluorine, or the group

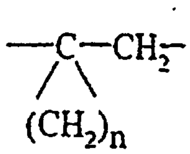

or

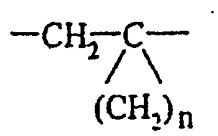

D may be a direct linkage, oxygen, sulphur, -C≡C-, -CH=$CR_7$, or together with

B also a direct linkage,

$R_5$ and $R_6$ are identical or different and are H or $C_1$-$C_4$-alkyl which is optionally substituted by hydroxyl groups, or $R_6$ is H and $R_5$ is $C_1$-$C_{15}$-alkanoyl or $R_8SO_2$-,

$R_7$ is H, $C_1$-$C_5$-alkyl, chlorine, bromine,

$R_8$ has the same meaning as $R_3$,

m is 1-3,

o is 0-5,

p is 0-5,

X is a direct linkage, oxygen, sulphur,

Y is an optionally mono- or multisubstituted $C_1$-$C_8$-alkyl, $C_3$-$C_{10}$-cycloalkyl, optionally substituted by aryl, and

n is 2-5, and, when $R_4$ is hydrogen, the salts thereof with physiologically tolerated bases and the cyclodextrin clathrates thereof.

2. Pharmaceutical products **characterized by** a content of leukotriene $B_4$ derivatives of the general formula I according to Claim 1.

3. Process for the preparation of leukotriene $B_4$ derivatives of the general formula I according to Claim 1, **characterized in that** a ketone of the formula II

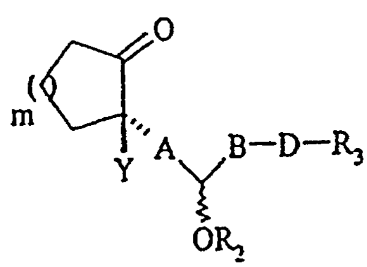

(II)

in which A, B, D, $R_2$, $R_3$ and Y have the meanings stated above, where appropriate after protection of free hydroxyl groups in $R_2$, is reacted with an olefination reagent of the general formula III

$$E\text{-}CH_2\text{-}(CH_2)_o\text{-}X\text{-}(CH_2)_p\text{-}R_1 \quad \text{(III)}$$

where E is a

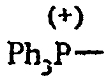 or 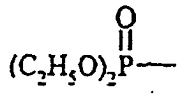 or 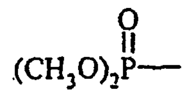 radical

and o, X, p, $R_1$ have the meanings stated above, in the presence of a base, and, where appropriate, subsequently in any sequence isomers are separated, protected hydroxyl groups are liberated and/or a free hydroxyl group is etherified and/or the 1-hydroxyl group is oxidized to the carboxylic acid and/or reduced and/or a carboxyl group is esterified and/or a free carboxyl group is converted into an amide, or a carboxyl group is converted with a physiologically tolerated base into a salt.

## Revendications

**1.** Dérivés de leucotriène $B_4$ de formule générale I,

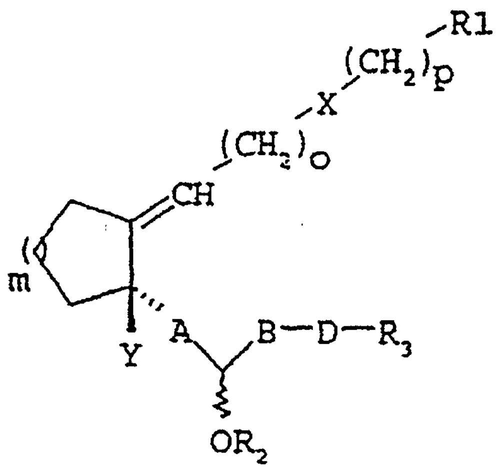

(I)

dans laquelle

| | |
|---|---|
| $R_1$ | représente $CH_2OH$, $CH_3$, $CF_3$, $COOR_4$, $CONR_5R_6$, et |
| $R_2$ | représente H ou un radical acide organique ayant de 1 à 15 atomes de carbone, |
| $R_3$ | représente H, un alkyle en $C_1$-$C_{14}$ éventuellement mono- ou polysubstitué, un cycloalkyle en $C_3$-$C_{10}$, un radical aryle en $C_6$-$C_{10}$ éventuellement mono- ou polysubstitué indépendamment par un halogène, un phényle, un alkyle en $C_1$-$C_4$, un alcoxy en $C_1$-$C_4$, un fluorométhyle, un chlorométhyle, un trifluorométhyle, un carbonyle, un carboxy ou un hydroxy, ou un noyau hétérocyclique aromatique à 5-6 chaînons ayant au moins 1 hétéroatome, |
| $R_4$ | représente un hydrogène, un alkyle en $C_1$-$C_{10}$, un cycloalkyle en $C_3$-$C_{10}$, un radical aryle en $C_6$-$C_{10}$ éventuellement mono- à trisubstitué par un halogène, un phényle, un alkyle en $C_1$-$C_4$, un alcoxy en $C_1$-$C_4$, un fluorométhyle, un chlorométhyle, un trifluorométhyle, un carboxy ou un hydroxy, un $CH_2$-CO-($C_6$-$C_{10}$)-aryle ou un noyau à 5-6 chaînons ayant au moins 1 hétéroatome, |
| A | représente un groupe trans, trans-CH=CH-CH=CH, -$CH_2$-$CH_2$-CH=CH- ou tétraméthylène, |
| B | représente un groupe alkylène en $C_1$-$C_{10}$ linéaire ou ramifié qui peut être éventuellement substitué par un fluor ou le groupe |

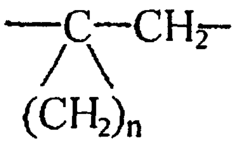 ou 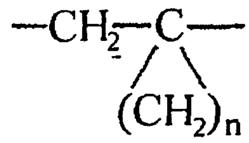

| | |
|---|---|
| D | représente une liaison directe, un hydrogène, un soufre, un -C=C-, un -CH=$CR_7$, ou conjointement avec |
| B | peut également représenter une liaison directe, |
| $R_5$ et $R_6$ | sont identiques ou différents et représentent H ou un alkyle en $C_1$-$C_4$ éventuellement substitué par des groupes hydroxy, ou $R_6$ représente H et $R_5$ représente un alcanoyle en $C_1$-$C_{15}$ ou $R_8SO_2$-, |
| $R_7$ | représente H, un alkyle en $C_1$-$C_5$, un chlore ou un brome, |
| $R_8$ | présente la même signification que $R_3$, |
| m | vaut de 1 à 3, |
| o | vaut de 0 à 5, |
| p | vaut de 0 à 5, |
| X | représente un liaison directe, un oxygène ou un soufre, |
| Y | représente un alkyle en $C_1$-$C_8$ éventuellement mono- ou polysubstitué ou un cycloalkyle en $C_3$-$C_{10}$ éventuellement substitué par un aryle, et |
| n | vaut de 2 à 5, si $R_4$ représente un hydrogène, leurs sels avec des bases physiologiquement compatibles et leurs clathrates de cyclodextrine. |

2. Préparations pharmaceutiques **caractérisées par** une teneur en dérivés de leucotriène $B_4$ de formule générale I selon la revendication 1.

3. Procédé de préparation de dérivés de leucotriène $B_4$ de formule générale I selon la revendication 1, **caractérisé en ce qu'**une cétone de formule II

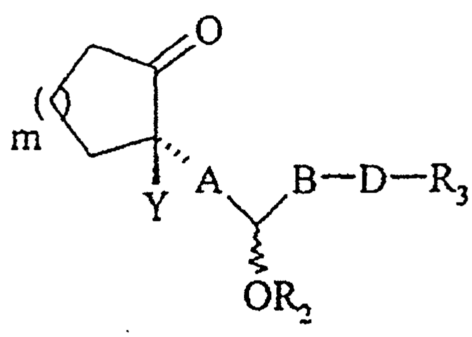 (II)

dans laquelle A, B, D, $R_2$, $R_3$ et Y ont la signification indiquée ci-dessus, éventuellement après protection des groupes hydroxy libres de $R_2$, est mise à réagir avec un réactif d'oléfination de formule générale III

$$E\text{-}CH_2\text{-}(CH_2)_o\text{-}X\text{-}(CH_2)_p\text{-}R_1 \qquad \text{(III)}$$

dans laquelle E représente un radical

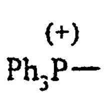 ou 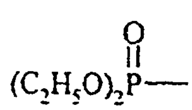 ou 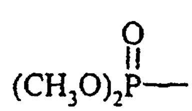

et o, X, p et $R_1$ présentent la signification indiquée ci-dessus, en présence d'une base, et ensuite éventuellement, dans un ordre quelconque, les isomères sont séparés, les groupes hydroxy protégés sont libérés et/ou un groupe hydroxy libre est éthérifié et/ou le groupe 1-hydroxy par rapport à l'acide carboxylique est oxydé et/ou réduit et/ou un groupe carboxy est estérifié et/ou un groupe carboxy libre est transformé en amide ou un groupe carboxy est transformé en sel avec une base physiologiquement compatible.